(19) Europäisches Patentamt
European Patent Office
Office européen des brevets

(11) EP 4 467 142 A2

(12) # EUROPEAN PATENT APPLICATION
published in accordance with Art. 153(4) EPC

(88) Date of publication A3:
**15.06.2023 Bulletin 0000/00**

(43) Date of publication:
**27.11.2024 Bulletin 2024/48**

(21) Application number: **23714052.0**

(22) Date of filing: **18.01.2023**

(51) International Patent Classification (IPC):
***A61K 31/53*** *(2006.01)* ***A61K 9/10*** *(2006.01)*
***A61K 9/20*** *(2006.01)* ***A61K 31/194*** *(2006.01)*
***A61K 47/12*** *(2006.01)* ***A61K 47/26*** *(2006.01)*
***A61P 11/00*** *(2006.01)* ***A61P 31/12*** *(2006.01)*
***A61P 31/14*** *(2006.01)* ***A61P 43/00*** *(2006.01)*
***C07D 403/14*** *(2006.01)*

(52) Cooperative Patent Classification (CPC):
**A61K 9/10; A61K 9/20; A61K 31/194; A61K 31/53; A61K 47/12; A61K 47/26; A61P 11/00; A61P 31/12; A61P 31/14; A61P 43/00; C07D 403/14**

(86) International application number:
**PCT/JP2023/001243**

(87) International publication number:
**WO 2023/054732 (06.04.2023 Gazette 2023/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.01.2022 JP 2022006725**
**28.01.2022 JP 2022012386**
**07.02.2022 JP 2022017132**
**25.02.2022 JP 2022027629**
**28.07.2022 JP 2022120325**
**21.09.2022 JP 2022149666**

(71) Applicant: **Shionogi & Co., Ltd**
**Osaka-shi, Osaka 541-0045 (JP)**

(72) Inventors:
- **SHIMIZU Ryosuke**
**Osaka-shi, Osaka 541-0045 (JP)**
- **MATSUO Yumiko**
**Osaka-shi, Osaka 541-0045 (JP)**
- **FUKUHARA Takahiro**
**Osaka-shi, Osaka 541-0045 (JP)**
- **FUKAO Keita**
**Osaka-shi, Osaka 541-0045 (JP)**
- **KURODA Takayuki**
**Osaka-shi, Osaka 541-0045 (JP)**
- **NOBORI Haruaki**
**Osaka-shi, Osaka 541-0045 (JP)**
- **ISHIBASHI Toru**
**Osaka-shi, Osaka 541-0045 (JP)**

(74) Representative: **Vossius & Partner**
**Patentanwälte Rechtsanwälte mbB**
**Siebertstrasse 3**
**81675 München (DE)**

Remarks:
The complete document including Reference Table(s) and the Sequence Listing(s) can be downloaded from the EPO website

(54) **PHARMACEUTICAL FOR TREATING NOVEL CORONAVIRUS INFECTION**

(57) The present invention provides a pharmaceutical composition containing a coronavirus 3CL protease inhibitor for treating novel coronavirus infections (COVID-19).

A pharmaceutical composition for treating novel coronavirus infections (COVID-19) is provided, the composition containing, as an active ingredient, a complex that contains:
a compound represented by Formula (I):

EP 4 467 142 A2

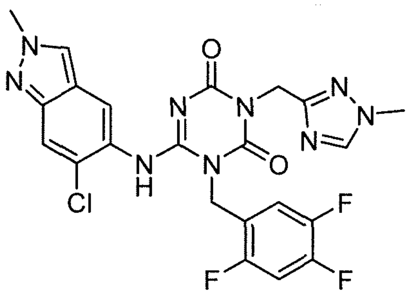 ( I )

;

and
fumaric acid.

## Description

[TECHNICAL FIELD]

The present invention relates to the treatment of diseases associated with a coronavirus 3CL protease. In particular, the present invention relates to a pharmaceutical composition containing a coronavirus 3CL protease inhibitor for treating novel coronavirus infections (COVID-19).

[BACKGROUND ART]

From the 1960s to 2018, six coronaviruses have been reported. Among them, symptoms of four types (OC43, 229E, NL63 and HKU1) are mild symptoms such as normal cold symptoms and gastrointestinal symptoms. On the other hand, an infectious disease caused by severe acute respiratory syndrome coronavirus (SARS-CoV), which occurred in Asia and Canada in 2002 to 2003, and an infectious disease caused by Middle East respiratory syndrome coronavirus (MERS-CoV), which occurred in the Middle East region in 2012 or later are zoonoses, and their high infectivity and high lethality in humans have raised serious public health concerns (Non-patent document 1).

In December 2019, cluster outbreaks of pneumonia of unknown cause were reported in several locations in Wuhan City, Hubei Province, People's Republic of China. As a result of epidemiological investigation, it was confirmed that this pneumonia was caused by SARS-CoV-2 (Non-patent Document 1).

SARS-CoV-2 invades host cells when the spike protein on its outer shell specifically binds to angiotensin-converting enzyme 2 (ACE2), which is expressed on the cell membrane surface of target host cells. ACE2 is expressed in nasal mucosal epithelial cells, alveolar epithelial cells, epithelial cells of the small intestine, and the like (Non-patent Document 2).

An infection by SARS-CoV-2 is named COVID-19 and the World Health Organization (WHO) declared an "Public Health Emergency of International Concern (PHEIC)" on COVID-19 on January 30, 2020. Then, on March 11, 2020, they announced that COVID-19 could be considered a pandemic (global outbreak) due to the global spread of infection and its severity (Non-patent Document 3). SARS-CoV-2 was originally thought to be infected only from animals to humans, but in addition to infection to humans from patients infected with diseases, infection to humans from asymptomatic or presymptomatic infected patients has been also revealed (Non-patent Document 1).

Main symptoms of COVID-19 include many symptoms similar to cold, such as fever, coughing, runny nose, nasal congestion, sore throat, muscle pain, and diarrhea. In addition, symptoms such as loss of taste and smell have also been reported. Young people are less likely to develop severe symptoms, but older patients and high-risk patients with cardiovascular disease, respiratory disease, kidney disease, diabetes, obesity, or immunodeficiency are more likely to develop severe pneumonia. The pneumonia progresses rapidly, leading to shortness of breath, and dyspnea, which may require treatment with oxygen inhalation, a ventilator, or extracorporeal membrane oxygenation (ECMO). Thromboembolism is also a hallmark of severe symptom of COVID-19, with high mortality. Therefore, tightness and collapse of the medical system have occurred in various countries around the world (Non-patent Document 1).

In December 2021, the cumulative number of infected people (the number of dead people) spread in 223 countries and regions, 1.7 million cases (18,000 cases) or more in Japan and 276 million cases (5.3 million cases) or more in the world. There are 60 countries in which the number of infected people is reported to exceed 300,000 (Non-patent Document 4).

As of December 2021, vaccination has progressed worldwide, but spike protein mutant strains that cause increased infectivity and pathogenicity and immune escape have also been recognized, and there is a possibility that this will become a problem in the future. As COVID-19 therapeutic agents, in Japan, remdesivir which is a direct-acting antiviral agent, dexamethasone and baricitinib which are immunomodulators/immunosuppressants, caciliviumab and imdevimab which are anti- SARS-CoV-2 monoclonal antibodies, and sotrovimab, and molnupiravir which is an RNA protease inhibitor have been approved. However, there is no end in sight for the spread of infection, and development of a new therapeutic drug is desired.

In November 2021, it was reported on the Pfizer website, PAXLOVID (TM) (PF-07321332; ritonavir) reduced the risk of hospitalization or death by 89% in high-risk adult patients compared to placebo (Non-patent Document 5).

Furthermore, in December 2021, PAXLOVID (TM) was approved for emergency use in the United States, and on February 10, 2022, the Paxlovid®PACK was approved as special case approval in Japan.

The structural formula of PAXLOVID (TM) (PF-07321332) is as described below and is different from the chemical structure of the compound of the present invention (Non-patent Document 6).

[Chemical Formula 1]

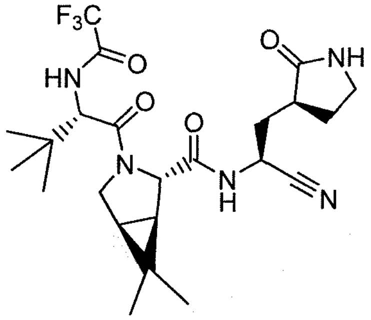

On November 29, 2021, the non-clinical study results of S-217622 targeting 3CL protease and its chemical structural formula were published. In addition, it was disclosed that good safety and tolerability were confirmed in a single ascending dose study in healthy Japanese adults. However, nothing has been disclosed regarding therapeutic effects on patients infected with novel coronavirus infections (COVID-19) and its dosage regimen (Non-patent Document 7).

[PRIOR ART REFERENCES]

[Non-patent Document]

[Non-patent Document 1] J Clin Med 2020; 9: 1225

[Non-patent Document 2] J Pathol 2004; 203: 631-7

[Non-patent Document 3] World Health Organization.WHO Director-General's opening remarks at the media briefing on COVID-19. 11 March 2020. [searched on December 19, 2021], Internet <URL: httpsV/www.who.int/dg/speeches/detail/who-director-general-s-opening-remarks-at-the-media-briefing-on-covid-19---11-march-2020>

[Non-patent Document 4] World Health Organization. Coronavirus Disease (COVID-19) Dashboard. 5 October 2020. [Searched on December 19, 2022], Internet <https://covid19.who.int/>

[Non-patent Document 5] "Pfizer's Novel COVID-19 Oral Antiviral Treatment Candidate Reduced Risk Of Hospitalization Or Death By 89% In Interim Analysis Of Phase 2/3 EPIC-HR Study", [online], November 5, 2021, Pfizer Press Release, [retrieved on September 21, 2022], Internet <URL: https://www.pfizer.com/news/press-release/press-release-detail/pfizers-novel-covid-19-oral-antiviral-treatment-candidate>

[Non-patent Document 6] 261st Am Chem Soc (ACS) Natl Meet • 2021-04-05 / 2021-04-16 • Virtual, N/A • Abst 243

[Non-patent Document 7] AIMECS 2021 (AFMC International Medicinal Chemistry Symposium 2021), Online Symposium, November 29 to December 2, 2021


[SUMMARY OF THE INVENTION]

[PROBLEMS TO BE SOLVED BY THE INVENTION]

It is an object of the present invention to provide a pharmaceutical composition for treating novel coronavirus infections (COVID-19), the composition having an excellent coronavirus 3CL protease inhibitory activity.

[MEANS FOR SOLVING THE PROBLEM]

As a result of intensive studies to achieve the above-described object, the present inventors have found that when the coronavirus 3CL protease inhibitor described in Non-patent Document 7 is administered to a human at a specific dosage regimen, the coronavirus 3CL protease inhibitor maintains an effective concentration in blood without exceeding NOAEL (no observable adverse effect level), and is useful for treatment of novel coronavirus infections (COVID-19). Thus, they completed the present invention.

The present invention relates to the following (1) to (36).

(1) A pharmaceutical composition for treating novel coronavirus infections (COVID-19), the composition containing, as an active ingredient, a complex that contains:

a compound represented by Formula (1):

[Chemical Formula 2]

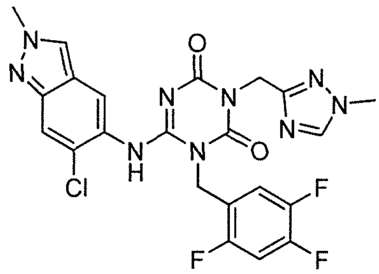

( I )

;

and
fumaric acid.

(2) The pharmaceutical composition according to (1), wherein a patient infected with novel coronavirus infections (COVID-19) is classified as asymptomatic, mild illness or moderate illness I.
(3) The pharmaceutical composition according to (1) or (2), which is used for reducing a disease duration of novel coronavirus infections (COVID-19).
(4) The pharmaceutical composition described in any one of (1) to (3), which is used for inhibiting virus replication of SARS-CoV-2.
(5) The pharmaceutical composition according to any one of (1) to (4), wherein the daily dose of the active ingredient of the pharmaceutical composition is 20 mg to 2000 mg.
(6) The pharmaceutical composition according to any of (1) to (5), wherein the daily dose of the active ingredient on Treatment Day 1 is 375 mg and the daily dose of the active ingredient from Treatment Day 2 to Treatment Day 5 is 125 mg each.
(7) The pharmaceutical composition according to any of (1) to (5), wherein the daily dose of the active ingredient on Treatment Day 1 is 750 mg and the daily dose of the active ingredient from Treatment Day 2 to Treatment Day 5 is 250 mg each.
(8) The pharmaceutical composition according to any one of (1) to (7), which is administered once a day.
(9) The pharmaceutical composition according to any one of (1) to (8), wherein the pharmaceutical composition is an orally administered drug.
(10) A method of treatment of novel coronavirus infections (COVID-19), the method including the step of administering an effective amount of a complex as an active ingredient to an individual in need of treatment of the novel coronavirus infections (COVID-19), the complex containing:

a compound represented by Formula (I):

[Chemical Formula 3]

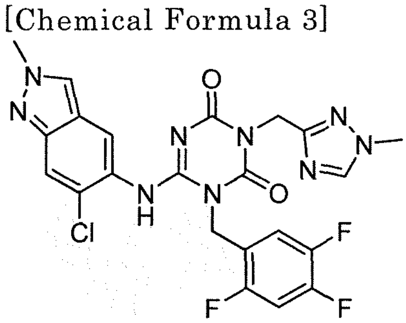

( I )

;

and
fumaric acid.

(11) The method of treatment according to (10), wherein a patient infected with novel coronavirus infections (COVID-19) is classified as asymptomatic, mild illness or moderate illness I.

(12) The method of treatment according to (10) or (11) to be used for reducing a disease duration of novel coronavirus infections (COVID-19).

(13) The method of treatment according to any one of (10) to (12) to be used for inhibiting virus replication of SARS-CoV-2.

(14) The method of treatment according to any one of (10) to (13), wherein the daily dose of the active ingredient is 20 mg to 2000 mg.

(15) The method of treatment according to any of (10) to (14), wherein the daily dose of the active ingredient on Treatment Day 1 is 375 mg and the daily dose of the active ingredient from Treatment Day 2 to Treatment Day 5 is 125 mg each.

(16) The method of treatment according to any of (10) to (14), wherein the daily dose of the active ingredient on Treatment Day 1 is 750 mg and the daily dose of the active ingredient from Treatment Day 2 to Treatment Day 5 is 250 mg each.

(17) The method of treatment according to any one of (10) to (16), wherein the administration is performed once a day.

(18) The method of treatment according to any one of (10) to (17), wherein the administration is in an orally administered drug form.

(19) A use of a complex as an active ingredient containing:

a compound represented by Formula (I):

[Chemical Formula 4]

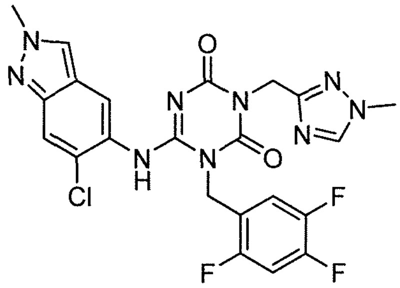

( I )

;

and

fumaric acid,

for producing a medicament for treatment of novel coronavirus infections (COVID-19).

(20) The use according to (19), wherein a patient infected with novel coronavirus infections (COVID-19) is classified as asymptomatic, mild illness or moderate illness I.

(21) The use according to (19) or (20) to be used for reducing a disease duration of novel coronavirus infections (COVID-19).

(22) The use according to any one of (19) to (21) to be used for inhibiting virus proliferation of SARS-CoV-2.

(23) The use according to any one of (19) to (22), wherein the daily dose of the active ingredient is 20 mg to 2000 mg.

(24) The use according to any of (19) to (23), wherein the daily dose of the active ingredient on Treatment Day 1 is 375 mg and the daily dose of the active ingredient from Treatment Day 2 to Treatment Day 5 is 125 mg each.

(25) The use according to any of (19) to (23), wherein the daily dose of the active ingredient on Treatment Day 1 is 750 mg and the daily dose of the active ingredient from Treatment Day 2 to Treatment Day 5 is 250 mg each.

(26) The use according to any one of (19) to (25), wherein the administration is performed once a day.

(27) The use according to any one of (19) to (26), wherein the administration is in an orally administered drug form.

(28) A complex as an active ingredient containing:

a compound represented by Formula (I):

[Chemical Formula 5]

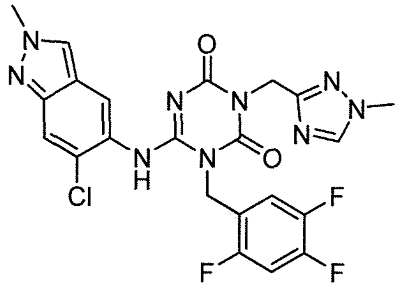

( I )

;

and
fumaric acid,
to be used for treatment of novel coronavirus infections (COVID-19).

(29) The complex containing the compound and the fumaric acid according to (28), wherein a patient infected with novel coronavirus infections (COVID-19) is classified as asymptomatic, mild illness or moderate illness I.
(30) The complex containing the compound and the fumaric acid according to (28) or (29) to be used for reducing a disease duration of novel coronavirus infections (COVID-19).
(31) The complex containing the compound and the fumaric acid according to any one of (28) to (30) to be used for inhibiting virus replication of SARS-CoV-2.
(32) The complex containing the compound and the fumaric acid according to any one of (28) to (31), wherein the daily dose of the active ingredient is 20 mg to 2000 mg.
(33) The complex containing the compound and the fumaric acid according to any one of (28) to (32), wherein the daily dose of the active ingredient on Treatment Day 1 is 375 mg and the daily dose of the active ingredient from Treatment Day 2 to Treatment Day 5 is 125 mg each.
(34) The complex containing the compound and the fumaric acid according to any one of (28) to (32), wherein the daily dose of the active ingredient on Treatment Day 1 is 750 mg and the daily dose of the active ingredient from Treatment Day 2 to Treatment Day 5 is 250 mg each.
(35) The complex containing the compound and the fumaric acid according to any one of (28) to (34), wherein the administration is performed once a day.
(36) The complex containing the compound and the fumaric acid according to any one of (28) to (35), being in an orally administered drug form.

In addition, the present invention also relates to the following items (1') to (10').

(1') The complex containing the compound and the fumaric acid according to any one of (28) to (32), wherein the daily dose of the active ingredient on Treatment Day 1 is 375 mg and the daily dose of the active ingredient from Treatment Day 2 to Treatment Day 5 is 125 mg each.
(2') The complex containing the compound and the fumaric acid according to any one of (28) to (32), wherein the daily dose of the active ingredient on Treatment Day 1 is 250 mg and the daily dose of the active ingredient from Treatment Day 2 to Treatment Day 5 is 125 mg each.
(3') The complex containing the compound and the fumaric acid according to any one of (28) to (32), wherein the daily dose of the active ingredient on Treatment Day 1 is 250 mg and the daily dose of the active ingredient from Treatment Day 2 to Treatment Day 5 is 100 mg each.
(4') The complex containing the compound and the fumaric acid according to any one of (28) to (32), wherein the daily dose of the active ingredient on Treatment Day 1 is 125 mg and the daily dose of the active ingredient from Treatment Day 2 to Treatment Day 5 is 75 mg each.
(5') The complex containing the compound and the fumaric acid according to any one of (28) to (32), wherein the daily dose of the active ingredient on Treatment Day 1 is 150 mg and the daily dose of the active ingredient from Treatment Day 2 to Treatment Day 5 is 75 mg each.
(6') The complex containing the compound and the fumaric acid according to any one of (28) to (32), wherein the daily dose of the active ingredient on Treatment Day 1 is 75 mg and the daily dose of the active ingredient from Treatment Day 2 to Treatment Day 5 is 50 mg each.
(7') The complex containing the compound and the fumaric acid according to any one of (28) to (32), wherein the daily dose of the active ingredient on Treatment Day 1 is 100 mg and the daily dose of the active ingredient from Treatment Day 2 to Treatment Day 5 is 50 mg each.

(8') The complex containing the compound and the fumaric acid according to any one of (28) to (32), wherein the daily dose of the active ingredient on Treatment Day 1 is 6 mg and the daily dose of the active ingredient from Treatment Day 2 to Treatment Day 5 is 3 mg each.

(9') The complex containing the compound and the fumaric acid according to any one of (1') to (8'), wherein the administration is performed once daily.

(10') The complex containing the compound and the fumaric acid according to any one of (1') to (9'), being in an orally administered drug form.

[EFFECT OF THE INVENTION]

The complex containing the compound represented by Formula (I) and fumaric acid, contained in the pharmaceutical composition of the present invention, has an excellent effect of being effective for treating novel coronavirus infections (COVID-19). In addition, the complex containing the compound represented by Formula (I) and fumaric acid, contained in the pharmaceutical composition of the present invention is a highly safe drug which can maintain an effective concentration in blood without exceeding NOAEL by being administered to a human at a specific dosage regimen.

[BRIEF DESCRIPTION OF THE DRAWINGS]

Fig. 1 shows a powder X-ray diffraction pattern of a fumaric acid cocrystal Form I crystal (Form I) of the compound represented by Formula (I). The axis of abscissa represents 2θ(°), and the axis of ordinate represents the intensity (Count).

Fig. 2 shows a molecular structure in an asymmetric unit of the fumaric acid cocrystal Form I crystal (Form I) of the compound represented by Formula (I).

Fig. 3 shows a simulation of plasma drug concentration changes at various doses. The vertical axis represents the simulated drug concentration in plasma. The horizontal axis represents the elapsed time from the first administration of the drug.

Fig. 4 shows the change from baseline in SARS-CoV-2 virus titer at each time point in mild/moderate and asymptomatic SARS-CoV-2-infected patients (modified intention-to-treat [mITT] population) in Phase 2a Part. The vertical axis represents the change from baseline in virus titer ($\log_{10}$ ($TCID_{50}$/mL)), and the horizontal axis represents an evaluation time point.

Fig. 5 shows the time to the first negative SARS-CoV-2 virus titer in mild/moderate and asymptomatic SARS-CoV-2-infected patients (mITT population) in Phase 2a Part. The vertical axis represents the proportion (unit: %) of patients with the negative SARS-CoV-2 virus titer. The horizontal axis represents the time (unit: hour) from the start of intervention.

Fig. 6 shows the change from baseline in the total score of 12 COVID-19 symptoms at each time point in mild/moderate SARS-CoV-2-infected patients (intention-to-treat [ITT] population) in Phase 2a Part. The vertical axis represents the change from baseline in the total score of 12 COVID-19 symptoms. The horizontal axis represents the number of days from the start of intervention.

Fig. 7 shows the time to resolution of five COVID-19 in mild/moderate SARS-CoV-2-infected patients (part of the ITT population whose time from onset of COVID-19 to randomization was less than 72 hours) in Phase 3 Part. The vertical axis represents the proportion (unit: %) of subjects who were resolved from the five COVID-19 symptoms. The horizontal axis represents the time (unit: hour) from the start of treatment.

Fig. 8 shows the time to resolution of five COVID-19 symptoms in mild/moderate SARS-CoV-2-infected patients (ITT population) in Phase 3 Part. The vertical axis represents the proportion (unit: %) of subjects who were resolved from the five COVID-19 symptoms. The horizontal axis represents the time (unit: hour) from the start of treatment.

Fig. 9 shows the time to the first negative SARS-CoV-2 virus titer in mild/moderate SARS-CoV-2-infected patients (part of the mITT population whose time from onset of COVID-19 to randomization was less than 72 hours) in Phase 3 Part. The vertical axis represents the proportion (unit: %) of patients with the negative SARS-CoV-2 virus titer. The horizontal axis represents the time (unit: hour) from the start of treatment.

Fig. 10 shows the time to the first negative SARS-CoV-2 virus titer in mild/moderate SARS-CoV-2-infected patients (mITT population) in Phase 3 Part. The vertical axis represents the proportion (unit: %) of patients with the negative SARS-CoV-2 virus titer. The horizontal axis represents the time (unit: hour) from the start of treatment.

Fig. 11 shows the time to resolution of 12 COVID-19 symptoms in mild/moderate SARS-CoV-2-infected patients (part of the ITT population whose time from onset of COVID-19 to randomization was less than 72 hours) in Phase 3 Part. The vertical axis represents the proportion (unit: %) of subjects who were resolved from the 12 COVID-19 symptoms. The horizontal axis represents the time (unit: hour) from the start of treatment.

Fig. 12 shows the time to resolution of 14 COVID-19 symptoms in mild/moderate SARS-CoV-2-infected patients (part of the ITT population whose time from onset of COVID-19 to randomization was less than 72 hours) in Phase 3 Part. The vertical axis represents the proportion (unit: %) of subjects who were resolved from the 14 COVID-19 symptoms. The horizontal axis represents the time (unit: hour) from the start of treatment.

[MODE FOR CARRYING OUT THE INVENTION]

The term "consist of" means having only the constituent elements. The term "comprise" means that elements are not limited to the constituent elements, and elements that are not described are not excluded.

Hereinafter, the present invention will be described while showing exemplary embodiments. Throughout the present specification, it should be understood that, unless particularly stated otherwise, an expression of a singular form also includes the concept of a plural form thereof. Therefore, it should be understood that, unless particularly stated otherwise, an article for a singular form (for example, in the case of English, "a", "an", "the", or the like) also includes the concept of a plural form thereof.

Furthermore, it should be understood that, unless particularly stated otherwise, the terms used in the present specification are used in the meanings normally used in the above-described art. Accordingly, unless otherwise defined, all terminologies and scientific and technical terms used in the present specification have the same meanings as commonly understood by those having ordinary skill in the art to which the present invention belongs. In a case of contradiction, priority is given to the present specification (including definitions).

The pharmaceutical composition for treating novel coronavirus infections (COVID-19) according to the present invention is characterized in that it is a pharmaceutical composition containing, as an active ingredient, a complex that contains:

a compound represented by Formula (1):

[Chemical Formula 6]

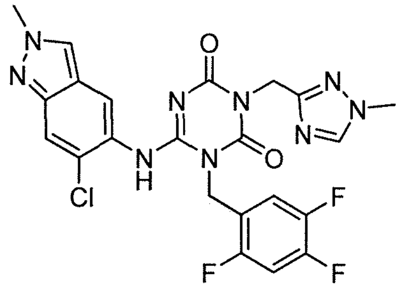

( I )

;

and

fumaric acid. In the present specification, the pharmaceutical composition for treating novel coronavirus infections (COVID-19) according to the present invention is also referred to as a medicament for novel coronavirus infections (COVID-19).

The compound represented by Formula (I) is (6E)-6-[(6-chloro-2-methyl-2H-indazol-5-yl)imino]-3-[(1-methyl-1H-1,2,4-triazol-3-yl)methyl]-1-[(2,4,5-trifluorophenyl)methyl]-1,3,5-triazinane-2,4-dione, and has a coronavirus 3CL protease inhibitory activity. In addition, the compound represented by Formula (I) includes the following tautomer.

[Chemical Formula 7]

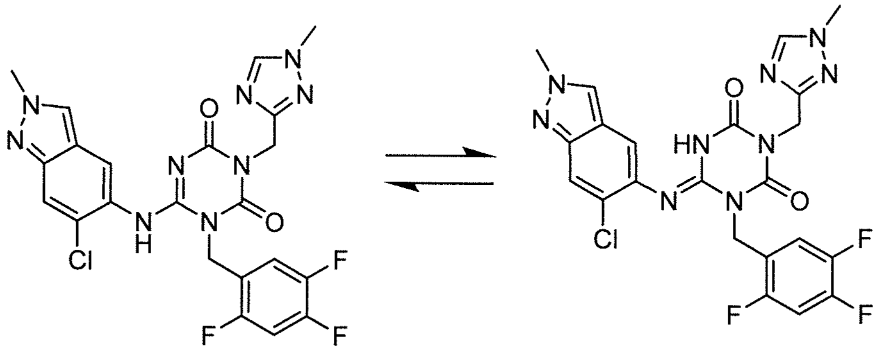

(I) (I)

The complex containing the compound represented by Formula (I) and fumaric acid may form a solvate (for example, hydrate, etc.), a cocrystal and/or a clathrate, and these are described as "complex" herein.

The "cocrystal" used herein means that counter molecules are regularly arranged in the same crystal lattice and may include any number of counter molecules. Furthermore, cocrystal implies that an intermolecular interaction between a compound and a counter molecule involves non-covalent and non-ionic chemical interaction such as hydrogen bonding and van der Waals force.

For example, the cocrystal of the compound represented by Formula (I) may be composed of the compound represented by Formula (I) and a counter molecule and may include any number of counter molecules. Preferably, the cocrystal may be composed of the compound represented by Formula (I) and fumaric acid and may include any number of fumaric acids. Further preferably, the cocrystal is a cocrystal composed of the compound represented by Formula (I) and fumaric acid at a molar ratio of 1 : 1.

A cocrystal is distinguished from a salt from the viewpoint that the compound is essentially uncharged or neutral.

The cocrystal is distinguished from hydrate or solvate from the viewpoint that the counter molecule is neither water nor a solvent.

"Crystal" as used in the present specification means a solid in which constituent atoms, ions, molecules, and the like are three-dimensionally arranged with regularity, and is distinguished from an amorphous solid that does not have such a regular internal structure. The crystal of the present invention may be a single crystal, a twin crystal, a polycrystal, or the like.

Furthermore, the "crystal" may include "crystalline polymorphs" that have the same composition but different arrangements in the crystal, and crystals including those crystalline polymorphs are referred to as "crystalline forms".

The crystalline form and the crystallinity can be measured by numerous technologies including, for example, powder X-ray diffraction measurement, Raman spectroscopy, infrared absorption spectrometry, water absorption and desorption measurement, differential scanning calorimetry, and dissolution characteristics.

Furthermore, a "crystalline polymorphism" may be formed by recrystallization of the complex containing the compound represented by Formula (I) and fumaric acid.

In the formulation of the present invention, such various salts, complexes (hydrate, solvate, cocrystal, and clathrate), and the crystalline polymorphism can be used, and a mixture of two or more kinds thereof can also be used.

(Powder X-ray diffraction (XRPD))

The X-ray powder diffraction (XRPD) is one of the most sensitive analytical methods for measuring the crystalline form and crystallinity of solid. When X-rays are irradiated on a crystal, the rays are reflected at crystal lattice planes, interfere with each other, and show well-ordered diffraction lines corresponding to the period of the structure. On the other hand, in an amorphous solid, a diffraction phenomenon does not occur because an amorphous solid usually does not have a well-ordered repetitive period in the structure, and an uncharacterized broad XRPD pattern (also called halo pattern) is exhibited.

The crystalline form of the compound represented by Formula (I) can be identified by the X-ray powder diffraction pattern and characteristic diffraction peaks. The crystalline form of the compounds represented by Formula (I) can be distinguished from the other crystalline form by the presence of characteristic diffraction peaks.

Characteristic diffraction peaks used in the present specification are peaks selected from an observed diffraction pattern. Characteristic diffraction peaks are preferably selected from about 10 peaks, more preferably about 5 peaks, and even more preferably about 3 peaks, in a diffraction pattern.

When distinguishing a plurality of crystals, a peak that is identified in the relevant crystal and is not identified in other crystals becomes a characteristic peak preferable for characterizing the crystal, rather than the intensity of a peak.

With such characteristic peaks, even one or two peaks can characterize the crystal. When the charts obtained by measurement are compared and these characteristic peaks are found to coincide, it can be said that the powder X-ray diffraction patterns substantially coincide.

Generally, since the diffraction angle (2θ) in powder X-ray diffraction may cause an error within the range of ±0.2°, it should be understood that a value of the diffraction angle of powder X-ray diffraction also includes numerical values within the range of about ±0.2°. Therefore, not only crystals in which the diffraction angles of peaks in powder X-ray diffraction perfectly coincide, but also crystals in which the diffraction angles of peaks coincide with an error of about ±0.2°, are included in the present invention.

It is known that generally, the intensity of a peak indicated in the following tables and drawings may fluctuate due to many factors, for example, the effect of selective orientation of crystals with respect to an X-ray beam, the influence of coarse particles, the purity of the substance to be analyzed, or the crystallinity of a sample. Furthermore, the peak position can also be shifted based on the fluctuation of the sample height. In addition, when the peak position is measured using different wavelengths, different shifts can be obtained according to Bragg equation ($n\lambda = 2d\sin\theta$); however, other XRPD patterns obtainable by using such other wavelengths are also included in the scope of the present invention.

(Single crystal structure analysis)

In one of the methods for characterizing a crystal, crystallographic parameters for the relevant crystal, as well as the atomic coordinates (values indicating the spatial positional relationship of each atom) and a three-dimensional structural model can be obtained. See "Guidance on X-ray Structural Analysis", written by Toshio Sakurai, published by Shokabo Co., Ltd. (1983); X-Ray Structure Determination: A Practical Guide, written by Stout & Jensen, Macmillan Co, New York (1968); and the like. When the crystal structures of a complex, a salt, an optical isomer, a tautomer, and a geometrical isomer such as the present invention are identified, single crystal structure analysis is useful.

The compound represented by Formula (I) has coronavirus 3CL protease inhibitory activity, and thus is useful as a therapeutic and/or prophylactic agent for diseases associated with the coronavirus 3CL protease. When the term "therapeutic agent and/or prophylactic agent" is used in the present invention, this also includes a symptom ameliorating agent. The disease associated with coronavirus 3CL proteases may be virus infections, and preferably coronavirus infections.

According to an aspect, the coronavirus may be a coronavirus that infects human beings. The coronavirus that infects human beings may be HCoV-229E, HCoV-NL63, HCoV-HKU1, HCoV-OC43, SARS-CoV, MERS-CoV, and/or SARS-CoV-2.

As an embodiment, as the coronavirus, Alphacoronavirus and/or Betacoronavirus, more preferably Betacoronavirus, and further preferably Sarbecovirus are exemplified.

According to an aspect, the alphacoronavirus may be HCoV-229E and HCoV-NL63. The alphacoronavirus may be particularly preferably HCoV-229E.

According to an aspect, the betacoronavirus may be HCoV-HKU1, HCoV-OC43, SARS-CoV, MERS-CoV, and/or SARS-CoV-2. The betacoronavirus may be HCoV-OC43 or SARS-CoV-2, and particularly preferably SARS-CoV-2.

According to an aspect, the betacoronavirus may be betacoronavirus lineage A (B-coronavirus lineage A), betacoronavirus lineage B (B-coronavirus lineage B), and betacoronavirus lineage C (B-coronavirus lineage C). The betacoronavirus may be more preferably betacoronavirus lineage A (β-coronavirus lineage A) and betacoronavirus lineage B (β-coronavirus lineage B) and particularly preferably betacoronavirus lineage B (β-coronavirus lineage B).

Examples of the betacoronavirus lineage A (B-coronavirus lineage A) include HCoV-HKU1 and HCoV-OC43, and preferably HCoV-OC43. Examples of the betacoronavirus lineage B (B-coronavirus lineage B) include SARS-CoV and SARS-CoV-2, and preferably SARS-CoV-2. The betacoronavirus lineage C (β-coronavirus lineage C) may be MERS-CoV.

According to an aspect, the coronavirus may be HCoV-229E, HCoV-OC43, and/or SARS-CoV-2, and particularly preferably SARS-CoV-2.

The coronavirus infections may be infections caused by HCoV-229E, HCoV-NL63, HCoV-OC43, HCoV-HKU1, SARS-CoV, MERS-CoV, and/or SARS-CoV-2. Preferably, the coronavirus infections may be infections caused by HCoV-229E, HCoV-OC43, and/or SARS-CoV-2, and particularly preferably infection caused by SARS-CoV-2.

The coronavirus infections may be particularly preferably novel coronavirus infections (COVID-19).

The classification of the degree of severity of novel coronavirus infected patients is exemplified below, for example. (Reference: Coronavirus disease 2019 (COVID-19) Guidance of medical examination 6.0 Edition (Ministry of Health, Labour and Welfare))

(Mild illness)

The oxygen saturation is 96% or more. In the clinical state, there is no respiratory symptom or there is only

coughing without dyspnea, and in any cases, a finding of pneumonia is not recognized.

(Moderate illness I)

The oxygen saturation is less than 96% to more than 93%. There is dyspnea, and a finding of pneumonia is recognized.

(Moderate illness II)

The oxygen saturation is 93% or less. There is respiratory failure, and oxygen administration is required.

(Severe illness)

A patient is in ICU or requires an inhalator.

The above classification is based on definition of the degree of severity in Japan, and for example, the classification of the degree of severity in China or U.S. NIH can be employed.

Furthermore, an asymptomatic SARS-CoV-2-infected patient (asymptomatic patient) means a patient having an asymptomatic pathogen. For example, patients who do not have fourteen COVID-19 symptoms recognized are exemplified.

(Fourteen COVID-19 symptoms: low energy (fatigue); pains in muscles or body; headache; chills/sweats; feverishness or fever; dysgeusia; dysosmia; runny nose or stuffy nose; sore throat; coughing; shortness of breath (dyspnea); nausea; emesis; and diarrhea)

Herein, 12 COVID-19 symptoms include low energy (fatigue), pains in muscles or body, headache, chills/sweats, feverishness or fever, runny nose or stuffy nose, sore throat, coughing, shortness of breath (dyspnea), nausea, emesis, and diarrhea. The five COVID-19 symptoms include runny nose or stuffy nose, sore throat, coughing, feverishness or fever, and low energy (feeling of fatigue).

Herein, the exacerbation suppression means that the symptom of an asymptomatic SARS-CoV-2-infected patient is suppressed from having a greater degree of severity, such as mild illness, moderate illness I, moderate illness II, or severe illness.

Herein, the exacerbation suppression means that the symptom of an asymptomatic SARS-CoV-2-infected patient or a SARS-CoV-2-infected patient having mild illness is suppressed from having a greater degree of severity, such as moderate illness I, moderate illness II, or severe illness.

Herein, the exacerbation suppression means that the symptom of an asymptomatic SARS-CoV-2-infected patient, a SARS-CoV-2-infected patient having mild illness, or a SARS-CoV-2-infected patient having moderate illness I is suppressed from having a greater degree of severity, such as moderate illness II, or severe illness.

Herein, the exacerbation suppression means that the symptom of an asymptomatic SARS-CoV-2-infected patient, a SARS-CoV-2-infected patient having mild illness, a SARS-CoV-2-infected patient having moderate illness I, or a SARS-CoV-2-infected patient having moderate illness II is suppressed from having a greater degree of severity, such as severe illness.

Herein, the exacerbation suppression means that the hospitalization or death risk of a SARS-CoV-2-infected patient is decreased through the virus replication suppression effect of the present agent.

Herein, the exacerbation suppression means that the inflammation in the lung of a SARS-CoV-2-infected patient is alleviated through the virus replication suppression effect of the present agent.

Herein, the exacerbation suppression means that pneumonia caused by virus infection of SARS-CoV-2 is suppressed through the virus replication suppression effect of the present agent.

Herein, the exacerbation suppression means that overactive immune response of a host caused by virus infection of SARS-CoV-2 is suppressed through the virus replication suppression effect of the present agent.

Herein, the exacerbation suppression means the reduction of the deterioration rate regarding the Ordinal Scale (an ordinal scale that classifies clinical severity into eight grades). Specifically, it means that deterioration of the Ordinal Scale to 3 or more for the first time after the start of treatment is suppressed.

Herein, the shortening of the disease duration means that the time to resolution or improvement is shortened, and indirectly includes an improvement in the total score of the COVID-19 symptoms (14 symptoms).

Herein, the shortening of the disease duration means that the time to resolution or improvement is shortened, and indirectly includes an improvement in the total score of the COVID-19 symptoms (12 symptoms).

Herein, the shortening of the disease duration means that the time to resolution or improvement is shortened, and indirectly includes an improvement in the total score of the COVID-19 symptoms (five symptoms).

As an embodiment, the pharmaceutical composition of the present invention is administered to an infected patient having at least one of exacerbation risk factors and used for suppressing the exacerbation of COVID-19 symptoms.

As an embodiment, the pharmaceutical composition of the present invention is administered to an infected patient having at least one of exacerbation risk factors shown below among SARS-CoV-2-infected patients.

- 50 years old or older
- Obesity (BMI 30 $kg/m^2$ or more)
- Cardiovascular disease (including cardiovascular disease including high-blood pressure and congenital cardiac disease)
- Chronic pulmonary disease (including asthma and interstitial pulmonary disease)
- Type 1 or type 2 diabetes
- Chronic renal impairment (including dialyzed patients)
- Chronic hepatic disease
- Immunosuppressed state (e.g.: malignancy treatment, bone marrow or organ transplantation, immune deficiency, uncontrolled HIV, AIDS, sickle-cell anemia, thalassemia, and long-term administration of an immunosuppressant)
- Chronic obstructive pulmonary disease (COPD)
- Hyperlipidemia
- Smoking
- Immune deficiency after solid organ transplantation
- Pregnancy
- Patient having a neurodevelopmental disease or complicated clinical conditions (e.g.: cerebral palsy and congenital disease)
- Patient having a high degree of medical dependence (e.g.: tracheostomy, gastric fistula, and positive pressure ventilation)

As an embodiment, the pharmaceutical composition of the present invention is used for a patient having pneumonia caused by SARS-CoV-2.
As an embodiment, the pharmaceutical composition of the present invention is administered to an infected patient having at least one of clinical states shown below among SARS-CoV-2-infected patients.

- Patient with installation of extracorporeal membrane oxygenation (ECMO)
- Patient with installation of inhalator
- Patient in ICU
- Patient having an oxygen saturation ($SpO_2$) of 93% (room air) or less, or requiring oxygen inhalation

As an embodiment, the pharmaceutical composition of the present invention is administered to an infected patient having at least one of clinical states shown below among SARS-CoV-2-infected patients.

- Oxygen saturation (SpO2) of less than 94% (room air, sea level)
- PaO2/FiO2 of less than 300 mmHg
- Respiration rate of 30 or more/min
- Pulmonary infiltration of 50% or more

(Production method of compound represented by Formula (I))

The compound represented by Formula (I) can be produced, for example, by a synthesis method described below. Regarding extraction, purification, and the like, the treatments carried out in ordinary experiments of organic chemistry may be carried out. The compound can be synthesized with reference to methods known in the art. Regarding extraction, purification, and the like, the treatments carried out in ordinary experiments of organic chemistry may be carried out.
The compound represented by Formula (I) can be produced with reference to techniques known in the art. The compound can be produced, for example, with reference to WO 2010092966, WO 2012020749, WO 2013089212, WO 2014200078, WO 2012020742, WO 2013118855, and the like.

(Method for identifying compound)

NMR analysis was performed by 400 MHz using DMSO-$d_6$ and CDCls. Furthermore, when NMR data are shown, there are occasions in which all the measured peaks are not described.
The term RT in the specification indicates retention time in an LC/MS: liquid chromatography / mass analysis, and the retention time was measured under the following conditions.

(Measurement conditions 1)

Column: ACQUITY UPLC (registered trademark) BEH C18 (1.7 pm i.d. 2.1 × 50 mm) (Waters)
Flow rate: 0.8 mL/min
UV detection wavelength: 254 nm
Mobile phase: [A] was 0.1% formic acid-containing aqueous solution, and [B] was 0.1% formic acid-containing acetonitrile solution.
Gradient: A linear gradient of 5% to 100% solvent [B] was carried out for 3.5 minutes, and then 100% solvent [B] was maintained for 0.5 minutes.

(Measurement of powder X-ray diffraction pattern)

Powder X-ray diffraction measurement of crystals obtained was performed according to the powder X-ray diffraction measurement method described in the General Testing Methods of the Japanese Pharmacopoeia. Measurement conditions are shown below.

(Apparatus)

SmartLab manufactured by Rigaku Corporation
(Operation method)
Measurement method: Reflection method
Wavelength used: CuKa radiation
Tube current: 200 mA
Tube voltage: 45 kV
Sample plate: Aluminum
Incident angle of X-rays: 2.5°
Sampling width: 0.02°
Detector: HyPix-3000 (two-dimensional detection mode)

(Measurement and analysis method for single crystal structure analysis)

The measurement conditions and analysis method for single crystal structure analysis will be described below.

(Apparatus)

XtaLAB P200 MM007 manufactured by Rigaku Corporation
(Measurement conditions)
Measured temperature: 25°C
Wavelength used: CuKa radiation ($\lambda$ = 1.5418Å)
Software: CrysAlisPro 1.171.39.46e (Rigaku Oxford Diffraction, 2018)
(Data processing)
Software: CrysAlisPro 1.171.39.46e (Rigaku Oxford Diffraction, 2018)
The data were subjected to Lorentz and polarization correction and absorption correction.

(Crystal structure analysis)

Phase determination was performed using a direct method program, ShelXT (Sheldrick, G. M., 2015), and regarding refinement, a full-matrix least squares method was carried out using ShelXL (Sheldrick, G. M., 2015). The temperature factors of non-hydrogen atoms were all subjected to refinement with anisotropy. Hydrogen atoms were computationally introduced using the default parameters of ShelXL and were treated as riding atoms. All the hydrogen atoms were subjected to refinement with isotropic parameters.
Fig. 2 was made using PLUTON (Spek, 1991)/ORTEP (Johnson, 1976).

Exemplary synthesis of the compound represented by formula (I) is shown below.

[Chemical Formula 8]

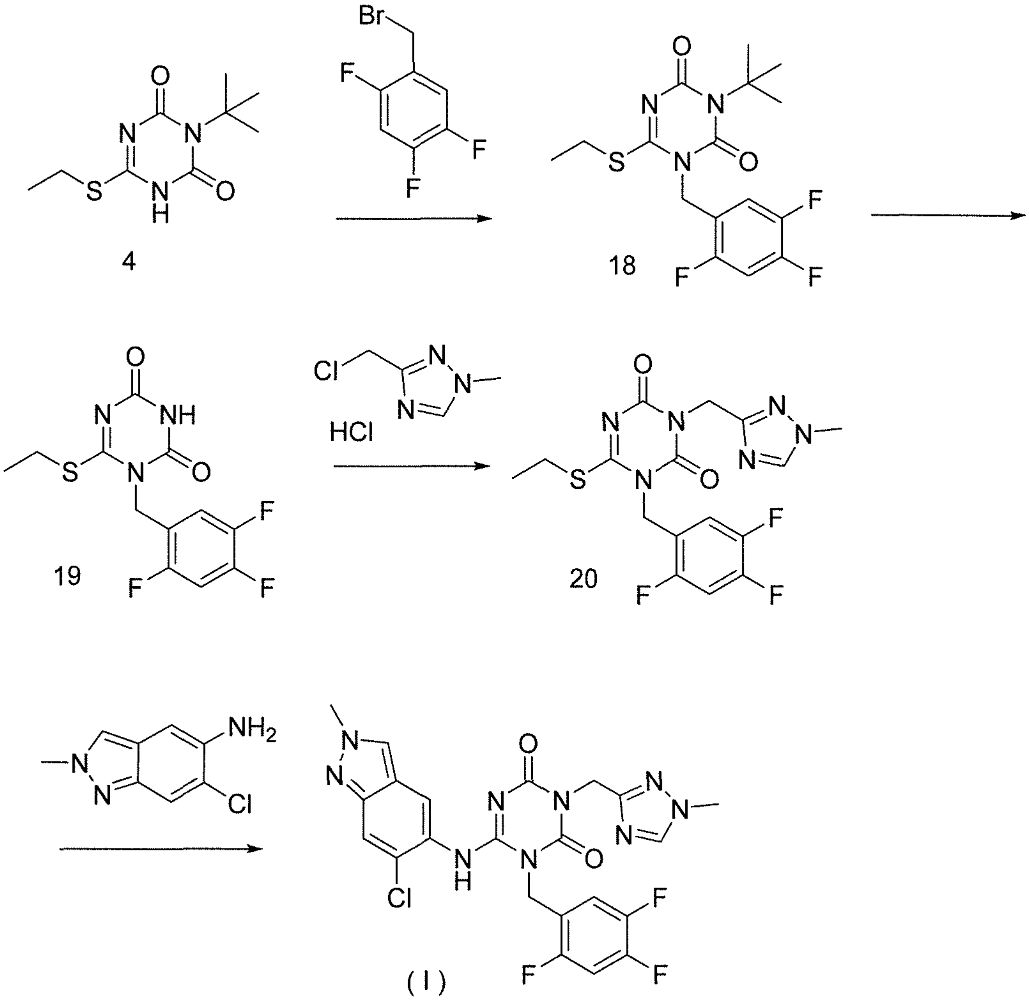


Step 1 Synthesis of Compound 18

Compound 4 (926mg, 4.04mmol), acetonitrile (7.41mL), potassium carbonate (726mg, 5.25mmol) and 2,4,5-trifluorobenzyl bromide (1000mg, 4.44mmol) were mixed. The reaction solution was stirred at 80 °C for 40 minutes, allowed to cool, and then diluted with ethyl acetate. After filtration of the insoluble material, the filtrate was concentrated to give the crude product of Compound 18 (1.51g, 4.04mmol, yield : quant.)
LC/MS (ESI): m/z = 374, RT = 2.54min, LC/MS measured condition 1

Step 2 Synthesis of Compound 19

Compound 18 (1.51g, 4.04mmol) and TFA (3.02mL) were mixed. The reaction solution was stirred at room temperature for 4 hours and allowed to stand overnight. TFA was distilled off under reduced pressure, and then toluene was added to the residue and azeotroped. Isopropyl Ether was added to the residue, suspended, and collected by filtration to give Compound 19 (1.22g, 3.84mmol, yield : 95%).
LC/MS (ESI): m/z = 318, RT = 1.68min, LC/MS measured condition 1

Step 3 Synthesis of Compound 20

Compound 19 (200 mg, 0.63 mmol), DMF (1.8 mL), potassium carbonate (261 mg, 1.89 mmol), and 3-(chloromethyl)-1-methyl-1H-1,2,4-triazole hydrochloride (159 mg, 0.946 mmol) were mixed. The reaction solution was stirred for 2 hours at 60 ° C., and a saturated aqueous ammonium chloride solution was added. The aqueous layer was extracted

with ethyl acetate and the organic layer was washed with brine. The organic layer was dried over magnesium sulfate, filtered and concentrated. The residue was suspended in a mixed solvent of isopropyl ether, hexane, ethyl acetate and chloroform and collected by filtration. The residue, DMF (1.8 mL), potassium carbonate (261 mg, 1.89 mmol), and 3-(chloromethyl)-1-methyl-1H-1,2,4-triazole hydrochloride (159 mg, 0.946 mmol) were mixed. The reaction solution was stirred at 60 °C for 6 hours, and a saturated aqueous ammonium chloride solution was added. The aqueous layer was extracted with ethyl acetate and the organic layer was washed with brine. The organic layer was dried over magnesium sulfate, filtered and concentrated. The residue was suspended in a mixed solvent of isopropyl ether, hexane, ethyl acetate and chloroform and collected by filtration to give Compound 20 (116mg, 0.281mmol, 45% yield)
LC/MS (ESI): m/z = 413, RT = 1.84min, LC/MS measured condition: 1

Step 4 Synthesis of compound represented by Formula (I)

Compound 20 (115mg, 0.279mmol), THF (2.30mL) and 6-chloro-2-methyl-2H-indazole-5-amine (60.8mg, 0.335mmol) were mixed. The reaction mixture was added dropwise LHMDS (558μL, 0.558mmol) at 0°C. The reaction mixture was stirred at 0°C for 2.5 hours and stirred at room temperature for 40 minutes, then saturated ammonium chloride solution was added to the reaction mixture. The reaction mixture was extracted with chloroform and the organic layer was concentrated. The residue was purified by silicagel column chromatography (chloroform/methanol) to give the compound represented by Formula (I) (61.8mg, 0.116mmol, Yield 42%).

$^1$H-NMR ($CDCl_3$) δ: 7.96 (s, 1H), 7.82 (d, J = 2.5Hz, 2H), 7.48 (br s, 1H), 7.45-7.37 (m, 1H), 7.08 (s, 1H), 6.97-6.88 (m, 1H), 5.35 (s, 2H), 5.17 (s, 2H), 4.21 (s, 3H), 3.89 (s, 3H).
LC/MS(ESI): m/z = 532, RT = 1.70 min, LC/MS measurement method 1

To the compound represented by Compound (I), 1170mg, were added fumaric acid (278mg, 1.1 eq) and ethyl acetate(5.85mL). The mixture was stirred at room temperature for 45 minutes. The resulting solids were collected by filtration and dried to give fumaric acid cocrystal Form I crystal of the compound represented by Formula (I) (1369.4mg, 94.6%).

The results of the single crystal structure analysis of the fumaric acid cocrystal Form I crystal of the compound represented by Formula (I) are shown below.

R1 (I> 2.00s (I)) was 0.0470, and it was confirmed from the final difference Fourier that there was no lack of electron density or misplacement.

Crystallographic data are shown in Table 1.

[Table 1]

| | |
|---|---|
| Space Group | P-1 |
| a (Å) | 8.4374(2) |
| b (Å) | 11.6780(3) |
| c (Å) | 15.1612(4) |
| α (°) | 83.827(2) |
| β (°) | 78.868(2) |
| γ (°) | 77.147(2) |
| Volume (Å$^3$) | 1425.77(6) |
| Z | 2 |
| Density (calculated value) (g/cm$^3$) | 1.509 |
| Measured temperature (K) | 298 |

wherein Volume indicates the unit lattice volume, Z indicates chemical unit number per unit cell.

Furthermore, the atomic coordinates of non-hydrogen atoms are indicated in Tables 2 and 3. Here, U(eq) means an equivalent isotropic temperature factor.

Fractional Atomic Coordinates ($\times 10^4$) and Equivalent Isotropic Displacement Parameters (Å$^2 \times 10^3$). U(eq) is defined as 1/3 of the trace of the orthogonalized Uij tensor.

[Table 2]

| Atom | x | y | z | U (eq) |
|---|---|---|---|---|
| Cl36 | 8115.3(9) | 8341.6(8) | 5010.7(5) | 79.9(3) |
| F32 | 8958.5(19) | 7981.3(17) | 307.5(9) | 78.5(5) |
| O35 | 7267(2) | 5961.4(16) | 1399.9(10) | 56.3(5) |
| O34 | 5322(3) | 4254.8(16) | 4098.2(11) | 63.3(5) |
| O38 | 3536(2) | 9367.5(19) | 8936.3(12) | 64.2(5) |
| N12 | 6506(2) | 7056.8(18) | 2611.0(12) | 44.2(5) |
| F33 | 13870(2) | 7642(2) | 1402.1(13) | 100.3(7) |
| N16 | 5475(2) | 6174.4(18) | 3988.1(12) | 48.2(5) |
| N14 | 6120(3) | 5115.3(18) | 2713.0(12) | 47.3(5) |
| N9 | 2815(3) | 8924(2) | 7397.8(13) | 55.4(6) |
| N10 | 5772(3) | 8146(2) | 3856.1(13) | 55.1(6) |
| N1 | 1276(3) | 8864(2) | 7324.6(14) | 60.2(6) |
| F31 | 12197(3) | 7751(3) | 3084.6(13) | 124.9(9) |
| N23 | 3644(3) | 4434(2) | 1818.7(15) | 64.5(6) |
| N20 | 3122(3) | 4249(2) | 1061.4(15) | 64.9(6) |
| C11 | 6673(3) | 6043(2) | 2193.8(15) | ’44.7(6) |
| C9 | 5879(3) | 7178(2) | 3527.6(15) | 44.2(6) |
| C10 | 5619(3) | 5119(2) | 3639.3(15) | 48.4(6) |
| N22 | 5784(3) | 3621(2) | 814.1(15) | 67.8(7) |
| O39 | 6151(3) | 8893(3) | 8285.8(15) | 109.2(10) |
| C12 | 6985(3) | 8068(2) | 2049.4(15) | 47.2(6) |
| C20 | 5248(3) | 4044(2) | 1633.9(16) | 50.7(6) |
| C7 | 5022(3) | 8298(2) | 4770.9(15) | 50.8 (6) |
| C4 | 3693(3) | 8762(2) | 6554.3(16) | 49.4(6) |
| C13 | 8823(3) | 7976(2) | 1872.6(16) | 48.8(6) |
| C5 | 5385(3) | 8700(2) | 6267.8(17) | 56.4(7) |
| C19 | 6380(3) | 4009(2) | 2279.5(17) | 54.5(7) |
| C14 | 9741(3) | 7934(2) | 1013.2(16) | 54.7(7) |
| C3 | 2685(3) | 8593(2) | 5965.2(17) | 54.4(7) |
| C6 | 6015(3) | 8469(2) | 5392.0(16) | 54.3(7) |
| C23 | 5121(4) | 9287(3) | 8898.3(18) | 62.1(7) |
| O41 | 1842(3) | 4874(3) | 3529.1(18) | 119.8(10) |
| C8 | 3370(3) | 8376(2) | 5054.8(17) | 57.4(7) |
| C24 | 5542(3) | 9730(3) | 9679.7(17) | 61.9(7) |
| C18 | 9684(4) | 7917(3) | 2570.7(18) | 67.1(8) |
| C15 | 11431(3) | 7827(3) | 831.3(19) | 67.8(8) |
| C16 | 12217(3) | 7760(3) | 1541(2) | 67.9(8) |
| C2 | 1134(4) | 8667(3) | 6497.1(18) | 67.4(8) |

[Table 3]

| Atom | x | y | z | U(eq) |
|---|---|---|---|---|
| C17 | 11360(4) | 7806(3) | 2405(2) | 75.0(9) |
| C21 | 4400(4) | 3767(3) | 485.7(19) | 70.6(8) |
| O43 | -464(4) | 4618(4) | 3203.2(19) | 154.2(15) |
| C1 | 9(4) | 8943(3) | 8139(2) | 81.7(10) |
| C26 | 307(4) | 4766(4) | 3745(2) | 93.6(12) |
| C25 | -384(4) | 4909(4) | 4700(2) | 92.1(11) |
| C22 | 1397(4) | 4562(4) | 963(3) | 102.7(13) |

Next, the atomic coordinates of hydrogen atoms are shown in Table 4. Here, U(iso) means the isotropic temperature factor. Furthermore, the numbers of hydrogen atoms in Table 4 were assigned in relation to the numbers of non-hydrogen atoms that are bonded.

Hydrogen Atom Coordinates (Å $\times 10^4$) and Isotropic Displacement Parameters (Å $^2 \times 10^3$).

[Table 4]

| Atom | x | y | Z | U(iso) |
|---|---|---|---|---|
| H38 | 3370.9 | 9206.88 | 8452.86 | 96 |
| H16 | 5092.25 | 6215.55 | 4554.71 | 58 |
| H12A | 6452.59 | 8783.45 | 2347.49 | 57 |
| H12B | 6603.63 | 8119.01 | 1479.7 | 57 |
| H5 | 6053.99 | 8811.71 | 6658.45 | 68 |
| H19A | 6229.72 | 3381.57 | 2741.61 | 65 |
| H19B | 7509.94 | 3824.57 | 1962.58 | 65 |
| H41 | 2202.36 | 4700.41 | 3007.94 | 180 |
| H8 | 2702.01 | 8287.11 | 4656.27 | 69 |
| H24 | 6652.83 | 9619.42 | 9719.44 | 74 |
| H18 | 9115.24 | 7953.15 | 3160.4 | 81 |
| H15 | 12010.7 | 7800.84 | 243.55 | 81 |
| H2 | 176.44 | 8593.16 | 6310.6 | 81 |
| H21 | 4344.44 | 3553.57 | -79.51 | 85 |
| H1A | 260.69 | 8258.79 | 8539.89 | 122 |
| H1B | -1049.48 | 8985.26 | 7978.29 | 122 |
| H1C | -14.15 | 9635.57 | 8433.78 | 122 |
| H25 | -1486.76 | 4863.66 | 4886.06 | 110 |
| H22A | 719.4 | 4375.73 | 1521.91 | 154 |
| H22B | 1225.91 | 4127.33 | 499 | 154 |
| H22C | 1105.77 | 5390.24 | 801.98 | 154 |

Further, the interatomic bond length (unit: angstrom) is shown in Table 5.

[Table 5]

| Atom | Atom | Length/Å |
|---|---|---|
| Cl36 | C6 | 1.733(3) |

(continued)

| Atom | Atom | Length/Å |
|---|---|---|
| F32 | C14 | 1.352(3) |
| O35 | C11 | 1.216(3) |
| O34 | C10 | 1.208(3) |
| O38 | C23 | 1.310(3) |
| N12 | C11 | 1.369(3) |
| N12 | C9 | 1.398(3) |
| N12 | C12 | 1.465(3) |
| F33 | C16 | 1.347(3) |
| N16 | C9 | 1.373(3) |
| N16 | C10 | 1.365(3) |
| N14 | C11 | 1.382(3) |
| N14 | C10 | 1.386(3) |
| N14 | C19 | 1.466(3) |
| N9 | N1 | 1.342(3) |
| N9 | C4 | 1.358(3) |
| N10 | C9 | 1.262 (3) |
| N10 | C7 | 1.421(3) |
| N1 | C2 | 1.332(3) |
| N1 | C1 | 1.466(3) |
| F31 | C17 | 1.345(3) |
| N23 | N20 | 1.360(3) |
| N23 | C20 | 1.313(3) |
| N20 | C21 | 1.309(4) |
| N20 | C22 | 1.453(4) |
| N22 | C20 | 1.345(3) |
| N22 | C21 | 1.326(4) |
| 039 | C23 | 1.196(3) |
| C12 | C13 | 1.503(3) |
| C20 | C19 | 1.485(4) |
| C7 | C6 | 1.431(4) |
| C7 | C8 | 1.362(4) |
| C4 | C5 | 1.398(4) |
| C4 | C3 | 1.402(3) |
| C13 | C14 | 1.381(3) |
| C13 | C18 | 1.383(4) |
| C5 | C6 | 1.364(3) |
| C14 | C15 | 1.379(4) |
| C3 | C8 | 1.416(3) |
| C3 | C2 | 1.388(4) |

(continued)

| Atom | Atom | Length/Å |
|---|---|---|
| C23 | C24 | 1.475(4) |
| O41 | C26 | 1.304(4) |
| C24 | C24[1] | 1.307(5) |
| C18 | C17 | 1.367(4) |
| C15 | C16 | 1.355(4) |
| C16 | C17 | 1.370(4) |
| O43 | C26 | 1.189(4) |
| C26 | C25 | 1.466(5) |
| C25 | C25[2] | 1.273(7) |

In the fumaric acid cocrystal Form I crystal of the compound represented by Formula (I), one molecule of the compound represented by Formula (I) and one molecule of fumaric acid were present in the asymmetric unit. A molecular structure obtained from X-ray structure analysis of the fumaric acid cocrystal Form I crystal of the compound represented by Formula (I) is shown Fig. 2.

The numbers of non-hydrogen atoms in Tables 2, 3, and 5 correspond to the numbers shown in Fig. 2, respectively.

As shown in Table 5, the bond length of N10-C9 was about 1.26 Å, and the bond length of N16-C9 was about 1.37 Å.

Since the bond length of N10-C9 (about 1.26 Å) is shorter than that of N16-C9 (about 1.37 Å), the fumaric acid cocrystal Form I of the compound represented by Formula (I) was identified as imino structure.

[Chemical Formula 9]

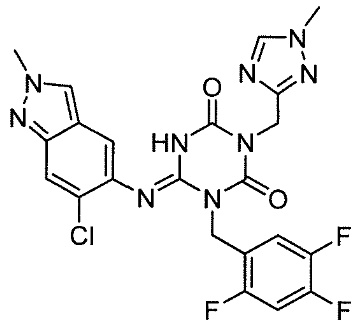

Further, the result of the powder X-ray diffraction of the fumaric acid cocrystal Form I crystal of the compound represented by Formula (I) is shown.

In the powder X-ray diffraction pattern, peaks were observed at the diffraction angle (2θ): 7.8±0.2°, 9.5±0.2°, 10.1±0.2°, 10.9±0.2°, 13.8±0.2°, 14.7±0.2°, 18.6±0.2°, 22.6±0.2°, 23.5±0.2° and 24.6±0.2°.

In the above-described X-ray powder diffraction pattern, the peaks of the diffraction angle (2θ): 9.5 ± 0.2°, 10.9 ± 0.2°, 18.6 ± 0.2°, 23.5 ± 0.2°, and 24.6 ± 0.2° are particularly characteristic as the fumaric acid cocrystal Form I crystal of the compound represented by Formula (I).

The compound represented by Formula (I) has coronavirus 3CL protease inhibitory activity, and thus is useful as a therapeutic and/or prophylactic agent for virus infection.

Furthermore, the compound represented by Formula (I) has utility as a medicament, and preferably, the compound of the present invention has any one or a plurality of the following excellent features.

a) Inhibitory action against CYP enzymes (e.g., CYP1A2, CYP2C9, CYP2C19, CYP2D6, and CYP3A4) is weak.
b) Satisfactory pharmacokinetics such as high bioavailability and moderate clearance are exhibited.
c) Metabolic stability is high.
d) Irreversible inhibitory action is not exhibited against CYP enzymes (e.g., CYP3A4) within the concentration range of the measurement conditions described in the present specification.
e) Mutagenicity is not exhibited.

f) The cardiovascular risk is low.
g) The compound shows high solubility.
h) The compound shows a high protein unbinding ratio (fu value).
i) The compound has high coronavirus 3CL protease selectivity.
j) The compound has high coronavirus proliferation inhibitory activity. For example, the compound has high coronavirus proliferation inhibitory activity with addition of human serum (HS) or human serum albumin (HSA).

Examples of a coronavirus proliferation inhibitor include embodiments, for example, having $EC_{50}$ of 10 μM or less, preferably 1 μM or less, and more preferably 100 nM or less, for example, in a CPE suppression effect confirmation test (SARS-CoV-2) described below.

Furthermore, the crystal complex (cocrystal) of the compound represented by Formula (I) has usefulness as a pharmaceutical preparation and has preferably any or a plurality of the following superior properties.

A) It shows excellent pharmacokinetics such as high bioavailability, moderate clearance, high AUC, or high maximum drug concentration.
B) It shows high solubility, high chemical stability, and low moisture absorbency.

The pharmaceutical composition of the present invention can be administered orally or parenterally. Examples of a method of parenteral administration include dermal, subcutaneous, intravenous, intraarterial, intramuscular, intraperitoneal, transmucosal, inhalation, transnasal, ophthalmic, inner ear, or vaginal administration.

In the case of oral administration, the pharmaceutical composition may be prepared into any dosage form that is commonly used, such as a solid preparation for internal use (e.g., a tablet, a powder, a granule, a capsule, a pill, a film, or the like) or a liquid preparation for internal use (e.g., a suspension, an emulsion, an elixir, a syrup, a lemonade, a spirit, an aromatic water, an extract, a decoction, a tincture, or the like), and administered. The tablet may be a sugar-coated tablet, a film-coated tablet, an enteric-coated tablet, a sustained-release tablet, a troche tablet, a sublingual tablet, a buccal tablet, a chewable tablet, or an orally disintegrating tablet; the powder and the granule may be a dry syrup; and the capsule may be a soft capsule, a micro capsule, or a sustained-release capsule.

In the case of parenteral administration, the pharmaceutical composition can be suitably administered in any dosage form that is commonly used, such as an injection, an infusion, or a preparation for external use (e.g., an eye drop, a nasal drop, an ear drop, an aerosol, an inhalant, a lotion, an impregnating agent, a liniment, a gargling agent, an enema, an ointment, a plaster, a jelly, a cream, a patch, a poultice, a powder for external use, a suppository, or the like). The injectable preparation may be an O/W, W/O, O/W/O, or W/O/W type emulsion, or the like.

The pharmaceutical composition can be produced by mixing an effective amount of the compound represented by Formula (I) with various pharmaceutical additives suitable for forms of the formulation, such as excipients, binders, disintegrants, and lubricants, as necessary. Further, the pharmaceutical composition can also be used for pediatric patients, geriatric patients, serious cases, or operations by appropriately changing the effective amount of the compound represented by Formula (I), formulation and/or various pharmaceutical additives. For example, a pharmaceutical composition for use for a child may be administered to a neonate (less than 4 weeks after birth), an infant (from 4 weeks after birth to less than 1 year), a preschool child (from 1 year to less than 7 years), a child (from 7 years to less than 15 years), or a patient 15 year to 18 years of age. For example, a pharmaceutical composition for an elderly may be administered to a patient 65 years of age or older.

Although it is desirable to set the dose of the pharmaceutical composition of the present invention (the pharmaceutical composition containing fumaric acid cocrystal Form I crystal of the compound represented by Formula (I)) in consideration of the age and body weight of the patient, disease type and degree, administration route, and the like, the dose in the case of oral administration is within the range of usually 0.05 to 200 mg/kg/day and preferably 0.1 to 100 mg/kg/day. In the case of parenteral administration, the amount of administration may vary greatly depending on the route of administration; however, the amount of administration is usually 0.005 to 200 mg/kg/day and is preferably in the range of 0.01 to 100 mg/kg/day. This may be administered once a day or several times a day.

For example, for a child having a body weight of 40 kg or more and an age of 6 years or more and less than 12 years, a child having an age of 12 years or more, and an adult, 375 mg of the compound represented by Formula (I) can be orally administered once a day on Treatment Day 1, and 125 mg of the same can be orally administered once a day from Treatment Day 2 to Treatment Day 5.

For example, for a child having a body weight of 30 kg or more and less than 40 kg and an age of 6 years or more and less than 12 years, 250 mg of the compound represented by Formula (I) can be orally administered once a day on Treatment Day 1, and 125 mg of the same can be orally administered once a day from Treatment Day 2 to Treatment Day 5.

For example, for a child having a body weight of 30 kg or more and less than 40 kg as well as an age of 6 years or more and less than 12 years, 250 mg of the compound represented by Formula (I) can be orally administered once a day on Treatment Day 1, and 100 mg of the same can be orally administered once a day from Treatment Day 2 to Treatment Day 5.

For example, for a child having a body weight of 20 kg or more and less than 30 kg as well as an age of 6 years or more and less than 12 years,125 mg of the compound represented by Formula (I) can be orally administered once a day on Treatment Day 1, and 75 mg of the same can be orally administered once a day from Treatment Day 2 to Treatment Day 5.

For example, for a child having a body weight of 20 kg or more and less than 30 kg as well as an age of 6 years or more and less than 12 years, 150 mg of the compound represented by Formula (I) can be orally administered once a day on Treatment Day 1, and 75 mg of the same can be orally administered once a day from Treatment Day 2 to Treatment Day 5.

For example, for a child having a body weight of 15 kg or more and less than 20 kg, 75 mg of the compound represented by Formula (I) can be orally administered once a day on Treatment Day 1, and 50 mg of the same can be orally administered once a day from Treatment Day 2 to Treatment Day 5.

For example, for a child having a body weight of 15 kg or more and less than 20 kg, 100 mg of the compound represented by Formula (I) can be orally administered once a day on Treatment Day 1, and 50 mg of the same can be orally administered once a day from Treatment Day 2 to Treatment Day 5.

For example, for a child having a body weight of 15 kg or more and less than 20 kg, 6 mg/kg of the compound represented by Formula (I) can be orally administered once a day on Treatment Day 1, and 3 mg/kg of the same can be orally administered once a day from Treatment Day 2 to Treatment Day 5.

The compound represented by Formula (I) may be used, for example, combining other medicaments for treating novel coronavirus infections (COVID-19) (as the therapeutic agents, including approved medicaments, and medicaments which are under development or will be developed in the future) (hereinafter, referred to as a concomitant medicament) for the purpose of enforcement of the activity of the compound or reduction of the dose of the compound or the like. In this case, timing of administration of the compound represented by Formula (I) and the concomitant medicament is not limited and these may be administered to the subject simultaneously or at regular intervals. Further, the compound represented by Formula (I) and the concomitant medicament may be administered as two or more kinds of different formulations containing each active ingredient or as a single formulation containing both active ingredients.

The amount of administration of the concomitant drug can be appropriately selected based on the clinically used dosage. Furthermore, the mixing ratio of the compound represented by Formula (I) and the concomitant medicament can be appropriately selected in consideration of the subject of administration, administration route, target diseases, symptoms, combinations, and the like. For example, when the subject of administration is human, the concomitant medicament may be used in the range of 0.01 to 100 parts by weight with respect to 1 part by weight of the compound represented by Formula (I).

As illustrated in the Examples below, the present invention provides methods of treating novel coronavirus infections (COVID-19) and/or alleviating symptoms caused by novel coronavirus infections (COVID-19).

The present invention also has the advantage of producing substantially no side effects when the pharmaceutical composition of the present invention is administered to a human.

Furthermore, the present invention provides a method of reducing symptoms (Long COVID, e.g., feelings of low energy, fatigue, dyspnea, etc.) that continue after recovery from novel coronavirus infections (COVID-19).

[EXAMPLES]

Hereinafter, the present invention will be described based on examples. However, the present invention is not limited to these examples and the like.

Test Example 1: Cytopathic effect (CPE) suppression effect confirmation test using Vero E6 cells expressing human TMPRSS2 (Vero E6/TMPRSS2 cells)

<Operational procedure>

• Dilution and dispensing of sample to be tested

The test sample is preliminarily diluted with DMSO to an appropriate concentration, and a 3-fold serial dilution series is prepared and then dispensed into a 96-well plate.

• Dilution and dispensing of cells and SARS-CoV-2

VeroE6/TMPRSS2 cells (JCRB1819, $1.5 \times 10^4$ cells/well) and SARS-CoV-2 hCoV-19/Japan/TY/WK-521/2020, hCoV-19/Japan/QK002/2020, hCoV-19/Japan/QHN001/2020, hCoV-19/Japan/QHN002/2020, hCoV-19/Japan/TY7-501/2021, hCoV-19/Japan/TY7-503/2021, hCoV-19/Japan/TY8-612/2021, hCoV-19/Japan/TY11-927-P1/2021, hCoV-19/Japan/TY33-456/2021, hCoV-19/Japan/TY28-444/2021, hCoV-19/Japan/TY26-717/2021, hCoV-19/Japan/TY38-871/2021, hCoV-19/Japan/TY40-385/2022, hCoV-19/Japan/TY41-721/2022, hCoV-19/Japan/TY41-716/2022, hCoV-19/Japan/TY41-703/2022, hCoV-19/Japan/TY41-702/2022, hCoV-19/Japan/TY41-796/2022, hCoV-19/Japan/TY41-795/2022, hCoV-19/Japan/TY41-686/2022 (30-3000 $TCID_{50}$/well) are mixed in a culture medium (MEM, 2% FBS, penicillin-streptomycin), dispensed into a well containing the test sample, and then cultured in a $CO_2$ incubator for 3 days or 4 days.

• Dispensing and measuring of luminescence signal of CellTiter-Glo (registered trademark) 2.0

After returning the plate cultured for 3 days to room temperature, CellTiter-Glo (registered trademark) 2.0 is dispensed into each well and mixed with a plate mixer. The plate is left to stand for a certain time, and then the luminescence signals (Lum) is measured with a plate reader.

<Calculation of each measurement item>

• Calculation of 50% SARS-CoV-2 infected cell death inhibitory concentration ($EC_{50}$)

When x is taken as the logarithmic value of the compound concentration and y as %Efficacy, the inhibition curve is approximated by the following Logistic regression equation, and the value of x when substituting 50 (%) for y is calculated as $EC_{50}$.

$$y = min + (max - min)/\{1 + (X50/x)\ \text{^}Hill\}$$

$$\%Efficacy = \{(Sample - virus\ control) / (cell\ control - virus\ control)\} * 100\%$$

cell control: the average of Lum of cell control wells
virus control: the average of Lum of virus control wells
min: lower limit of y-axis, max: upper limit of y-axis, X50: x-coordinate of inflection point, Hill: slope of curve at midpoint between min and max

The fumaric acid cocrystal Form I crystal of the compound represented by Formula (I) was substantively tested. Results are shown below.

[Table 6]

| Virus strain | Fumaric acid cocrystal Form I crystal of compound represented by Formula (I) $EC_{50}$ (μM) |
|---|---|
| WT strain (WK-521) | 0.37 |
| Alpha strain (QHN001/QHN002/QK002) | 0.31/0.46/0.33 |
| Beta strain (TY8-612) | 0.40 |
| Gamma strain (TY7-501/TY7-503) | 0.50/0.43 |
| Delta strain (TY11-927-P1) | 0.41 |
| Lambda strain (TY33-456) | 0.27 |
| Theta strain (TY28-444) | 0.29 |
| Mu strain (TY26-717) | 0.43 |
| Omicron BA.1 strain (TY38-873) | 0.29 |
| Omicron BA. 1.1 strain (TY38-871) | 0.36 |
| Omicron BA.2 strain (TY40-385) | 0.52 |
| Omicron BA. 2. 12.1 strain (TY41-721) | 0.24 |
| Omicron BA. 2. 75 strain (TY41-716) | 0.30 |
| Omicron BA.4 strain (TY41-703) | 0.22 |
| Omicron BA.5 strain (TY41-702) | 0.40 |

(continued)

| Virus strain | Fumaric acid cocrystal Form I crystal of compound represented by Formula (I) $EC_{50}$ (μM) |
|---|---|
| Omicron BQ. 1. 1 strain (TY41-796) | 0.48 |
| Omicron XBB.1 strain (TY41-795) | 0.33 |
| Omicron XE strain (TY41-686) | 0.44 |

Test Example 2 Inhibitory activity test against SARS-CoV-2 3CL proteases

<Materials>

- Commercially available Recombinant SARS-CoV-2 3CL Protease
- Commercially available substrate peptide
  Dabcyl-Lys-Thr-Ser-Ala-Val-Leu-Gln-Ser-Gly-Phe-Arg-Lys-Met-Glu(Edans)-NH2 (SEQ ID NO: 1)
- Internal Standard peptide

  Dabcyl-Lys-Thr-Ser-Ala-Val-Leu(13C6,15N)-Gln (SEQ ID NO: 2)
  Dabcyl-Lys-Thr-Ser-Ala-Val-Leu(13C6,15N)-Gln can be synthesized with reference to the literature (Atherton, E.; Sheppard, R. C., "In Solid Phase Peptide Synthesis, A Practical Approach", IRL Press at Oxford University Pres, 1989. and Bioorg. Med. Chem., Volume 5, Issue 9, 1997, pp. 1883-1891, etc.). An example will be described below.
  H-Lys-Thr-Ser-Ala-Val-Leu(13C6, 15N)-Glu(resin)-OaOtBu (the Lys side chain is Boc-protected, the Thr side chain is protected with a tert-butyl group, the Ser side chain is protected with a tert-butyl group, the C-terminal OH of Glu is protected with a tert-butyl group, and the carboxylic acid of the Glu side chain is condensed into the resin) is synthesized using Rink amide resin by Fmoc solid-phase synthesis. Modification of the N-terminus Dabcyl group condenses 4-dimethylaminoazobenzene-4'-carboxylic acid (Dabcyl-OH) on resins using EDC/HOBT. Final deprotection and excision from the resins are performed by treatment with TFA/EDT = 95 : 5. Thereafter, purification is performed by reverse phase HPLC.

- RapidFire Cartridge C4 typeA

<Operational procedure>

• Preparation of assay buffer

In this study, an assay buffer consisting of 20 mM Tris-HCl, 1 mM EDTA, 10 mM DTT, and 0.01% BSA is used.

• Dilution and dispensing of sample to be tested

The test sample is preliminarily diluted with DMSO to an appropriate concentration, and a 3-fold serial dilution series is prepared and then dispensed into a 384-well plate.

• Addition of enzyme and substrate and enzymatic reaction

To the prepared compound plate, 8 μM of substrate and 6 nM of enzyme solution are added and incubation is performed for 3 hours at room temperature. Thereafter, a reaction stop solution (0.072 μM Internal Standard, 0.1% formic acid, 10% acetonitrile) is added to stop the enzymatic reaction.

• Measurement of reaction product

The plate in which the reaction has been completed is measured using RapidFire System 360 and mass spectrometer (Agilent Technologies, Inc., 6550 iFunnel Q-TOF). As the mobile phase at the time of measurement, A solution (75% isopropanol, 15% acetonitrile, 5 mM ammonium formate) and B solution (0.01% trifluoroacetic acid, 0.09% formic acid) are used.

Reaction products detected by the mass spectrometer are calculated using RapidFire Integrator and are taken as Product area value. Furthermore, Internal Standard detected at the same time is also calculated and taken as Internal Standard area value.

<Calculation of each measurement item>

• Calculation of P/IS

The area values obtained in the previous section is calculated by the following equation to calculate P/IS.

$$P/IS = \text{Product area value}/\text{Internal Standard area value}$$

• Calculation of 50% SARS-CoV-2 3CL protease inhibitory concentration ($IC_{50}$)

When x is taken as the logarithmic value of the compound concentration and y as %Inhibition, the inhibition curve is approximated by the following Logistic regression equation, and the value of x when substituting 50 (%) for y is calculated as $IC_{50}$.

$$y = \min + (\max - \min)/\{1 + (X50/x)\ \text{^}Hill\}$$

$$\%Inhibition = \{1-(Sample - Control(-))\ /\ Control(+)-Control(-))\} * 100$$

Control(-): the average of P/IS ratio in the wells without SARS-CoV-2 3CL protease and test substance
Control(+): the average of P/IS ratio in the wells with SARS-CoV-2 3CL protease and without test substance
min: lower limit of y-axis, max: upper limit of y-axis, X50: x-coordinate of inflection point, Hill: slope of curve at midpoint between min and max

The fumaric acid cocrystal Form I crystal of the compound represented by Formula (I) was substantively tested. Results are shown below.
Fumaric acid cocrystal Form I crystal of the compound represented by Formula (I): 0.0132 μM

Test Example 3-1 Phase I Clinical Study

All studies were conducted based on Good Clinical Practice (GCP). The phase I clinical study was composed of Part 1 to Part 7, and in Part 1, the safety, tolerability, and pharmacokinetics were confirmed when the fumaric acid cocrystal Form I crystal of the compound represented by Formula (I) was administered once and when it was administered repeatedly.
The secondary objectives and some of the secondary endpoints of Part 1 are shown below.

[Table 7]

| Part 1 (Secondary objective) | Part 1 (Secondary endpoints) |
|---|---|
| To investigate pharmacokinetics when fumaric acid cocrystal Form I crystal of compound represented by Formula (I) is administered to healthy Japanese male adults. | $C_{max}$, $T_{max}$, $AUL_{0\text{-}last}$, $AUC_{0\text{-}inf}$, $t_{1/2,z}$, $\lambda_z$, MRT, CL/F, $V_z$/F, $CL_R$, and Feu |

The subjects who were determined to be eligible by the screening examination were randomly assigned to either the active drug group (see the following table for the dose of the fumaric acid cocrystal Form I crystal of the compound represented by Formula (I)) or the placebo group on the day before the day of administration of the investigational new drug (Day -1). On Day 1, the investigational new drug (active drug or placebo) was administered once in the fasted state.

(Results)

The results of $t_{1/2,z}$ at the time of single administration in the phase I are shown below.

[Table 8]

| | $t_{1/2,z}$ of fumaric acid cocrystal Form I crystal of compound represented by Formula (I) in plasma (suspension) | | | | | |
|---|---|---|---|---|---|---|
| | 20 mg (On empty stomach) | 70 mg (On empty stomach) | 250 mg (On empty stomach) | 250 mg (After meal) | 500 mg (On empty stomach) | 1000 mg (On empty stomach) |
| N | 6 | 6 | 8 | 8 | 6 | 6 |
| $t_{1/2,z}$ (hr) | 42.6 (18.6) | 45.7 (11.9) | 43.1 (20.2) | 40.5 (23.4) | 42.2 (14.6) | 48.1 (11.3) |

Geometric mean (coefficient of variation)

As shown in the above table, the half-life was more than 40 hours in any dose group, which shows that the half-life of the compound represented by Formula (I) was long.

In addition, no serious adverse events for which a causal relationship cannot be denied were reported in the phase 1 study.

Test Example 3-2 Prediction from single administration of 500 mg in the fasted state

The plasma drug concentration profile at the dosage regimen in Fig. 3 was simulated by the superposition method assuming that the pharmacokinetics is linear with respect to the active drug concentration in the plasma up to 336 hours of administration when 500 mg of the active drug (suspension formulation) was orally administered to 6 Japanese healthy adult male subjects once on empty stomach in the Japanese phase 1 study. From the pharmacokinetic/pharmacodynamic analysis using the SARS-CoV-2 infection mouse model, the maximum plasma concentrations ($C_{max}$) in humans that reduce the virus titer by 2 log and 3 log were estimated to be 14.1 pg/mL and 23.0 μg/mL, respectively. In addition, in order to reach the plasma drug concentration necessary for the expected drug efficacy to be achieved from the first day of administration, a loading dose was provided. From the above, as the dosage regimen for the phase 2/3 study, the following were set: repeated administration once a day for 5 days of a loading dose of 375 mg (Treatment Day 1) and a maintenance dose of 125 mg (Treatment Day 2 to Treatment Day 5), which were expected to provide the exposure necessary to reduce the virus titer by 2 logs or more; and repeated administration once a day for 5 days of a loading dose of 750 mg (Treatment Day 1) and a maintenance dose of 250 mg (Treatment Day 2 to Treatment Day 5), which were expected to provide the exposure necessary to reduce the virus titer by 3 logs. In the dosage regimen thus set, it was considered that the plasma concentration necessary for the expression of drug efficacy can be maintained without exceeding the NOAEL (75.2-77.6 μg/mL) obtained in the toxicity test using monkeys.

Test Example 4 Clinical study (Ph2/3 study)

Efficacy and safety of repeated oral administration of an active drug (active ingredient: fumaric acid cocrystal Form I crystal of the compound represented by Formula (I)) to SARS-CoV-2-infected patients having mild/moderate severity (hereinafter referred to as mild/moderate SARS-CoV-2-infected patients) and SARS-CoV-2-infected patients having asymptomatic/mild symptoms only were evaluated by a placebo-controlled, randomized, double-blind, comparative study.

The primary endpoint of Phase 2a Part was the change from baseline in SARS-CoV-2 virus titer at each time point in common among mild/moderate and asymptomatic SARS-CoV-2-infected patients, and the antiviral effect of the active drug was confirmed.

The primary endpoints of Phase 2b Part were the time-weighted average change in the total score of 12 COVID-19 symptoms per unit time from the start of the first administration (Day 1) to 120 hours (Day 6), and the change from baseline in SARS-CoV-2 virus titer on Day 4, in mild/moderate SARS-CoV-2-infected patients, and the clinical improvement and the antiviral effect of the active drug were confirmed.

The primary endpoint of Phase 3 Part was the time to resolution of five COVID-19 symptoms in mild/moderate SARS-CoV-2-infected patients, and the efficacy was verified by evaluation of the five COVID-19 symptoms in four grades (0: none, 1: mild, 2: moderate, 3: severe) by the patients themselves.

The primary endpoint of Phase 2b/3 Part is the proportion of patients with onset/worsening COVID-19 symptoms among SARS-CoV-2-infected patients having asymptomatic/mild symptoms only. The efficacy was verified by evaluation of the 12 COVID-19 symptoms in four grades (0: none, 1: mild, 2: moderate, 3: severe), and evaluation of dysgeusia and dysosmia in three grades (0: same as usual, 1: less than usual, 2: no sense), by the patients themselves.

The key secondary endpoints of Phase 3 Part and Phase 2b/3 Part were the change from baseline on Day 4 in the

amount of SARS-CoV-2 viral RNA, and the time to the first negative SARS-CoV-2 virus titer, and the antiviral effect was verified.

Regarding mild/moderate SARS-CoV-2-infected patients, patients who met all of the following criteria were selected.

(a) Male or female patients who are 12 years old or older and younger than 70 years old.
(b) Those diagnosed as SARS-CoV-2-positive within 120 hours prior to enrollment.
(c) Those whose time from onset of COVID-19 to enrollment is within 120 hours.
(d) Those having one or more moderate (COVID-19 symptom score: 2) or higher symptoms in any of the following symptoms due to COVID-19 (twelve COVID-19 symptoms) (except for symptoms that were present before onset of COVID-19) at the time of enrollment.

Systemic symptoms: low energy and tiredness, muscle or body aches, headache, chills or shivering, feeling hot or feverish
Respiratory symptoms: stuffy and runny nose, sore throat, cough, shortness of breath (difficulty breathing)
Gastrointestinal symptoms: nausea, vomiting, diarrhea

Regarding asymptomatic SARS-CoV-2-infected patients, patients who met all of the following criteria were selected.

(a) Male or female patients who are 12 years old or older and younger than 70 years old.
(b) Those diagnosed as SARS-CoV-2-positive within 120 hours prior to enrollment.
(c) Asymptomatic (Phase 2a Part): those who do not have the following COVID-19 symptoms (except for symptoms that were present before SARS-CoV-2 infection) within 2 weeks prior to enrollment.

Systemic symptoms: low energy and tiredness, muscle or body aches, headache, chills or shivering, feeling hot or feverish
Respiratory symptoms: stuffy and runny nose, sore throat, cough, shortness of breath (difficulty breathing)
Gastrointestinal symptoms: nausea, vomiting, diarrhea
Asymptomatic/mild symptoms only (Phase 2b/3 Part): those not having moderate (COVID-19 symptom score: 2) or higher symptoms in any of the following symptoms due to COVID-19 (twelve COVID-19 symptoms) (except for symptoms that were present before onset of COVID-19) within 2 weeks before randomization
Systemic symptoms: low energy and tiredness, muscle or body aches, headache, chills or shivering, feeling hot or feverish
Respiratory symptoms: stuffy and runny nose, sore throat, cough, shortness of breath (difficulty breathing)
Gastrointestinal symptoms: nausea, vomiting, diarrhea

Method of administration of investigational new drug

(i) Active drug

250 mg tablet: The tablet contains fumaric acid cocrystal Form I crystal of the compound represented by Formula (I), and contains 250 mg of the compound represented by Formula (I).

125 mg tablet: The tablet contains fumaric acid cocrystal Form I crystal of the compound represented by Formula (I), and contains 125 mg of the compound represented by Formula (I).

(ii) Placebo

Placebo-D tablet: It is a tablet having an appearance indistinguishable from the above 250 mg tablet, and does not contain the fumaric acid cocrystal Form I crystal of the compound represented by Formula (I).

Placebo-B tablet: It is a tablet having an appearance indistinguishable from the above 125 mg tablet, and does not contain the fumaric acid cocrystal Form I crystal of the compound represented by Formula (I).

Dose and method of administration

In Phase 2a Part, subjects who were determined to be eligible as mild/moderate or asymptomatic SARS-CoV-2-infected patients were randomly assigned at a ratio of 1 : 1 : 1 to any of the active drug 375/125 mg group, the active drug 750/250 mg group, and the placebo group.

Subjects determined to be eligible as mild/moderate SARS-CoV-2-infected patients in Phase 2b Part and Phase 3 Part, as well as subjects determined to be eligible as SARS-CoV-2-infected patients having asymptomatic/mild symptoms only in Phase 2b/3 Part, were randomly assigned at a ratio of 1 : 1 : 1 to any of the active drug 375/125 mg group, the active drug 750/250mg group, and the placebo group.
The administration of the active drug is repeated oral administration once a day for 5 days. The 375/125 mg group received 375 mg on Day 1 as a loading dose and 125 mg on Days 2 to 5 as a maintenance dose. The 750/250mg group received 750 mg on Day 1 as a loading dose and 250 mg on Days 2 to 5 as a maintenance dose.

Investigational new drug by treatment group

375/125 mg group

On Day 1, three 125 mg tablets and three Placebo-D tablets are administered on Day 1, and on Days 2 to 5, one 125 mg tablet and one Placebo-D tablet are administered per day.

750/250 mg group

On Day 1, three 250 mg tablets and three Placebo-B tablets are administered on Day 1, and on Days 2 to 5, one 250 mg tablet and one Placebo-B tablet are administered per day.

Placebo group

On Day 1, three Placebo-D tablets and three Placebo-B tablets are administered on Day 1, and on Days 2 to 5, one Placebo-D tablet and one Placebo-B tablet are administered per day.
"Day 1" represents the first day of administration, and "Day 2 to Day 5" represent the second to fifth days counted from the first day of administration.

Primary efficacy endpoint (Phase 2a Part)

The primary efficacy endpoint of Phase 2a Part was the change from baseline in SARS-CoV-2 virus titer at each time point in common among mild/moderate and asymptomatic SARS-CoV-2-infected patients. It was defined as an absolute change from baseline in observed value of SARS-CoV-2 virus titer at each time point.

Primary efficacy endpoint (Phase 2b Part: mild/moderate SARS-CoV-2-infected patients)

The primary efficacy endpoints of Phase 2b Part for mild/moderate SARS-CoV-2-infected patients were the time-weighted average change in the total score of 12 COVID-19 symptoms per unit time from the start of the first administration (Day 1) to 120 hours (Day 6), and the change from baseline in SARS-CoV-2 virus titer on Day 4. The total score of 12 COVID-19 symptoms is the sum of the scores determined by evaluation in four grades (0: none, 1: mild, 2: moderate, 3: severe) in the subject daily records for each of the 12 COVID-19 symptoms (low energy and tiredness, muscle or body aches, headache, chills or shivering, feeling hot or feverish, stuffy and runny nose, sore throat, cough, shortness of breath (difficulty breathing), nausea, vomiting, diarrhea). For the amount of change from baseline in the total score of 12 COVID-19 symptoms observed at each time point, the AUC from the start of the first administration (Day 1) to 120 hours (Day 6) divided by the evaluation period (unit: time) at the time of AUC calculation was determined as the amount of change per unit time.

Primary efficacy endpoint (Phase 3 Part: mild/moderate SARS-CoV-2-infected patients)

The primary efficacy endpoint of Phase 3 Part for mild/moderate SARS-CoV-2-infected patients was the time to resolution of the five COVID-19 symptoms. It was defined to be the time from the start of administration of investigational active drug to the time point of resolution from all of the five COVID-19 symptoms (stuffy and runny nose, sore throat, cough, feeling hot or feverish, low energy and tiredness).
COVID-19 symptoms were evaluated in four grades (0: none, 1: mild, 2: moderate, 3: severe) in the subject daily record, and recovery from the symptoms, with the condition of the same remaining for at least 24 hours, was determined according to the following rules.
Existing symptoms that existed before the onset of COVID-19 and were determined by the subject to be worsening at the baseline (pre-administration test) time point had to be improved or maintained from the severity at the baseline time point.

Severity at baseline was "severe" → "moderate", "mild", or "none"
Severity at baseline was "moderate" → "mild", or "none"
Severity at baseline was "mild" → "mild", or "none"
Existing symptoms that existed before the onset of COVID-19 and were determined by the subject not to be worsening at the baseline (pre-administration test) time point had to be maintained or improved from the severity at the baseline time point.
Severity at baseline was "severe" → "severe", "moderate", "mild", or "none"
Severity at baseline was "moderate" → "moderate", "mild", or "none"
Severity at baseline was "mild" → "mild", or "none"
Regarding symptoms other than the above, that is, symptoms that were not present before the onset of COVID-19 and developed at or after the baseline (pre-administration test) time point, such symptoms had to be eliminated.
Severity at baseline was "severe", "moderate", or "mild" → "none"

Primary efficacy endpoint (Phase 2b/3 Part: SARS-CoV-2-infected patients having asymptomatic/mild symptoms only)

The primary efficacy endpoint of Phase 2b/3 Part in the Phase 2/3 study in SARS-CoV-2-infected patients having asymptomatic/mild symptoms only is the proportion of patients who developed/worsened COVID-19 symptoms. It is defined as the proportion of subjects who developed/worsened either the twelve COVID-19 symptoms or dysgeusia and dysosmia during the evaluation period of the subject daily records.

COVID-19 symptoms are evaluated in three grades of 0 to 2 (0: same as usual, 1: less than usual, 2: no sense) for dysgeusia and dysosmia, and in four grades of 0 to 3 (0: no symptom, 1: mild, 2: moderate, 3: severe) for twelve COVID-19 symptoms, in a subject daily records recorded by subjects, and the onset of symptoms is determined according to the following rules.

Dysgeusia and dysosmia

The score of dysgeusia and dysosmia was worsened from "0: same as usual " to "1: less than usual" or "2: no sense", or from "1: less than usual" to "2: no sense"
Three symptoms of feeling hot or feverish, cough, shortness of breath (difficulty breathing)
When the score deteriorates by one or more grades from baseline for any of the symptoms:

Severity at baseline of "none" deteriorates → "mild", "moderate", or "severe"
Severity at baseline of "mild" deteriorates → "moderate", or "severe"
Severity at baseline of "moderate" deteriorates → "severe"
(Those with severe severity at baseline are excluded from subjects for determination of onset of COVID-19 symptoms)
Nine symptoms of low energy and tiredness, muscle or body aches, headache, chills or shivering, stuffy or runny nose, sore throat, nausea, vomiting, and diarrhea
When regarding two or more symptoms, the scores worsen by one or more grades from baseline at the same time point, and the condition persists for at least 24 hours:

Severity at baseline of "none" deteriorates → "mild", "moderate", or "severe"
Severity at baseline of "mild" deteriorates → "moderate", or "severe"
Severity at baseline of "moderate" deteriorates → "severe"
(Those with severe severity at baseline are excluded from subjects for determination of onset of COVID-19 symptoms)

Key secondary efficacy endpoint

The key secondary efficacy endpoints of Phase 3 Part and Phase 2b/3 Part in the phase 2/3 study were the change from baseline in the SARS-CoV-2 viral RNA amount on Day 4, and the time to the first negative SARS-CoV-2 virus titer. The time to the first negative SARS-CoV-2 virus titer was defined as the time from the start of administration of the investigational new drug until a virus titer of SARS-CoV-2 was first less than the detection limit.

Analysis of primary endpoint (Phase 2a Part)

For each population of the mild/moderate SARS-CoV-2-infected patients, the asymptomatic SARS-CoV-2-infected patients, and the merged population thereof, summary statistics of the change from baseline in SARS-CoV-2 virus titer at each time point were calculated for the mITT population. Furthermore, a van Elteren test was applied to a combined population of mild/moderate and asymptomatic SARS-CoV-2-infected patients. Pairwise comparisons were made between each active drug dose group and the placebo group for SARS-CoV-2 virus titer at each time point at a two-sided significance level of 5%. The population of the mild/moderate SARS-CoV-2-infected patients and the population of the asymptomatic SARS-CoV-2-infected patients were used as strata in the van Elteren test.

Analysis of primary endpoint (Phase 2b Part: mild/moderate SARS-CoV-2-infected patients)

As the primary analysis for the time-weighted average change in total score of COVID-19 symptoms from baseline up to 120 hours, analysis of covariance was applied to mild/moderate SARS-CoV-2-infected patients with 1 or more in a total score of 12 COVID-19 symptoms at the start of the first administration (Day 1) among the ITT1 population, with the change per unit time as a response, the time from onset of COVID-19 to randomization (less than 72 hours, 72 hours or more), the SARS-CoV-2 vaccination history (Yes or No), and the total score of the 12 COVID-19 symptoms at baseline as covariates. Pairwise comparison was performed between each active drug dose group and the placebo group. In addition, as the primary analysis for the change from baseline in SARS-CoV-2 virus titer on Day 4, analysis of covariance was applied to mild/moderate SARS-CoV-2-infected patients among the ITT1 population, with the change from baseline in SARS-CoV-2 virus titer as a response, the time from onset of COVID-19 to randomization (less than 72 hours, 72 hours or more), the SARS-CoV-2 vaccination history (Yes or No), and SARS-CoV-2 virus titer at baseline as covariates. Pairwise comparison was performed between each active drug dose group and the placebo group.

Analysis of primary endpoint (Phase 3 Part: mild/moderate SARS-CoV-2-infected patients)

As the primary analysis, for the population of the mild/moderate SARS-CoV-2-infected patients whose time from onset of COVID-19 to randomization was less than 72 hours in the ITT population, the stratified Peto-Prentice's generalized Wilcoxon test with the SARS-CoV-2 vaccination history (Yes or No) as a stratum was used, and comparison was performed between the 125 mg active drug group and the placebo group for the time to resolution of the five COVID-19 symptoms at a one-sided significance level of 0.025. As the key secondary analysis of the primary endpoint, the same analysis as the primary analysis was performed for the population of the mild/moderate SARS-CoV-2-infected patients in the ITT population. At this time, in addition to the SARS-CoV-2 vaccination history, the time from onset of COVID-19 to randomization (less than 72 hours, 72 hours or more) was also included in the strata.

Analysis of primary endpoint (Phase 2b/3 Part: SARS-CoV-2-infected patients having asymptomatic/mild symptoms only)

As the primary analysis, for the population of the SARS-CoV-2-infected patients having asymptomatic/mild symptoms only in the ITT population, the Mantel-Haenszel test with the SARS-CoV-2 vaccination history (Yes or No) as a stratum was used, and comparison was performed between the 125 mg active drug group and the placebo group for the patients who developed/worsened COVID-19 symptoms at a two-sided significance level of 5%.

Analysis of key secondary endpoints (Phase 3 Part)

Change from baseline in SARS-CoV-2 viral RNA amount on Day 4:

The primary analysis was planned for each of mild/moderate SARS-CoV-2-infected patients and SARS-CoV-2-infected patients having asymptomatic/mild symptoms only. Regarding mild/moderate SARS-CoV-2-infected patients, a population whose time from onset of COVID-19 to randomization was less than 72 hours among the ITT population of Phase 3 Part, as the primary analysis on the change from baseline in SARS-CoV-2 viral RNA amount on Day 4, analysis of covariance was applied with the change from baseline in the SARS-CoV-2 virus RNA amount as a response, the SARS-CoV-2 vaccination history (Yes or No), and the SARS-CoV-2 virus RNA amount at baseline as covariates. Pairwise comparison was performed between the 125 mg active drug group and the placebo group. As a key secondary analysis, a similar analysis was performed on the ITT population. At this time, in addition to the SARS-CoV-2 vaccination history, the SARS-CoV-2 viral RNA amount at baseline, the time from onset of COVID-19 to randomization (less than 72 hours, 72 hours or more) were also included in the covariates. Regarding SARS-CoV-2-infected patients having asymptomatic/mild symptoms only among the ITT population of Phase 2b/3 Part, as the primary analysis on the change from baseline in

SARS-CoV-2 viral RNA amount on Day 4, analysis of covariance was applied with the change from baseline in SARS-CoV-2 virus RNA amount as a response, the SARS-CoV-2 vaccination history (Yes or No), and the SARS-CoV-2 virus RNA amount at baseline as covariates. Pairwise comparison was performed between the 125 mg active drug group and the placebo group.

Time to the first negative SARS-CoV-2 virus titer:

The primary analysis was planned for each of mild/moderate SARS-CoV-2-infected patients and SARS-CoV-2-infected patients having asymptomatic/mild symptoms only. Regarding the mild/moderate SARS-CoV-2-infected patients, the population whose time from onset of COVID-19 to randomization was less than 72 hours in the mITT population of Phase 3 Part, the stratified log-rank test with the SARS-CoV-2 vaccination history (Yes or No) as a stratum was used. Comparison was performed between the 125 mg active drug group and the placebo group for the time to the first negative SARS-CoV-2 virus titer at a one-sided significance level of 0.025. As a key secondary analysis, a similar analysis was performed on the mITT population. At this time, in addition to the SARS-CoV-2 vaccination history, the time from onset of COVID-19 to randomization (less than 72 hours, 72 hours or more) was also included in the strata. Regarding the SARS-CoV-2-infected patients having asymptomatic/mild symptoms only among the mITT population of Phase 2b/3 Part, the stratified log-rank test with the SARS-CoV-2 vaccination history (Yes or No) as a stratum was used. Comparison was performed between the 125 mg active drug group and the placebo group for the time to the first negative SARS-CoV-2 virus titer at a one-sided significance level of 0.025.

Results of primary endpoint

(1) Change from the baseline in SARS-CoV-2 virus titer at each time point (Phase 2a Part)

69 cases were randomized in Phase 2a Part, and 22 cases (of which 1 case did not receive administration) were assigned to the 375/125 mg group, 23 cases were assigned to the 750/250 mg group, and 24 cases were assigned to the placebo group. Among the 69 cases, the number of cases positive for baseline RT-PCR was 44, and among them, the number of cases in which a baseline virus titer was detected was 40. The constitution of the 40 cases was 14 cases in the 375/125 mg group, 13 cases in the 750/250 mg group, and 13 cases in the placebo group. Note that the number of cases of these populations and the breakdown thereof were calculated on the basis of the RT-PCR measurement results and the virus titer measurement results available by January 17, 2022.

As a final result of Phase 2a Part, among the 69 cases, the number of cases positive for baseline RT-PCR was 47, and among them, the number of cases in which a baseline virus titer was detected was 43. The constitution of the 43 cases was 15 cases in the 375/125 mg group, 14 cases in the 750/250 mg group, and 14 cases in the placebo group.

The visit date defined in the study protocol is denoted by Visit, and the correspondence relationship with the administration date (Day) and the permitted window are as follows. Op V means Optional Visit and indicates any visit date.

[Table 9]

| Visit | V1 | V2 | Op V1 | V3 | Op V2 | V4 | V5 | V6 | V7 | V8 |
|---|---|---|---|---|---|---|---|---|---|---|
| Day | 1 | 2 | 3 | 4 | 5 | 6 | 9 | 14 | 21 | 28 |
| Permitted window | -1 | +1 | - | +1 | - | +1 | ±1 | ±2 | ±3 | ±3 |
| | Administration | Administration | Administration | Administration | Administration | | | | | |

Fig. 4 shows a mean change from baseline in SARS-CoV-2 virus titer for each group in the modified intention-to-treat population (all subjects randomized and positive for both baseline RT-PCR and virus titer). This analysis included mild/moderate SARS-CoV-2-infected patients and asymptomatic SARS-CoV-2-infected patients, and only Visit that was set as an essential visit date was displayed. In addition, when the virus titer was less than the lower detection limit (0.8 $\log_{10}$ ($TCID_{50}$/mL)), the value of the virus titer was handled as 0.8 $\log_{10}$ ($TCID_{50}$/mL). At the time points of Visit 3 (Day 4 after the start of administration) in the 375/125 mg group, Visit 2 (Day 2 after the start of administration) and Visit 3 (Day 4 after the start of administration) in the 750/250 mg group, the virus titer statistically significantly decreased at a significance level of 0.05 as compared to the placebo group. In the RT-PCR measurement results and the virus titer measurement results that were available by the time point of January 17, 2022, there was also a tendency for the virus titer to decrease as compared to the placebo group at all time points after Visit 3 (Day 4 after the start of administration) in the 375/125 mg group and after Visit 2 (Day 2 after the start of administration) in the 750/250 mg group.

A proportion of patients with a positive virus titer at each time point is shown below.

At the time point of Day 4, with respect to the proportion of patients with a positive virus titer of 0.8 or more in the placebo group, the proportion decreased by 80% in the 750/250 mg group and by 63% in the 375/125 mg group. At the time point of Day 6, with respect to the proportion of patients with a positive virus titer of 0.8 or more in the placebo group, the proportion decreased by 54% in the 750/250 mg group and by 100% in the 375/125 mg group.

At the time points of Day 4 and Day 6, the proportion of patients with a positive virus titer tended to be lower in both the 750/250 mg group and the 375/125 mg group compared to the placebo group. Therefore, it was suggested that by taking the pharmaceutical composition of the present invention, the number of patients who shed infectious viruses can be rapidly reduced.

Results of secondary endpoints

(1) Time to the first negative SARS-CoV-2 virus titer (Phase 2a Part)

Time to the first negative SARS-CoV-2 virus titer is shown in Table 10 below and Fig. 5.

Among 69 cases of Phase 2a Part, the results in 15 cases in the 375/125 mg group, 13 cases in the 750/250 mg group, and 14 cases in the placebo group are shown.

[Table 10]

| | 375/125 mg group | 750/250 mg group | Placebo group |
|---|---|---|---|
| Number of cases | 15 | 13 | 14 |
| Median (time) [95% confidence interval] | 61.3 [38.0, 68.4] | 62.7 [39.2, 72.3] | 111.1 [23.2, 158.5] |
| Difference of median value [95% confidence interval] | -49.8 [-96.7, 30.9] | -48.4 [-95.9, 28.5] | --- |
| Stratified log-rank test | P = 0.0159 | P = 0.0205 | --- |

[In the stratified log rank test, the subject layer (mild/moderate, asymptomatic/mild only) is used as a stratification factor]

As shown in Table 10, in the 375/125 mg group, the median was 49.8 hours shorter than that in the placebo group, and there was a significant difference in the time to the first negative SARS-CoV-2 virus titer (p = 0.0159). In the 750/250 mg group, the median was 48.4 hours shorter than that in the placebo group, and there was a significant difference in the time to the first negative SARS-CoV-2 virus titer (p = 0.0205).

In addition, as shown in Fig. 5, in the placebo group, the time to the first negative SARS-CoV-2 virus titer of 50% of patients became negative was about 4.6 days (111.1 hours) after the start of treatment. On the other hand, in the 750/250 mg group and the 375/125 mg group, the time to the first negative SARS-CoV-2 virus titer of 50% of patients became negative was about 2.6 days (61.3 to 62.7 hours) after the start of treatment. Thus, it was confirmed that the time to the first negative SARS-CoV-2 virus titer of 50% of patients was reduced by about 2 days.

(2) Amount of change from baseline in total score of 12 COVID-19 symptoms at each time point (Phase 2a Part)

An amount of change from baseline in the total score of 12 COVID-19 symptoms at each time point is shown in Fig. 6.

Among 69 cases of Phase 2a Part, the results in 13 cases in the 375/125 mg group, 12 cases in the 750/250 mg group, and 14 cases in the placebo group for subjects having mild/moderate severity are shown.

As shown in Fig. 6, in the 375/125 mg group and the 750/250 mg group, there was a tendency for the total score of

the twelve COVID-19 symptoms to numerically improve as compared to the placebo group at all time points on Day 2 (after 1 administration) and thereafter.

(3) Exacerbation suppression effect (Phase 2a Part)

Among 69 cases of Phase 2a Part, for subjects having mild/moderate severity, a proportion of subjects whose Ordinal scale (ordinal scale to classify clinical severity in 8 degrees, Table 11) score deteriorated to 3 or more for the first time at any time point after the start of administration is shown in Table 12.

[Table 11]

| 8-Point Ordinal Scale | Score |
|---|---|
| No symptom recognized | 0 |
| Having symptoms, and no trouble in daily life | 1 |
| Having symptoms, and trouble in daily life | 2 |
| Hospitalization or equivalent medical treatment is required | 3 |
| Subjects in need of hospitalization or equivalent medical treatment and oxygen dministration (less than 5 L/min) | 4 |
| Subjects in need of hospitalization or equivalent medical treatment and oxygen dministration (5 L/min or more) | 5 |
| Subjects in need of hospitalization or equivalent medical treatment and ventilator | 6 |
| Death | 7 |

[Table 12]

| | 375/125 mg group | 750/250 mg group | Placebo group |
|---|---|---|---|
| Exacerbation rate | 0.0% | 0.0% | 14.3% |
| Number of exacerbations/ number of cases to be analyzed | 0/13 | 0/12 | 2114 |

As shown in Table 12, in the 375/125 mg group and the 750/250 mg group, cases in which Ordinal scale deteriorated to 3 or more for the first time after the start of administration were not confirmed.

Results of primary endpoint

(1) Change from the baseline in SARS-CoV-2 virus titer at the time point of Day 4 (Phase 2b Part)

In Phase 2b Part, 428 cases were randomized, and there were 341 cases in which the virus titer of SARS-CoV-2 was detected at the baseline as the ITT population. The 341 cases were composed of 114 cases in the 375/125 mg group, 116 cases in the 750/250 mg group, and 111 cases in the placebo group.

The amount of change from baseline in SARS-CoV-2 virus titer on Day 4 (ITT1 population) is shown as to each group below.

[Table 13]

| Statistics | 375/125 mg group N=114 | 750/250 mg group N=116 | Placebo group N=111 |
|---|---|---|---|
| Number of cases | 106 | 112 | 107 |
| Mean value | -1.69 | -1.43 | -1.06 |
| Standard deviation | 0.84 | 0.83 | 0.99 |
| Minimum value | -3.7 | -3.4 | -3.0 |
| Median value | -1.70 | -1.40 | -1.00 |

(continued)

| Statistics | 375/125 mg group N=114 | 750/250 mg group N=116 | Placebo group N=111 |
|---|---|---|---|
| Maximum value | 0.0 | 0.0 | 2.0 |
| Adjusted mean value by covariance analysis (standard error) | -1.49 (0.04) | -1.49 (0.04) | -1.08 (0.04) |
| Difference of adjusted mean value from that of placebo group (standard error) | -0.41 (0.05) | -0.41 (0.05) | --- |
| 95% confidence interval | -0.51, -0.31 | -0.51, -0.31 | --- |
| P value | <.0001 | <.0001 | --- |

As shown in the above table, it was confirmed that in the 375/125 mg group and the 750/250 mg group, the virus titer was statistically significantly reduced on Day 4 as compared with the placebo group ($p < 0.0001$). Therefore, it was revealed that the administration of the pharmaceutical composition of the present invention exhibits a significantly excellent antiviral effect.

A proportion of patients with a positive virus titer of 1.1 or more on Day 4 is shown below.

In the placebo group, the proportion of patients with positive virus titers was 50.5%, whereas in the 375/125 mg group, the proportion of patients with positive virus titers was 1.9%, and in the 750/250 mg group, the proportion of patients with positive virus titers was 5.4%.

At the time point of Day 4, the proportion of patients with a positive virus titer tended to be clearly lower in both the 375/125 mg group and the 750/250 mg group compared to the placebo group. Therefore, it was suggested that by taking the pharmaceutical composition of the present invention, the number of patients who shed infectious viruses can be rapidly reduced.

(2-a) Change from baseline up to 120 hours in total score of 12 COVID-19 symptoms per unit time (Phase 2b Part)

Among the 341 cases in Phase 2b Part, the number of cases in which the total score of 12 COVID-19 symptoms was observed both at the baseline and after the start of administration, and the total score of twelve COVID-19 symptoms at the baseline was 1 or more was 332, and the 332 cases were composed of 109 cases in the 375/125 mg group, 113 cases in the 750/250 mg group, and 110 cases in the placebo group.

The change from baseline up to 120 hours in the total score of 12 COVID-19 symptoms per unit time (ITT1 population) is show in Table shown below.

[Table 14]

| Statistics | 375/125 mg group N=114 | 750/250 mg group N=116 | Placebo group N=111 |
|---|---|---|---|
| Number of cases | 109 | 113 | 110 |
| Mean value | -5.95 | -5.42 | -4.92 |
| Standard deviation | 4.02 | 3.70 | 3.25 |
| Minimum value | -20.37 | -18.16 | -13.90 |
| Median value | -5.40 | -4.92 | -4.57 |
| Maximum value | 2.43 | 2.47 | 1.99 |
| Adjusted mean value by covariance analysis (standard error) | -5.37 (0.24) | -5.17 (0.23) | -5.12 (0.24) |
| Difference of adjusted mean value from that of placebo group (standard error) | -0.24 (0.30) | -0.04 (0.29) | --- |
| 95% confidence interval | -0.83, 0.34 | -0.62, 0.53 | --- |
| P value | 0.4171 | 0.8806 | --- |

As shown in the above table, in the 375/125 mg group and the 750/250 mg group, a numerical decrease tendency

was observed as compared with the placebo group, but a statistically significant difference was not observed.

(2-b) Change from baseline up to 120 hours in sub-total score of COVID-19 symptoms per unit time (Phase 2b Part)

The results of classifying the twelve COVID-19 symptoms shown in the above (2-a) into 5 types (acute symptoms, major clinical symptoms, respiratory symptoms, systemic symptoms, and gastrointestinal symptoms) and analyzing the same (ITT population) are shown in the following table.

Acute symptoms: sore throat, cough, feeling hot and feverish
Major clinical symptoms: stuffy and runny nose, sore throat, cough, chills or shivering, feeling hot or feverish
Respiratory symptoms: stuffy and runny nose, sore throat, cough, shortness of breath (difficulty breathing)
Systemic symptoms: low energy and tiredness, muscle or body aches, headache, chills or shivering, feeling hot or feverish
Gastrointestinal symptoms: nausea, vomiting, diarrhea

[Table 15-1]

| Parameter | Statistics | 375/125 mg group N=114 | 750/250 mg group N=116 | Placebo group N=111 |
|---|---|---|---|---|
| Acute symptoms | Number of cases | 107 | 108 | 109 |
| | Mean value | -2.52 | -2.63 | -2.09 |
| | Standard deviation | 1.39 | 1.34 | 1.31 |
| | Minimum value | -6.19 | -6.93 | -4.92 |
| | Median value | -2.55 | -2.71 | -1.99 |
| | Maximum value | 0.45 | 0.52 | 1.18 |
| | Adjusted mean value by covariance analysis (standard error) | -2.36 (0.10) | -2.50 (0.10) | -2.16 (0.10) |
| | Difference of adjusted mean value from that of placebo group (standard error) | -0.20 (0.12) | -0.33 (0.12) | --- |
| | 95% confidence interval | -0.44, 0.04 | -0.58, -0.09 | --- |
| | P value | 0.1080 | 0.0070 | --- |

[Table 15-2]

| Parameter | Statistics | 375/125 mg group N=114 | 750/250 mg group N=116 | Placebo group N=111 |
|---|---|---|---|---|
| Major clinical symptoms | Number of cases | 109 | 111 | 110 |
| | Mean value | -3.56 | -3.54 | -2.94 |
| | Standard deviation | 2.01 | 2.00 | 1.94 |
| | Minimum value | -9.76 | -10.11 | -7.81 |
| | Median value | -3.33 | -3.42 | -2.91 |
| | Maximum value | 0.55 | 0.39 | 1.81 |
| | Adjusted mean value by covariance analysis (standard error) | -3.34 (0.14) | -3.42 (0.14) | -2.99 (0.14) |
| | Difference of adjusted mean value from that of placebo group (standard error) | -0.34 (0.17) | -0.42 (0.17) | --- |
| | 95% confidence interval | -0.68, 0.00 | -0.76, -0.08 | --- |
| | P value | 0.0504 | 0.0149 | --- |

[Table 15-3]

| Parameter | Statistics | 375/125 mg group N=114 | 750/250 mg group N=116 | Placebo group N=111 |
|---|---|---|---|---|
| Respiratory symptoms | Number of cases | 106 | 111 | 109 |
| | Mean value | -2.28 | -2.33 | -1.67 |
| | Standard deviation | 1.54 | 1.58 | 1.44 |
| | Minimum value | -6.44 | -6.34 | -5.56 |
| | Median value | -1.99 | -2.30 | -1.53 |
| | Maximum value | 1.30 | 2.29 | 1.81 |
| | Adjusted mean value by covariance analysis (standard error) | -2.09 (0.12) | -2.16 (0.12) | -1.72 (0.13) |
| | Difference of adjusted mean value from that of placebo group (standard error) | -0.37 (0.15) | -0.44 (0.15) | --- |
| | 95% confidence interval | -0.67, -0.07 | -0.74, -0.15 | --- |
| | P value | 0.0153 | 0.0032 | --- |

[Table 15-4]

| Parameter | Statistics | 375/125 mg group N=114 | 750/250 mg group N=116 | Placebo group N=111 |
|---|---|---|---|---|
| Systemic symptoms | Number of cases | 100 | 104 | 98 |
| | Mean value | -3.71 | -3.19 | -3.35 |
| | Standard deviation | 2.86 | 2.54 | 2.28 |
| | Minimum value | -14.30 | -9.66 | -11.26 |
| | Median value | -3.18 | -2.81 | -2.55 |
| | Maximum value | 1.80 | 2.71 | 0.60 |
| | Adjusted mean value by covariance analysis (standard error) | -3.31 (0.14) | -3.16 (0.14) | -3.46 (0.14) |
| | Difference of adjusted mean value from that of placebo group (standard error) | 0.15 (0.18) | 0.30 (0.17) | --- |
| | 95% confidence interval | -0.20, 0.49 | -0.04, 0.64 | --- |
| | P value | 0.4052 | 0.0843 | --- |

[Table 15-5]

| Parameter | Statistics | 375/125 mg group N=114 | 750/250 mg group N=116 | Placebo group N=111 |
|---|---|---|---|---|
| Gastrointestinal symptoms | Number of cases | 35 | 35 | 32 |
| | Mean value | -1.39 | -1.13 | -1.27 |
| | Standard deviation | 1.18 | 1.37 | 0.86 |
| | Minimum value | -5.80 | -5.78 | -3.58 |
| | Median value | -0.99 | -0.79 | -0.95 |
| | Maximum value | -0.16 | 0.50 | 0.19 |
| | Adjusted mean value by covariance analysis (standard error) | -1.27 (0.14) | -1.08 (0.14) | -1.32 (0.14) |
| | Difference of adjusted mean value from that of placebo group (standard error) | 0.06 (0.18) | 0.25 (0.18) | --- |
| | 95% confidence interval | -0.30, 0.42 | -0.11, 0.61 | --- |
| | P value | 0.7519 | 0.1769 | --- |

As shown in the above tables, among the 12 symptoms, in the respiratory symptoms (runny nose or stuffy nose, sore throat, coughing, shortness of breath (dyspnea)) which were characteristic symptoms in the population accumulated in this study, a significant improving effect was observed in the 375/125 mg group and the 750/250 mg group as compared with the placebo group (375/125 mg group: p = 0.0153, 750/250 mg group: p = 0.0032).

The results of the efficacy will be described focusing on the results of the 375/125 mg group used for verification of efficacy.

Results of primary endpoint

(1)Time to resolution of five symptoms of COVID-19 (Phase 3 Part)

Among the ITT population of mild/moderate SARS-CoV-2-infected patients in Phase 3 Part, the population whose time from onset of COVID-19 to randomization was less than 72 hours, which is the primary analysis target population included 347 cases in the 375/125 mg group and 343 cases in the placebo group. The ITT population included 603 cases in the 375/125 mg group and 600 cases in the placebo group.

The times to resolution of five symptoms of COVID-19 in mild/moderate SARS-CoV-2-infected patients (part of the ITT population whose time from onset of COVID-19 to randomization was less than 72 hours) and Kaplan-Meier curves thereof are shown in Table 16 below and Fig. 7.

[Table 16]

| | 375/125 mg group N = 347 | 750/250 mg group N = 340 | Placebo group N = 343 |
|---|---|---|---|
| Nominal significance level (one-sided p value) | 0.025 | --- | --- |
| Number of cases to be analyzed | 336 | 329 | 321 |
| Number of events | 254 (75.6) | 262 (79.6) | 233 (72.6) |
| Number of censored | 82 (24.4) | 67 (20.4) | 88 (27.4) |
| | | | |
| Kaplan-Meier estimated value (hour) | | | |
| Median value [95% confidence interval] | 167.9 [145.0, 197.6] | 171.2 [150.8, 190.3] | 192.2 [174.5, 238.3] |
| Difference of median value (vs placebo) [95% confidence interval] | -24.3 [-78.7, 11.7] | -21.0 [-73.8, 7.2] | --- |
| 25% point, 75% point | 99.5,424.8 | 96.7, 360.9 | 120.2, --- |

(continued)

| | | | |
|---|---|---|---|
| Kaplan-Meier estimated value (hour) | | | |
| Minimum value, maximum value [a] | 2.4,489.1 + | 9.4,490.1 + | 5.0,492.4 + |
| | | | |
| | | | |
| Restricted mean survival time during 21 days (RMST, hour) [95% confidence interval] | 241.6 [223.1, 260.2] | 234.8 [217.0,252.7] | 261.0 [242.4, 279.5] |
| Difference of RMST (vs placebo) [95% confidence interval] | -19.4 [-45.6, 6.9] | -26.1 [-51.9, -0.4] | --- |
| | | | |
| Stratified log-rank test (vs placebo) [b] | | | |
| - One-sided p value | 0.0735 | 0.0116 | --- |
| - Two-sided p value | 0.1471 | 0.0232 | --- |
| | | | |
| Primary analysis of primary endpoints | | | |
| Stratified Peto-Prentice's generalized Wilcoxon test (vs placebo) [b] | | | |
| - One-sided p value | 0.0204 | 0.0101 | --- |
| - Two-sided p value | 0.0407 | 0.0203 | --- |
| | | | |
| Stratified Cox proportional hazards model [b] | | | |
| - Hazard ratio [95% confidence interval] | 1.14 [0.95, 1.36] | 1.22 (1.03, 1.46) | --- |
| - Two-sided p value | 0.1513 | 0.0253 | --- |

Five symptoms (runny nose or stuffy nose, sore throat, coughing, feverishness or fever, low energy (fatigue))

[a] +: The maximum value includes censored data.
[b] Stratification factor: SARS-CoV-2 vaccination history [Yes, No]

As shown in the above table, in mild/moderate SARS-CoV-2-infected patients (part of the ITT population whose time from onset of COVID-19 to randomization was less than 72 hours), the time to resolution of the five COVID-19 symptoms was shortened by 24.3 hours (about one day) as the median in the 375/125 mg group as compared with the placebo group. In addition, statistically significant differences were also shown (the stratified Peto-Prentice's generalized Wilcoxon test: one-sided p = 0.0204).
Therefore, it was revealed that the administration of the pharmaceutical composition of the present invention exhibits a significantly excellent clinical symptom resolution effect.
The time to resolution of the five COVID-19 symptoms in mild/moderate SARS-CoV-2-infected patients (ITT population) and Kaplan-Meier curves thereof are shown in Table 17 below and Fig. 8.

[Table 17]

| | 375/125 mg group N = 603 | 750/250 mg group N = 595 | Placebo group N = 600 |
|---|---|---|---|
| Nominal significance level (one-sided p value) | 0.025 | --- | --- |
| Number of cases to be analyzed | 582 | 575 | 572 |
| Number of events | 425 (73.0) | 433 (75.3) | 412 (72.0) |
| Number of censored | 157 (27.0) | 142 (24.7) | 160 (28.0) |
| | | | |

(continued)

| | | | |
|---|---|---|---|
| Kaplan-Meier estimated value (hour) | | | |
| Median value [95% confidence interval] | 189.7 [161.6, 216.0] | 177.3 [155.4, 196.9] | 200.3 [180.8,235.2] |
| Difference of median value (vs placebo) [95% confidence interval] | -10.6 [-56.9, 21.3] | -23.0 [-64.6, 4.5] | --- |
| 25% point, 75% point | 104.3, --- | 99.2, 434.8 | 110.3, --- |
| Minimum value, maximum value [a] | 2.4,493.3 + | 9.4,493.1 + | 0.0,492.4 + |
| | | | |
| | | | |
| Restricted mean survival time during 21 days (RMST, hour) [95% confidence interval] | 254.4 [240.1, 268.6] | 245.1 [231.0, 259.2] | 260.0 [245.6, 274.4] |
| Difference of RMST (vs placebo) [95% confidence interval] | -5.6 [-25.9, 14.6] | -14.9 [-35.0, 5.2] | --- |
| | | | |
| Stratified log-rank test (vs placebo) [b] | | | |
| - One-sided p value | 0.3184 | 0.0676 | --- |
| - Two-sided p value | 0.6368 | 0.1351 | --- |
| | | | |
| Key secondary analysis of primary endpoints | | | |
| Stratified Peto-Prentice's generalized Wilcoxon test (vs placebo) [b] | | | |
| - One-sided p value | 0.2176 | 0.0636 | --- |
| - Two-sided p value | 0.4352 | 0.1273 | --- |
| | | | |
| Stratified Cox proportional hazards model [b] | | | |
| - Hazard ratio [95% confidence interval] | 1.03 [0.90, 1.18] | 1.11 [0.97, 1.27] | --- |
| - Two-sided p value | 0.6467 | 0.1324 | --- |

Five symptoms (runny nose or stuffy nose, sore throat, coughing, feverishness or fever, low energy (fatigue))

[a] +: The maximum value includes censored data.
[b] Stratification factor: the time from onset of COVID-19 to randomization [less than 72 hours, 72 hours or more], SARS-CoV-2 vaccination history [Yes, No]

As shown in the above table, no significant difference was observed regarding the time to resolution of the five symptoms of COVID-19 in the mild/moderate SARS-CoV-2-infected patients (ITT population). Incidentally, the time to resolution of the five symptoms of COVID-19 was shortened by 10.6 hours as the numerical value of the median value in the 375/125 mg group as compared with the placebo group.

Results of key secondary endpoints

(1) change from baseline in SARS-CoV-2 viral RNA amount on Day 4 (Phase 3 Part)

The change from baseline in the virus RNA amount on Day 4, in mild/moderate SARS-CoV-2-infected patients (part of the ITT population whose time from onset of COVID-19 to randomization was less than 72 hours), is shown in Table 18 below.

[Table 18]

| | 375/125 mg group N = 347 | 750/250 mg group N = 340 | Placebo group N = 343 |
|---|---|---|---|
| Nominal significance level (one-sided p value) | 0.025 | --- | --- |
| Number of cases to be analyzed | 340 | 333 | 337 |
| Mean value (standard deviation) | -2.737 (1.085) | -2.690 (0.974) | -1.235 (1.528) |
| Minimum value | -5.71 | -5.27 | -5.02 |
| Median value | -2.775 | -2.790 | -1.360 |
| Maximum value | 1.43 | 0.33 | 4.64 |
| | | | |
| Primary analysis of key secondary endpoint | | | |
| Covariance analysis (vs placebo) [a] | | | |
| - Least mean square (standard error) | -2.48 (0.08) | -2.49 (0.08) | -1.01 (0.08) |
| - Difference of least mean square (vs placebo) (standard error) | -1.47 (0.08) | -1.48 (0.08) | --- |
| - 95% confidence interval of difference | -1.63, -1.31 | -1.64, -1.32 | --- |
| - One-sided p value | <.0001 | <.0001 | --- |
| - Two-sided p value | <.0001 | <.0001 | --- |
| | | | |
| van Elteren test (vs placebo) | | | |
| - Two-sided p value | <.0001 | <.0001 | --- |

Unit: $\log_{10}$ copies/mL
Lower limit of quantitation of SARS-CoV-2 viral RNA amount: 2.08 $\log_{10}$ copies/mL

It was handled as 2.27 $\log_{10}$ copies/mL when the RT-PCR of SARS-CoV-2 was negative, and as 2.08 logic copies/mL when the SARS-CoV-2 viral RNA amount was less than the lower limit of quantitation.

[a] Covariates: SARS-CoV-2 viral RNA amount at baseline, SARS-CoV-2 vaccination history [Yes, No]
[b] Stratification factor: SARS-CoV-2 vaccination history [Yes, No]

As shown in the above table, the adjusted estimated value of the change in the SARS-CoV-2 viral RNA amount on Day 4 showed a decrease of about 1.5 $\log_{10}$ (copies/mL) in the 375/125 mg group as compared with the placebo group, and a significant difference was observed (covariance analysis: one-sided $p < 0.0001$).

Therefore, it was revealed that the administration of the pharmaceutical composition of the present invention exhibits a significantly excellent antiviral effect.

The change from baseline in the virus RNA amount on Day 4, in mild/moderate SARS-CoV-2-infected patients (ITT population), is shown in Table 19 below.

[Table 19]

| | 375/125 mg group N = 603 | 750/250 mg group N = 595 | Placebo group N = 600 |
|---|---|---|---|
| Nominal significance level (one-sided p value) | 0.025 | --- | --- |
| Number of cases to be analyzed | 592 | 579 | 589 |
| Mean value (standard deviation) | -2.646 (1.097) | -2.594 (1.010) | -1.419 (1.423) |
| Minimum value | -5.71 | -5.27 | -5.39 |
| Median value | -2.695 | -2.710 | -1.620 |
| Maximum value | 4.34 | 1.01 | 5.10 |

(continued)

| | 375/125 mg group N = 603 | 750/250 mg group N = 595 | Placebo group N = 600 |
|---|---|---|---|
| | | | |
| Key secondary analysis of key secondary endpoint | | | |
| Covariance analysis (vs placebo) [a] | | | |
| - Least mean square (standard error) | -2.46 (0.06) | -2.46 (0.06) | -1.26 (0.06) |
| - Difference of least mean square (vs placebo) (standard error) | -1.20 (0.06) | -1.20 (0.06) | --- |
| - 95% confidence interval of difference | -1.32, -1.08 | -1.32, -1.08 | --- |
| - One-sided p value | <.0001 | <.0001 | --- |
| - Two-sided p value | <.0001 | <.0001 | --- |
| | | | |
| van Elteren test (vs placebo) | | | |
| - Two-sided p value | <.0001 | <.0001 | --- |

Unit: $log_{10}$ copies/mL
Lower limit of quantitation of SARS-CoV-2 viral RNA amount: 2.08 $log_{10}$ copies/mL

It was handled as 2.27 $log_{10}$ copies/mL when the RT-PCR of SARS-CoV-2 was negative, and as 2.08 $log_{10}$ copies/mL when the SARS-CoV-2 viral RNA amount was less than the lower limit of quantitation.

[a] Covariate: SARS-CoV-2 viral RNA amount at baseline, time from onset of COVID-19 to randomization [less than 72 hours, 72 hours or more], SARS-CoV-2 vaccination history [Yes, No]
[b] Stratification factor: the time from onset of COVID-19 to randomization [less than 72 hours, 72 hours or more], SARS-CoV-2 vaccination history [Yes, No]

As shown in the above table, the adjusted estimated value of the change from baseline in the SARS-CoV-2 viral RNA amount on Day 4 showed a decrease of 1.2 $log_{10}$ (copies/mL) in the 375/125 mg group as compared with the placebo group.

(2) Time to the first negative SARS-CoV-2 virus titer(Phase 3 Part)

The time to the first negative SARS-CoV-2 virus titer in mild/moderate SARS-CoV-2-infected patients (part of the mITT population whose time from onset of COVID-19 to randomization was less than 72 hours) and Kaplan-Meier curves thereof are shown in Table 20 below and Fig. 9.

[Table 20]

| | 375/125 mg group N = 203 | 750/250 mg group N = 185 | Placebo group N = 214 |
|---|---|---|---|
| Nominal significance level (one-sided p value) | 0.025 | --- | --- |
| Number of cases to be analyzed | 199 | 183 | 211 |
| Number of events | 199 (100.0) | 183 (100.0) | 210 (99.5) |
| Number of censored | 0 (0.0) | 0 (0.0) | 1 (0.5) |
| | | | |
| Kaplan-Meier estimated value (hour) | | | |
| Median value [95% confidence interval] | 36.2 [23.4,43.2] | 22.7 [21.2,37.9] | 65.3 [62.0,66.8] |
| Difference of median value (vs placebo) [95% confidence interval] | -29.1 [-42.3, -21.1] | -42.6 [-44.6, -27.4] | --- |

(continued)

| | | | |
|---|---|---|---|
| Kaplan-Meier estimated value (hour) | | | |
| 25% point, 75% point | 19.5, 64.0 | 18.2, 60.6 | 21.8,93.6 |
| Minimum value, maximum value [a] | 8.8,139.3 | 11.9, 116.8 | 10.5,303.8 |
| | | | |
| | | | |
| Restricted mean survival time during 21 days (RMST, hour) [95% confidence interval] | 41.4 [37.7, 45.0] | 37.6 [33.9, 41.4] | 68.9 [62.2,75.5] |
| Difference of RMST (vs placebo) [95% confidence interval] | -27.5 [-35.1, -19.9] | -31.2 [-38.8,-23.6] | --- |
| | | | |
| Key analysis of key secondary endpoint | | | |
| Stratified log-rank test (vs placebo) [b] | | | |
| - One-sided p value | <.0001 | <.0001 | --- |
| - Two-sided p value | <.0001 | <.0001 | --- |
| | | | |
| Stratified Peto-Prentice's generalized Wilcoxon test (vs placebo) [b] | | | |
| - Two-sided p value | <.0001 | <.0001 | --- |
| | | | |
| Stratified Cox proportional hazards model [b] | | | |
| - Hazard ratio [95% confidence interval] | 2.20 [1.78, 2.71] | 2.48 [2.00, 3.08] | --- |
| - Two-sided p value | <.0001 | <.0001 | --- |
| [a] +: The maximum value includes censored data.<br>[b] Stratification factor: SARS-CoV-2 vaccination status [Yes, No] | | | |

As indicated above, the time to the first negative SARS-CoV-2 virus titer was reduced by a median of 29.1 hours (about 1 day) in the 375/125 mg group compared to the placebo group, with a statistically significant difference (stratified log-rank test: one-sided p<0.0001).

Therefore, it was revealed that the administration of the pharmaceutical composition of the present invention exhibits a significantly excellent antiviral effect.

The time to the first negative SARS-CoV-2 virus titer in mild/moderate SARS-CoV-2-infected patients (mITT population) and Kaplan-Meier curves thereof are shown in Table 21 below and Fig. 10.

[Table 21]

| | 375/125 mg group N = 318 | 750/250 mg group N = 304 | Placebo group N = 334 |
|---|---|---|---|
| Nominal significance level (one-sided p value) | 0.025 | --- | --- |
| Number of cases to be analyzed | 313 | 301 | 327 |
| Number of events | 313 (100.0) | 300 (99.7) | 326 (99.7) |
| Number of censored | 0 (0.0) | 1 (0.3) | 1 (0.3) |
| | | | |
| Kaplan-Meier estimated value (hour) | | | |
| Median value [95% confidence interval] | 24.5 [22.8, 38.4] | 22.6 [21.3,26.3] | 62.0 [45.0,64.0] |

(continued)

| | | | |
|---|---|---|---|
| Kaplan-Meier estimated value (hour) | | | |
| Difference of median value (vs placebo) [95% confidence interval] | -37.5 [-40.5, -17.4] | -39.4 [-41.9, -22.4] | --- |
| 25% point, 75% point | 18.8, 62.8 | 19.0, 48.0 | 20.8, 88.3 |
| Minimum value, maximum value [a] | 8.8, 139.3 | 11.9, 116.8 | 10.5, 303.8 |
| | | | |
| | | | |
| Restricted mean survival time during 21 days (RMST, hour) [95% confidence interval] | 39.0 [36.2,41.8] | 37.3 [34.5,40.1] | 62.0 [57.0,67.0] |
| Difference of RMST (vs placebo) [95% confidence interval] | -23.0 [-28.7, -17.3] | -24.7 [-30.4, -18.9] | --- |
| | | | |
| Key secondary analysis of key secondary endpoint | | | |
| Stratified log-rank test (vs placebo) [b] | | | |
| - One-sided p value | <.0001 | <.0001 | --- |
| - Two-sided p value | <.0001 | <.0001 | --- |
| | | | |
| Stratified Peto-Prentice's generalized Wilcoxon test (vs placebo) [b] | | | |
| - Two-sided p value | <.0001 | <.0001 | --- |
| | | | |
| Stratified Cox proportional hazards model [b] | | | |
| - Hazard ratio [95% confidence interval] | 2.00 [1.69, 2.36] | 2.02 [1.71,2.39] | --- |
| - Two-sided p value | <.0001 | <.0001 | --- |
| [a] +: The maximum value includes censored data.<br>[b] Stratification factor: the time from onset of COVID-19 to randomization (less than 72 hours, 72 hours or more], SARS-CoV-2 vaccination history [Yes, No] | | | |

As indicated above, the time to the first negative SARS-CoV-2 virus titer was reduced by a median of 37.5 hours (about 1.5 day) in the 375/125 mg group compared to the placebo group.

Results of secondary endpoints

(1) Time to resolution of 12 COVID-19 symptoms (Phase 3 Part)

The time to resolution of 12 COVID-19 symptoms in mild/moderate SARS-CoV-2-infected patients (part of the ITT population whose time from onset of COVID-19 to randomization was less than 72 hours) and Kaplan-Meier curves thereof are shown in Table 22 below and Fig. 11.

[Table 22]

| | 375/125 mg group N = 347 | 750/250 mg group N = 340 | Placebo group N = 343 |
|---|---|---|---|
| Number of cases to be analyzed | 336 | 330 | 321 |
| Number of events | 244 (72.6) | 258 (78.2) | 227 (70.7) |
| Number of censored | 92 (27.4) | 72 (21.8) | 94 (29.3) |

(continued)

| | 375/125 mg group N = 347 | 750/250 mg group N = 340 | Placebo group N = 343 |
|---|---|---|---|
| | | | |
| Kaplan-Meier estimated value (hour) | | | |
| Median value [95% confidence interval] | 179.2 [152.1, 212.1] | 184.9 [168.9, 226.2] | 213.2 [185.8,253.8] |
| Difference of median value (vs placebo) [95% confidence interval] | -34.0 [-85.9, 8.3] | -28.3 [-72.8, 14.7] | --- |
| 25% point, 75% point | 105.6, --- | 114.2,422.7 | 123.0, --- |
| Minimum value, maximum value [a] | 2.4, 489.1 + | 9.4, 490.1 + | 13.9, 492.4 + |
| | | | |
| | | | |
| Restricted mean survival time during 21 days (RMST, hour) [95% confidence interval] | 253.9 [235.1,272.7] | 249.9 [232.2, 267.7] | 270.8 [252.2,289.3] |
| Difference of RMST (vs placebo) [95% confidence interval] | -16.9 [-43.3, 9.5] | -20.8 [-46.5,4.8] | --- |
| | | | |
| Stratified log-rank test (vs placebo) [b] | | | |
| - One-sided p value | 0.1184 | 0.0253 | --- |
| - Two-sided p value | 0.2367 | 0.0505 | --- |
| | | | |
| Stratified Peto-Prentice's generalized Wilcoxon test (vs placebo) [b] | | | |
| - Two-sided p value | 0.0651 | 0.0843 | --- |
| | | | |
| Stratified Cox proportional hazards model [b] | | | |
| - Hazard ratio [95% confidence interval] | 1.11 [0.93, 1.33] | 1.19 [1.00, 1.42] | --- |
| - Two-sided p value | 0.2441 | 0.0558 | --- |

Twelve symptoms (low energy (fatigue); pains in muscles or body; headache; chills/sweats; feverishness or fever; runny nose or stuffy nose; sore throat; coughing; shortness of breath (dyspnea); nausea; emesis; and diarrhea)

[a] +: The maximum value includes censored data.
[b] Stratification factor: SARS-CoV-2 vaccination history [Yes, No]

As indicated above, the time to resolution of 12 COVID-19 symptoms tended to be reduced by a median of 34.0 hours (about 1.5 day) in the 375/125 mg group compared to the placebo group.

(2) Time to resolution of 14 COVID-19 symptoms (Phase 3 Part)

The times to resolution of 14 COVID-19 symptoms in mild/moderate SARS-CoV-2-infected patients (part of the ITT population whose time from onset of COVID-19 to randomization was less than 72 hours) and Kaplan-Meier curves thereof are shown in Table 23 below and Fig. 12.

[Table 23]

| | 375/125 mg group N = 347 | 750/250 mg group N = 340 | Placebo group N = 343 |
|---|---|---|---|
| Number of cases to be analyzed | 336 | 330 | 321 |
| Number of events | 241 (71.7) | 255 (77.3) | 224 (69.8) |
| Number of censored | 95 (28.3) | 75 (22.7) | 97 (30.2) |
| | | | |
| Kaplan-Meier estimated value (hour) | | | |
| Median value [95% confidence interval] | 187.8 [156.4,217.0] | 190.3 [171.4, 244.0] | 231.8 [192.1,265.8] |
| Difference of median value (vs placebo) [95% confidence interval] | -44.1 [-95.3, 4.5] | -41.5 [-81.2, 27.3] | --- |
| 25% point, 75% point | 105.6, --- | 117.1,431.6 | 129.7, --- |
| Minimum value, maximum value [a] | 2.4, 489.1 + | 9.4,490.1 + | 13.9, 492.4 + |
| | | | |
| | | | |
| Restricted mean survival time during 21 days (RMST, hour) [95% confidence interval] | 257.9 [239.0,276.8] | 256.0 [238.1, 273.8] | 278.0 [259.5, 296.4] |
| Difference of RMST (vs placebo) [95% confidence interval] | -20.1 [-46.5, 6.3] | -22.0 [-47.7, 3.7] | --- |
| | | | |
| | | | |
| Stratified log-rank test (vs placebo) [b] | 0.0892 | 0.0206 | --- |
| . One-sided p value | 0.1784 | 0.0412 | --- |
| . Two-sided p value | | | |
| Stratified Peto-Prentice's generalized Wilcoxon test (vs placebo) [b] | | | |
| - Two-sided p value | 0.0304 | 0.0525 | --- |
| | | | |
| Stratified Cox proportional hazards model [b] | | | |
| - Hazard ratio [95% confidence interval] | 1.13 [0.94, 1.36] | 1.20 [1.00, 1.44] | --- |
| - Two-sided p value | 0.1867 | 0.0455 | --- |

Fourteen symptoms (low energy (fatigue); pains in muscles or body; headache; chills/sweats; feverishness or fever; runny nose or stuffy nose; sore throat; coughing; shortness of breath (dyspnea); nausea; emesis; diarrhea; dysgeusia; and dysosmia)

[a] +: The maximum value includes censored data.
[b] Stratification factor: SARS-CoV-2 vaccination history [Yes, No]

As indicated above, the time to resolution of 14 COVID-19 symptoms tended to be reduced by a median of 44.1 hours (about 2 day) in the 375/125 mg group compared to the placebo group.

Frequency of adverse events

In Phase 2a Part, there were no deaths, serious adverse events, or adverse events leading to discontinuation of administration.

In addition, in Phase 2a Part, there were no adverse events leading to deaths. Two serious adverse events were observed in the placebo group, but not in the active drug group. Two adverse events leading to the discontinuation of administration were observed in the 375/125 mg group (active drug group), and both were determined to have a causal relationship with the investigational new drug.

In Phase 3 Part, there were no adverse events leading to deaths. A serious adverse event was observed in one case in each of the 375/125 mg group and the placebo group, but it was determined that there was no causal relationship with the investigational new drug in any case. In the adverse events leading to the discontinuation of administration, eczema and emesis in the 375/125 mg group, rash (2 cases) in the 750/250 mg group, and muscle weakness and hypesthesia in the placebo group were determined to be causally related to the investigational new drug, but all of them were recovering or recovered after the discontinuation of administration of the study drug.

Test Example 5 Pediatric clinical study

The safety and pharmacokinetics of repeated oral administration of the active drug (active ingredient: fumaric acid cocrystal Form I crystal of the compound represented by Formula (I)) to mild/moderate SARS-CoV-2-infected patients who are 6 years old or older and under 12 years old were evaluated by a placebo-controlled, randomized, double-blind, comparative study.

The primary endpoints of this study are adverse events, clinical examinations, and vital signs regarding safety, and plasma concentrations on Day 2 and Day 6 regarding pharmacokinetics.

Patients meeting all of the following criteria are selected.

(a) Male or female patients who are 6 years old or older and younger than 12 years old.
(b) Those diagnosed as SARS-CoV-2-positive within 72 hours prior to enrollment.
(c) Those whose time from onset of COVID-19 to enrollment is within 72 hours.
(d) Those having one or more moderate (COVID-19 symptom score: 2) or higher symptoms in any of the following symptoms due to COVID-19 (twelve COVID-19 symptoms) (except for symptoms that were present before onset of COVID-19) at the time of enrollment.

Systemic symptoms: low energy and tiredness, muscle or body aches, headache, chills or shivering, feeling hot or feverish
Respiratory symptoms: stuffy and runny nose, sore throat, cough, shortness of breath (difficulty breathing)
Gastrointestinal symptoms: nausea, vomiting, diarrhea

(e) Those with a body weight of 20 kg or more.

Method of administration of investigational new drug

(i) Active drug

25 mg tablet: The tablet contains fumaric acid cocrystal Form I crystal of the compound represented by Formula (I), and contains 25 mg of the compound represented by Formula (I).

125 mg tablet: The tablet contains fumaric acid cocrystal Form I crystal of the compound represented by Formula (I), and contains 125 mg of the compound represented by Formula (I).

(ii) Placebo

Placebo-A tablet: It is a tablet having an appearance indistinguishable from the above 25 mg tablet, and does not contain the fumaric acid cocrystal Form I crystal of the compound represented by Formula (I).

Placebo-B tablet: It is a tablet having an appearance indistinguishable from the above 125 mg tablet, and does not contain the fumaric acid cocrystal Form I crystal of the compound represented by Formula (I).

Dose and method of administration

Subjects determined to be eligible are randomly assigned at a ratio of 2:1 to either the active drug group or the placebo group at a ratio of 2:1 for each weight category.

The administration of the active drug is repeated oral administration once a day for 5 days. Those in the category of 40 kg or more receive 375 mg on Day 1 as a loading dose and 125 mg on Days 2 to 5 as a maintenance dose. Those in the category of 30 kg or more and less than 40 kg receive 250 mg on Day 1 as a loading dose and 125 mg on Days 2 to 5 as a

maintenance dose. Those in the category of 20 kg or more and less than 30 kg receive 125 mg on Day 1 as a loading dose and 75 mg on Days 2 to 5 as a maintenance dose.

In addition, other exemplary doses to be administered are as follows.

Those in the category of 40 kg or more receive 375 mg on Day 1 as a loading dose and 125 mg on Days 2 to 5 as a maintenance dose. Those in the category of 30 kg or more and less than 40 kg receive 250 mg on Day 1 as a loading dose and 100 mg on Days 2 to 5 as a maintenance dose. Those in the category of 20 kg or more and less than 30 kg receive 150 mg on Day 1 as a loading dose and 75 mg on Days 2 to 5 as a maintenance dose.

Investigational new drug by weight category

Active drug group

40 kg or more

Three 125 mg tablets are administered on Day 1, and one 125 mg tablet is administered per day on Days 2 to 5.

30 kg or more and less than 40 kg

Two 125 mg tablets are administered on Day 1, and one 125 mg tablet is administered per day on Days 2 to 5.

20 kg or more and less than 30 kg

One 125 mg tablet is administered on Day 1, and three 25 mg tablets are administered per day on Days 2 to 5.

In addition, still other exemplary doses to be administered are as follows.

40 kg or more

Three 125 mg tablets are administered on Day 1, and one 125 mg tablet is administered per day on Days 2 to 5.

30 kg or more and less than 40 kg

Two 125 mg tablets are administered on Day 1, and four 25 mg tablets are administered per day on Days 2 to 5.

20 kg or more and less than 30 kg

One 125 mg tablet and one 25 mg tablet are administered on Day 1, and three 25 mg tablets are administered per day on Days 2 to 5.

Placebo group

40 kg or more

Three Placebo-B tablets are administered on Day 1, and one Placebo-B tablet is administered per day on Days 2 to 5.

30 kg or more and less than 40 kg

Two Placebo-B tablets are administered on Day 1, and one Placebo-B tablet is administered per day on Days 2 to 5.

20 kg or more and less than 30 kg

One Placebo-B tablet is administered on Day 1, and three Placebo-B tablets are administered per day on Days 2 to 5.

In addition, still other exemplary doses to be administered are as follows.

40 kg or more

Three Placebo-B tablets are administered on Day 1, and one Placebo-B tablet is administered per day on Days 2 to 5.

30 kg or more and less than 40 kg

Two Placebo-B tablets are administered on Day 1, and four Placebo-A tablets are administered per day on Days 2 to 5.

20 kg or more and less than 30 kg

One Placebo-B tablet and one Placebo-A tablet are administered on Day 1, and three Placebo-A tablets are administered per day on Days 2 to 5.

"Day 1" represents the first day of administration, and "Day 2 to Day 5" represent the second to fifth days counted from the first day of administration.

Primary safety endpoints

Adverse events, clinical examinations, vital signs

Primary pharmacokinetics endpoints

Plasma concentrations of active drug on Day 2 and Day 6

Analysis of primary endpoints: safety

Adverse events are classified according to System Organ Class (SOC) and Preferred Terms (PT) of ICH Medical Dictionary for Regulatory Activities (MedDRA). Among the adverse events reported in electronic case report form (eCRF), adverse events that occurred after the first administration of the investigational new drug are used for safety analysis.

The number of treatment-emergent adverse events (TEAEs), deaths, other serious TEAEs, and TEAEs in which administration was discontinued as well as the ratios thereof are tabulated for each dose. The 95% confidence interval of these occurrence rates is calculated by the Clopper-Pearson method. The number of reported adverse events is also shown. An adverse event that has been determined to be "associated" with the investigational new drug regarding causal relationship therebetween is defined as an adverse reaction, and is summarized in the same manner as that for TEAE.

The summary statistic is calculated for each dose group for the amount of change at each time point based on the observation value at the measurement time point scheduled after randomization and the baseline. Qualitative clinical examination data create a shift table between the baseline and the scheduled measurement time points for examination categories.

The summary statistic is calculated for each dose group for the amount of change at each time point based on the observation value at the measurement time point scheduled after randomization and the baseline.

Analysis of primary endpoints: pharmacokinetics

For the PK concentration population, a list of plasma concentrations of the compound represented by Formula (I) is prepared for each body weight category and Day together with the elapsed time from administration of the investigational new drug immediately before blood collection to blood collection. In addition, the concentrations of the compound represented by Formula (I) in the plasma at a blood collection time that is 24 hours after administration on Day 2 and Day 6 are tabulated, and summarized as N, Mean, SD, coefficient of variation, geometric mean value and coefficient of variation thereof, median value, minimum value, and maximum value.

Test Example 6 Clinical study of preexposure prophylactic administration at home

This study is a randomized, double-blind, multicenter, parallel-group, placebo-controlled comparative study involving approximately 2,040 household cohabitants of newly diagnosed symptomatic COVID-19 patients. Subjects are randomly assigned at a ratio of 1 : 1 to either the 375/125 mg active drug group or the placebo group. It is defined to be the proportion of subjects infected with SARS-CoV-2 by Day 10 (confirmed RT-PCR positive by central laboratory) and having developed COVID-19 symptoms.

As the newly diagnosed symptomatic COVID-19 patients, those meeting the following criteria are selected.

- No age restriction
- The first COVID-19 patient among the household cohabitants in the six months before obtaining consent
- Having one or more COVID-19 symptoms within 24 hours after obtaining consent
- SARS-CoV-2 positive in respiratory tract specimens collected up to 72 hours before randomization

As the household cohabitants, subjects meeting the following criteria are selected.

- 12 years old or older
- Negative for SARS-CoV-2 at the time of screening by SARS-CoV-2 test using respiratory tract specimen
- Not determined to be infected with SARS-CoV-2 by the principal investigator (co-investigator)
- Living together with the newly diagnosed patient and living together with the same household cohabitant(s) until the end of the examination

Method of administration of investigational new drug

(i) Active drug

125 mg tablet: The tablet contains fumaric acid cocrystal Form I crystal of the compound represented by Formula (I), and contains 125 mg of the compound represented by Formula (I).

(ii) Placebo

Placebo tablet: It is a tablet having an appearance indistinguishable from the above 125 mg tablet, and does not contain the fumaric acid cocrystal Form I crystal of the compound represented by Formula (I).

Dose and method of administration

Newly diagnosed symptomatic COVID-19 patients are randomly assigned at a ratio of 1 : 1 to either the active drug group or the placebo group.
The administration of the active drug is repeated oral administration once a day for 5 days. The patient receives 375 mg on Day 1 as a loading dose and 125 mg on Days 2 to 5 as a maintenance dose.
The follow-up period is 10 days after the investigational new drug is administered for 5 days.

Investigational new drug by treatment group

Active drug group

Three 125 mg tablets are administered on Day 1, and one 125 mg tablet is administered per day on Days 2 to 5.

Placebo group

Three Placebo tablets are administered on Day 1, and one Placebo tablet is administered per day on Days 2 to 5.

"Day 1" represents the first day of administration, and "Day 2 to Day 5" represent the second to fifth days counted from the first day of administration.

The primary endpoint is the proportion of subjects infected with SARS-CoV-2 by Day 10 (confirmed RT-PCR positive by central laboratory) and having developed COVID-19 symptoms. The subjects infected with SARS-CoV-2 by Day 10 (confirmed RT-PCR positive by central laboratory) and having developed COVID-19 symptoms are subjects who meet both the following two criteria among the mITT population.

- Infection with SARS-CoV-2 confirmed by RT-PCR positive determination in central laboratory by Day 10 after initial administration of investigational new drug
- Onset of COVID-19 symptoms by Day 10 after initial administration of investigational new drug (see definitions below)

The definition of the onset of COVID-19 symptoms in a subject shall meet any one or more of the following criteria:

- One or more of the following symptoms are observed (or if COVID-19 like symptoms are observed at baseline, the symptoms are worsened): (a) fever or (b) shortness of breath or difficulty breathing.

- Two or more of the following seven COVID-19 symptoms are observed for 24 hours or more (or if COVID-19 like symptoms are observed at baseline, the symptoms are worsened): (1) nasal congestion or runny nose; (2) sore throat; (3) cough; (4) feverishness, (5) low energy, (6) dysgeusia, (7) dysosmia.

Analysis of primary endpoint: for the main solution analysis, a modified Poisson regression model using robust variance is used. A risk ratio of the active drug group to that of the placebo group is estimated, and the p value for the null hypothesis with the risk ratio of 1 and the 95% confidence interval (CI) of the risk ratio are calculated using the modified Poisson regression model. Exemplary covariates in the model include the time from onset of COVID-19 symptoms in the newly diagnosed patient to the time of subject inclusion (less than 48 hours, 48 hours or more), and the geographic region (North America, South America, Europe, Africa, Japan, and Asia other than Japan).

Formulation Example

The preparation examples shown below are only for illustrative purposes and are by no means intended to limit the scope of the invention.

(Formulation Example 1) Suspension

For example, water for injection was added to the active pharmaceutical ingredient, the compound represented by Formula (I), to prepare a suspension.

(Formulation Example 2) Tablets

For example, D-mannitol and magnesium stearate were added as additives to the active pharmaceutical ingredient, the compound represented by Formula (I), to prepare tablets.

(Formulation Example 3) Tablets

To 125 mg of the compound represented by Formula (I) (152.3 mg of fumaric acid cocrystal Form I crystal of the compound represented by Formula (I)), D-mannitol, croscarmellose sodium, hydroxypropyl cellulose, light anhydrous silicic acid, crystalline cellulose, and magnesium stearate were added as additives to prepare tablets.

(Formulation Example 4) Tablets

To 25 mg of the compound represented by Formula (I) (30.46 mg of fumaric acid cocrystal Form I crystal of the compound represented by Formula (I)), D-mannitol, croscarmellose sodium, hydroxypropyl cellulose, light anhydrous silicic acid, crystalline cellulose, and magnesium stearate were added as additives to prepare tablets.

(Formulation Example 5) Granules

For example, D-mannitol and magnesium stearate were added as additives to the active pharmaceutical ingredient, the compound represented by Formula (I), to prepare granules.

(Formulation Example 6) Granules

To 125 mg of the compound represented by Formula (I) (152.3 mg of fumaric acid cocrystal Form I crystal of the compound represented by Formula (I)), D-mannitol, powdered reduced maltose syrup (maltitol), croscarmellose sodium, hydroxypropyl cellulose, light anhydrous silicic acid, magnesium stearate, and sucralose were added as additives to prepare granules.

[INDUSTRIAL APPLICABILITY]

The method for treating novel coronavirus infections (COVID-19) and the pharmaceutical composition used therefor according to the present invention is considered to exhibit an excellent therapeutic effect by administering a predetermined amount of a complex as an active ingredient containing a compound represented by Formula (I) and

fumaric acid to a patient. In addition, it is a highly safe pharmaceutical composition for treating COVID-19 as it can maintain an effective concentration in blood without exceeding NOAEL when being administered at a specific dosage regimen.

## Claims

1. A pharmaceutical composition for treating novel coronavirus infections (COVID-19), the composition containing, as an active ingredient, a complex that contains:

   a compound represented by Formula (I):

   [Chemical Formula 1]

   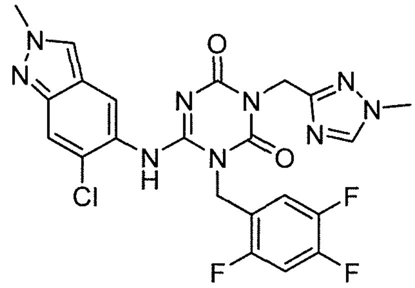

   (I)

   ;

   and
   fumaric acid.

2. The pharmaceutical composition according to claim 1, wherein a patient infected with novel coronavirus infections (COVID-19) is classified as asymptomatic, mild illness or moderate illness I.

3. The pharmaceutical composition according to claim 1 or 2, which is used for reducing a disease duration of novel coronavirus infections (COVID-19).

4. The pharmaceutical composition according to any one of claims 1 to 3, which is used for inhibiting virus replication of SARS-CoV-2.

5. The pharmaceutical composition according to any one of claims 1 to 4, wherein the daily dose of the active ingredient of the pharmaceutical composition is 20 mg to 2000 mg.

6. The pharmaceutical composition according to any one of claims 1 to 5, wherein the daily dose of the active ingredient on Treatment Day 1 is 375 mg and the daily dose of the active ingredient from Treatment Day 2 to Treatment Day 5 is 125 mg each.

7. The pharmaceutical composition according to any one of claims 1 to 5, wherein the daily dose of the active ingredient on Treatment Day 1 is 750 mg and the daily dose of the active ingredient from Treatment Day 2 to Treatment Day 5 is 250 mg each.

8. The pharmaceutical composition according to any one of claims 1 to 7, which is administered once a day.

9. The pharmaceutical composition according to any of claim 1 to 8, wherein the pharmaceutical composition is an orally administered drug.

[Fig.1]

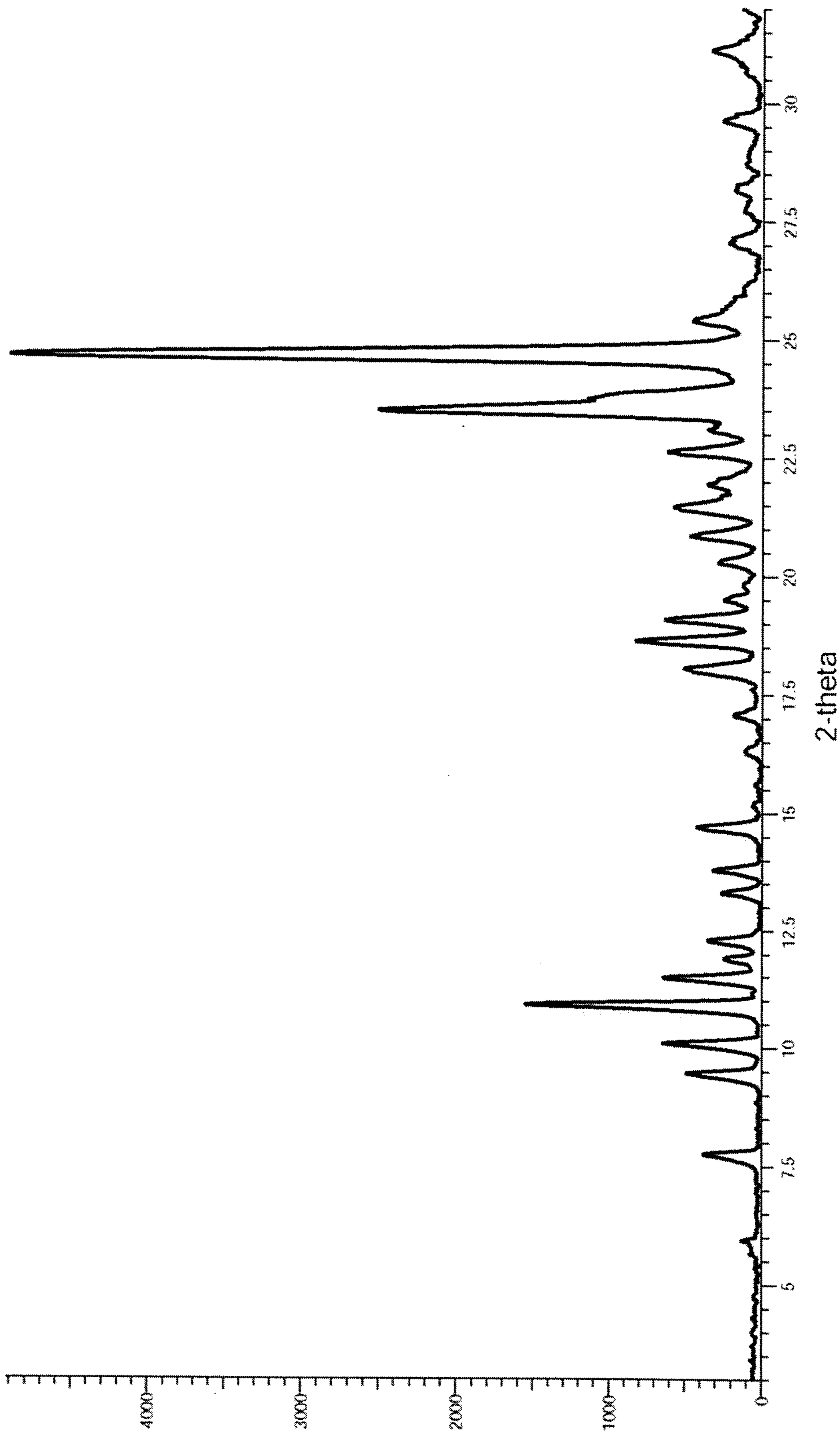

[Fig.2]

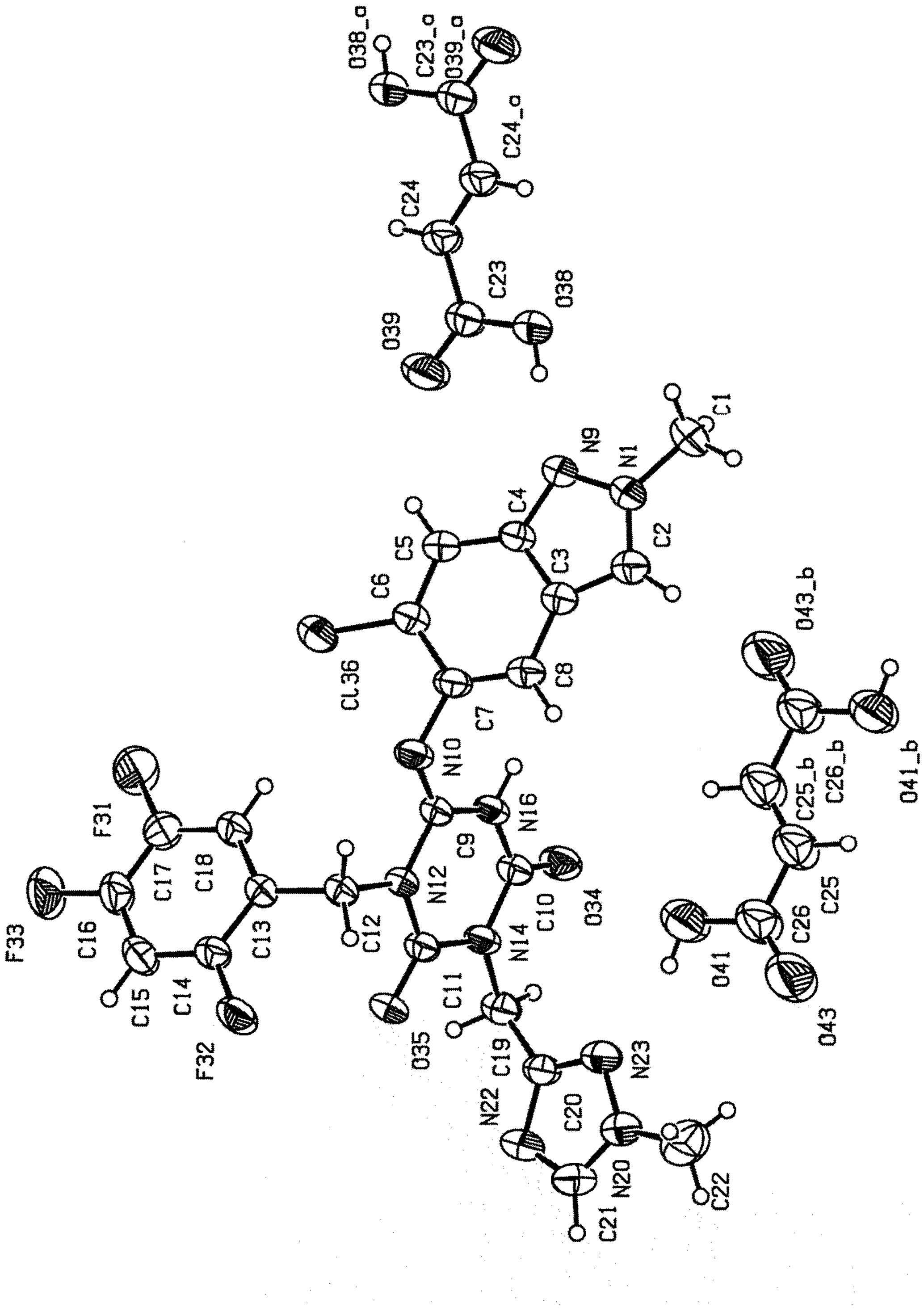

[Fig.3]

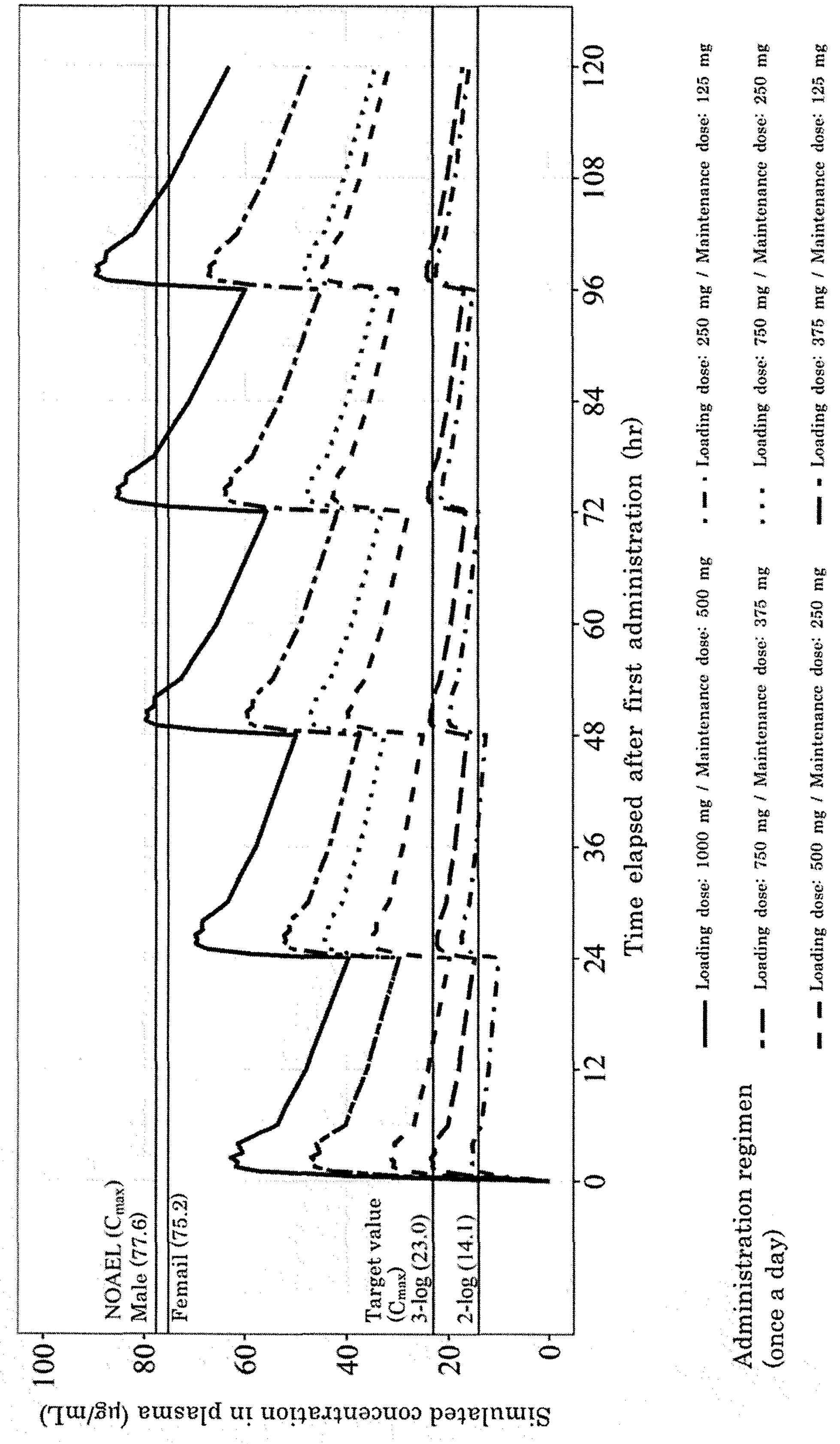
Simulated concentration in plasma (μg/mL)
100
80
60
40
20
0
NOAEL ($C_{max}$)
Male (77.6)
Femail (75.2)
Target value ($C_{max}$)
3-log (23.0)
2-log (14.1)
0
12
24
36
48
60
72
84
96
108
120
Time elapsed after first administration (hr)
Administration regimen (once a day)
Loading dose: 1000 mg / Maintenance dose: 500 mg
Loading dose: 250 mg / Maintenance dose: 125 mg
Loading dose: 750 mg / Maintenance dose: 375 mg
Loading dose: 750 mg / Maintenance dose: 250 mg
Loading dose: 500 mg / Maintenance dose: 250 mg
Loading dose: 375 mg / Maintenance dose: 125 mg

[Fig.4]

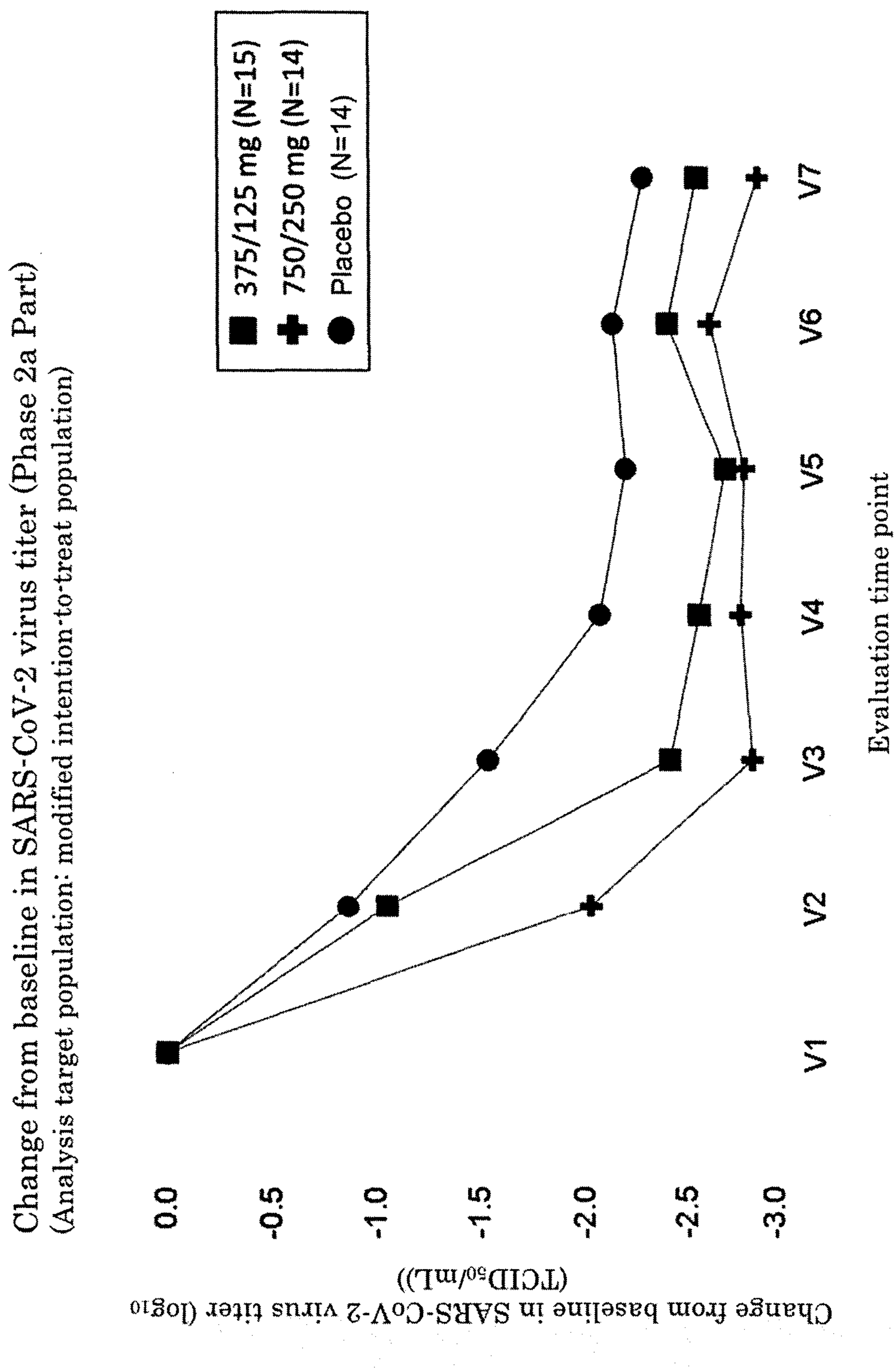
Change from baseline in SARS-CoV-2 virus titer (Phase 2a Part)
(Analysis target population: modified intention-to-treat population)
375/125 mg (N=15)
750/250 mg (N=14)
Placebo (N=14)
0.0
-0.5
-1.0
-1.5
-2.0
-2.5
-3.0
Change from baseline in SARS-CoV-2 virus titer (log10 (TCID50/mL))
V1
V2
V3
V4
V5
V6
V7
Evaluation time point

[Fig.5]

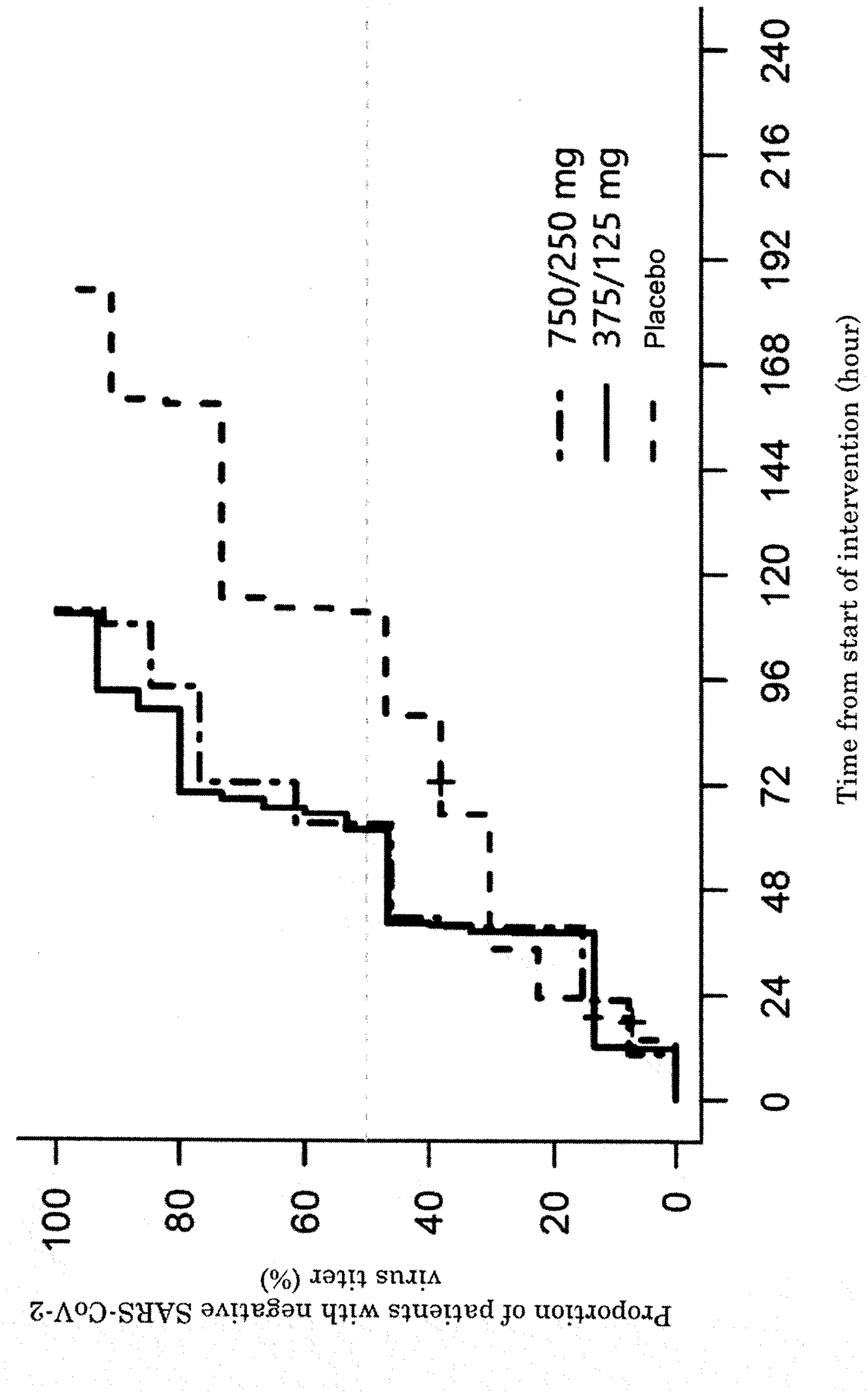
750/250 mg
375/125 mg
Placebo
Time from start of intervention (hour)
Proportion of patients with negative SARS-CoV-2 virus titer (%)
0
24
48
72
96
120
144
168
192
216
240
0
20
40
60
80
100

[Fig.6]

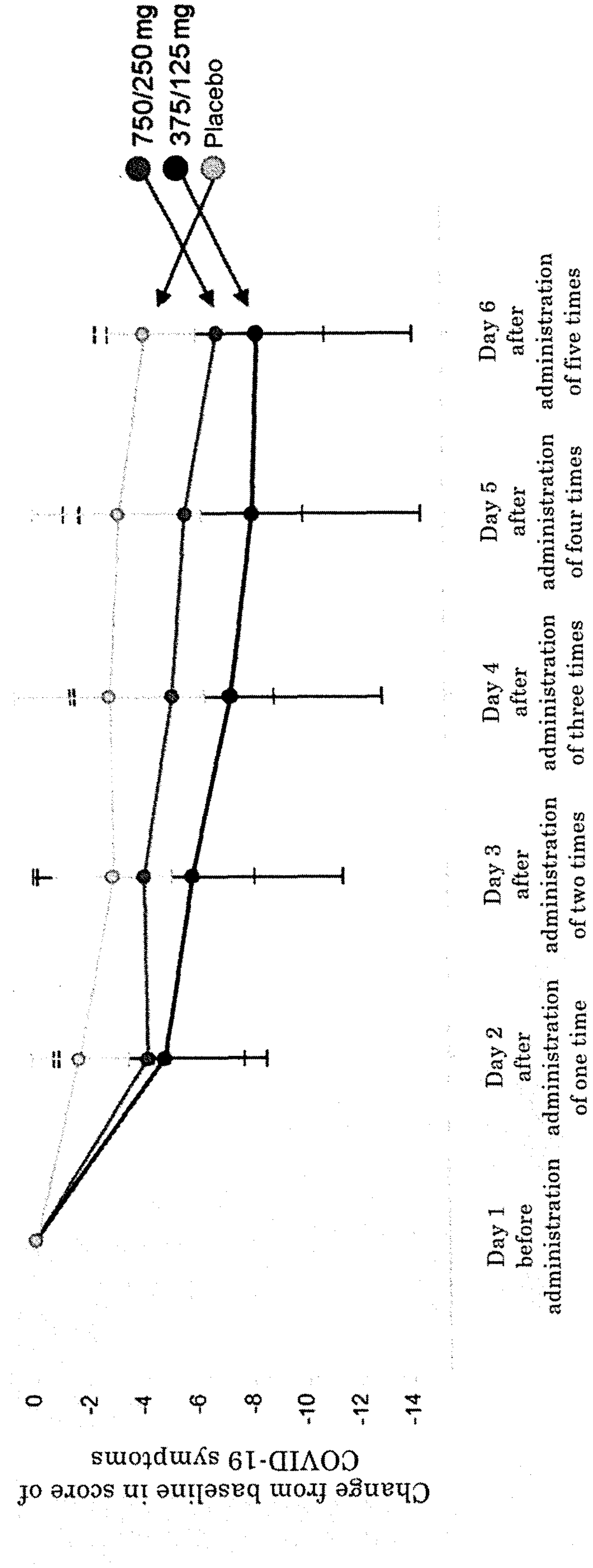
750/250 mg
375/125 mg
Placebo
Change from baseline in score of COVID-19 symptoms
0
-2
-4
-6
-8
-10
-12
-14
Day 1 before administration
Day 2 after administration of one time
Day 3 after administration of two times
Day 4 after administration of three times
Day 5 after administration of four times
Day 6 after administration of five times

[Fig.7]

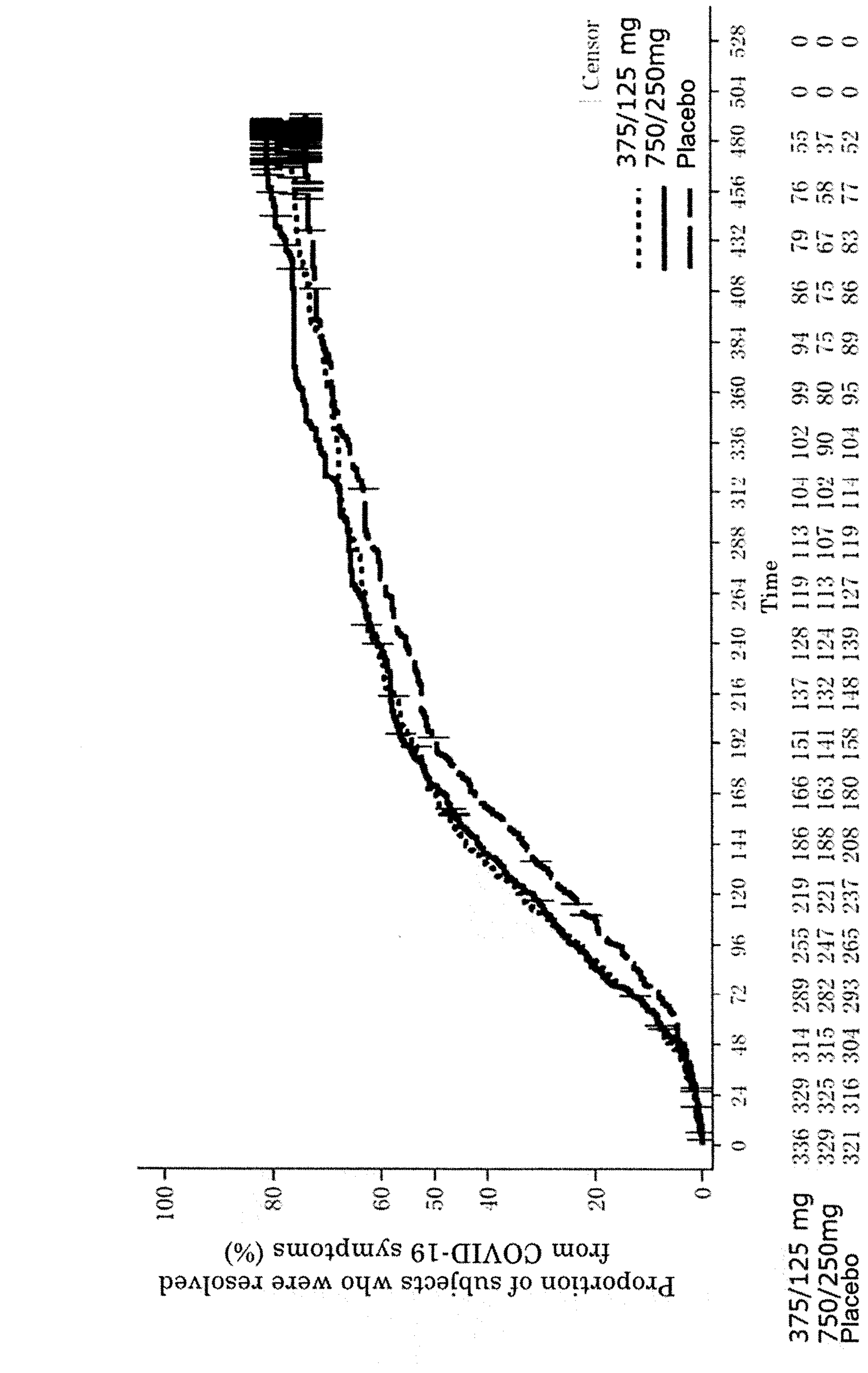
Proportion of subjects who were resolved from COVID-19 symptoms (%)
0
20
40
50
60
80
100
Time
0 24 48 72 96 120 144 168 192 216 240 264 288 312 336 360 384 408 432 456 480 504 528
Censor
375/125 mg
750/250mg
Placebo
375/125 mg 336 329 314 289 255 219 186 166 151 137 128 119 113 104 102 99 94 86 79 76 55 0 0
750/250mg 329 325 315 282 247 221 188 163 141 132 124 113 107 102 90 80 75 75 67 58 37 0 0
Placebo 321 316 304 293 265 237 208 180 158 148 139 127 119 114 104 95 89 86 83 77 52 0 0

[Fig.8]

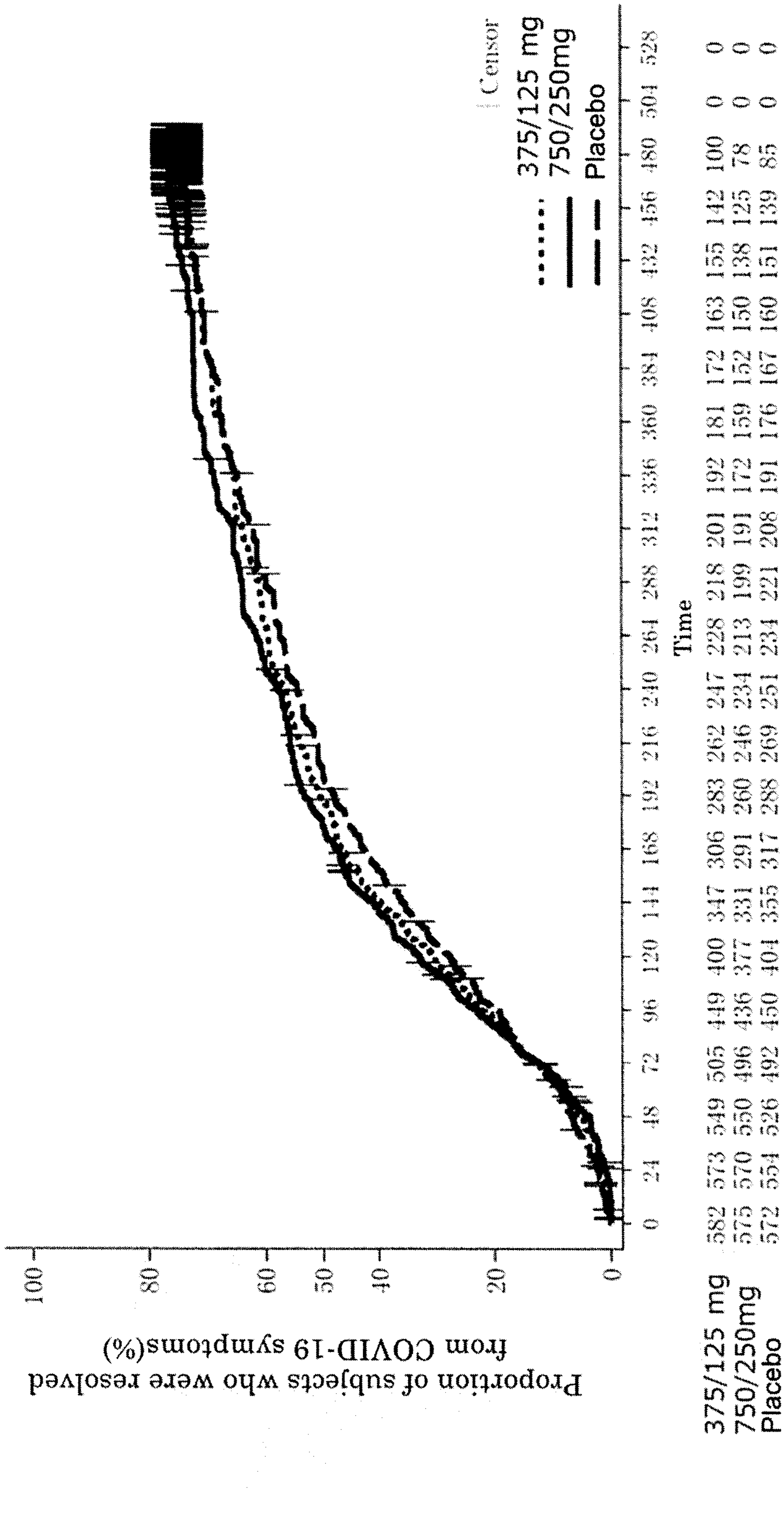
Proportion of subjects who were resolved from COVID-19 symptoms(%)
0
20
40
50
60
80
100
Time
0 24 48 72 96 120 144 168 192 216 240 264 288 312 336 360 384 408 432 456 480 504 528
Censor
375/125 mg
750/250mg
Placebo
375/125 mg 582 573 549 505 449 400 347 306 283 262 247 228 218 201 192 181 172 163 155 142 100 0 0
750/250mg 575 570 550 496 436 377 331 291 260 246 234 213 199 191 172 159 152 150 138 125 78 0 0
Placebo 572 554 526 492 450 404 355 317 288 269 251 234 221 208 191 176 167 160 151 139 85 0 0

[Fig.9]

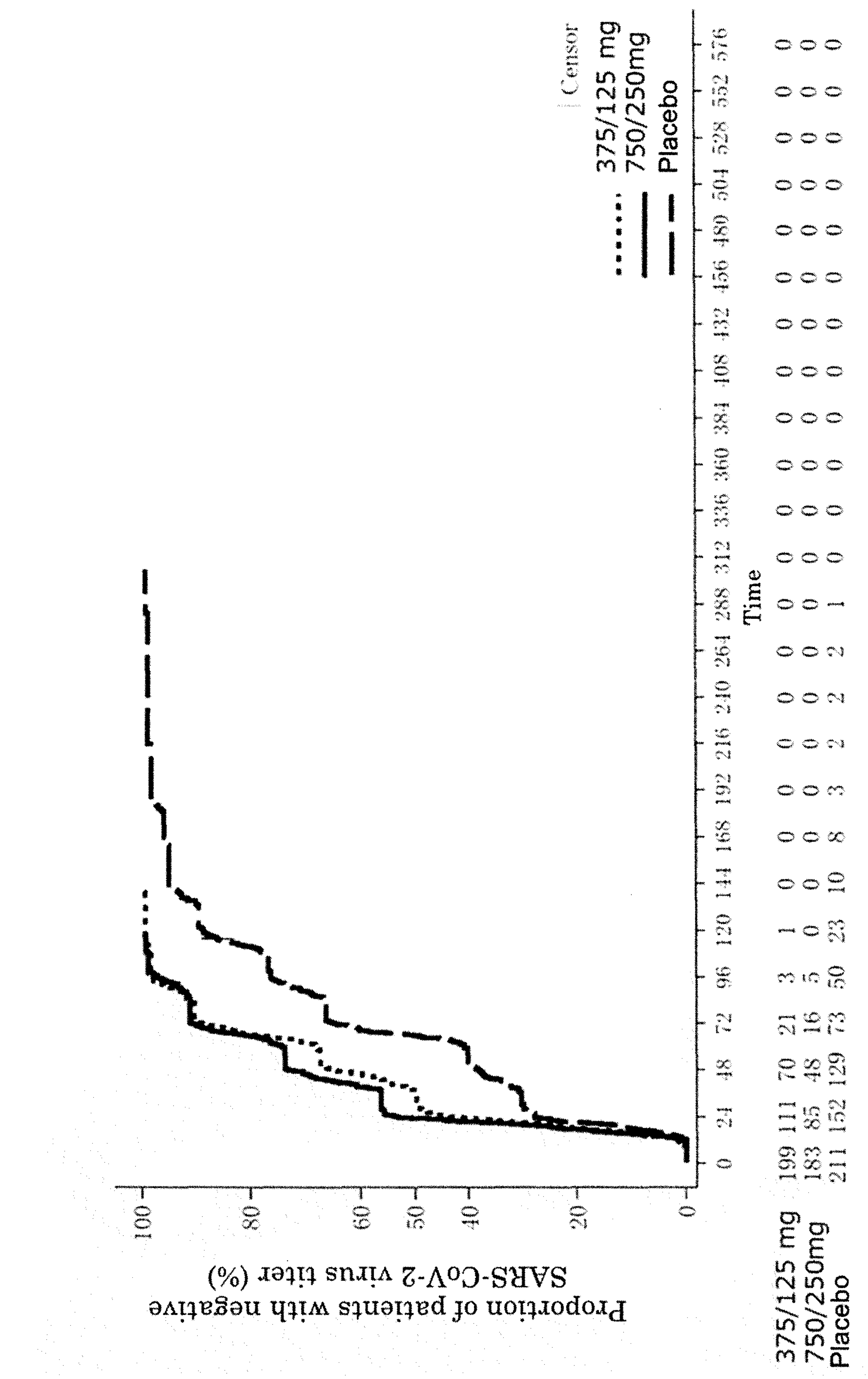
Proportion of patients with negative SARS-CoV-2 virus titer (%)
0
20
40
50
60
80
100
Time
0 24 48 72 96 120 144 168 192 216 240 264 288 312 336 360 384 408 432 456 480 504 528 552 576
Censor
375/125 mg
750/250mg
Placebo
375/125 mg 199 111 70 21 3 1 0 0 0 0 0 0 0 0 0 0 0 0 0 0 0 0 0 0 0
750/250mg 183 85 48 16 5 0 0 0 0 0 0 0 0 0 0 0 0 0 0 0 0 0 0 0 0
Placebo 211 152 129 73 50 23 10 8 3 2 2 2 1 0 0 0 0 0 0 0 0 0 0 0 0

[Fig.10]

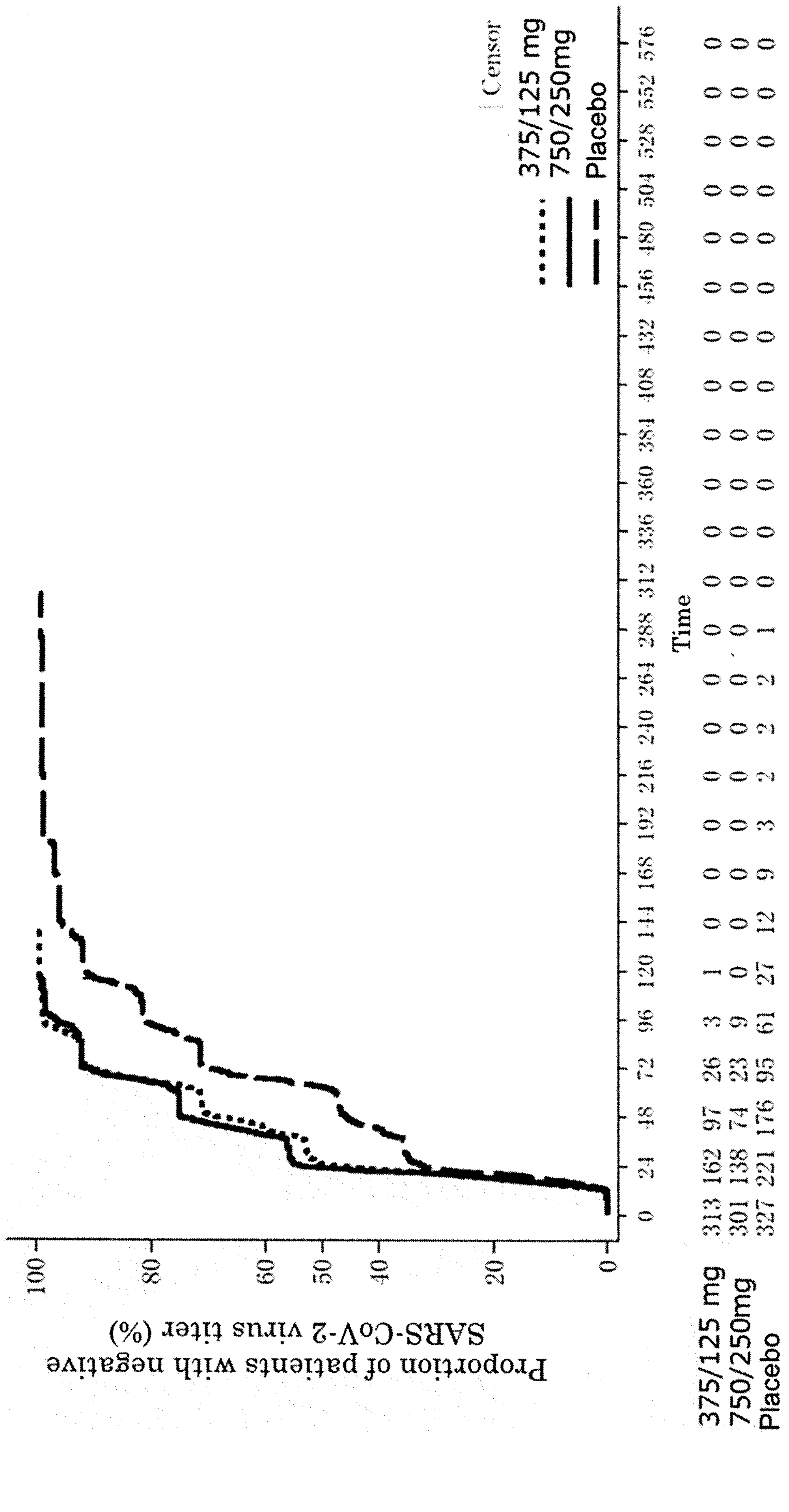
Proportion of patients with negative SARS-CoV-2 virus titer (%)
Time
Censor
375/125 mg
750/250mg
Placebo
375/125 mg 313 162 97 26 3 1 0 0 0 0 0 0 0 0 0 0 0 0 0 0 0 0 0 0 0
750/250mg 301 138 74 23 9 0 0 0 0 0 0 0 0 0 0 0 0 0 0 0 0 0 0 0 0
Placebo 327 221 176 95 61 27 12 9 3 2 2 2 1 0 0 0 0 0 0 0 0 0 0 0 0

[Fig.11]

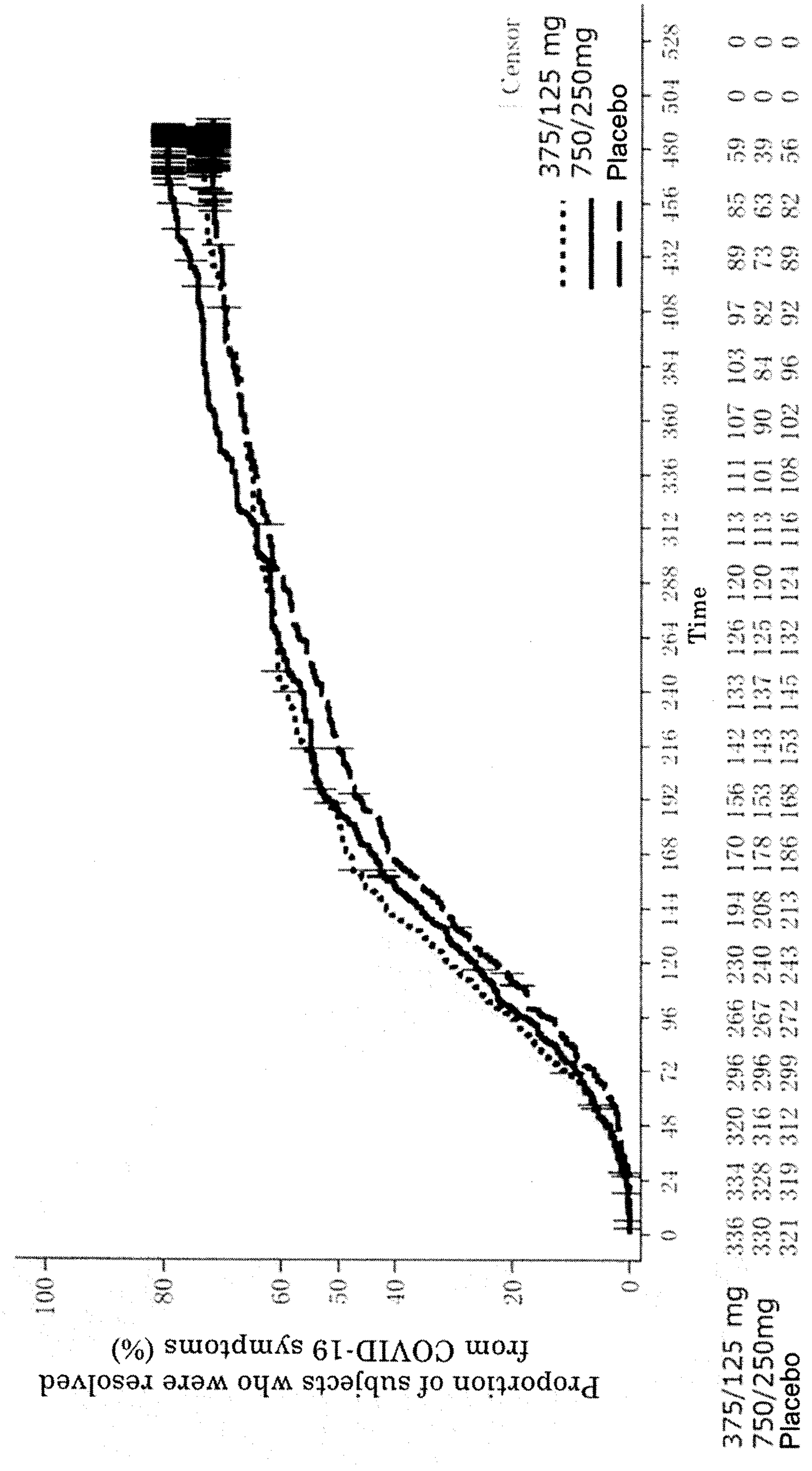
Proportion of subjects who were resolved from COVID-19 symptoms (%)
0
20
40
50
60
80
100
Time
0 24 48 72 96 120 144 168 192 216 240 264 288 312 336 360 384 408 432 456 480 504 528
Censor
375/125 mg
750/250mg
Placebo
375/125 mg 336 334 320 296 266 230 194 170 156 142 133 126 120 113 111 107 103 97 89 85 59 0 0
750/250mg 330 328 316 296 267 240 208 178 153 143 137 125 120 113 101 90 84 82 73 63 39 0 0
Placebo 321 319 312 299 272 243 213 186 168 153 145 132 121 116 108 102 96 92 89 82 56 0 0

[Fig.12]

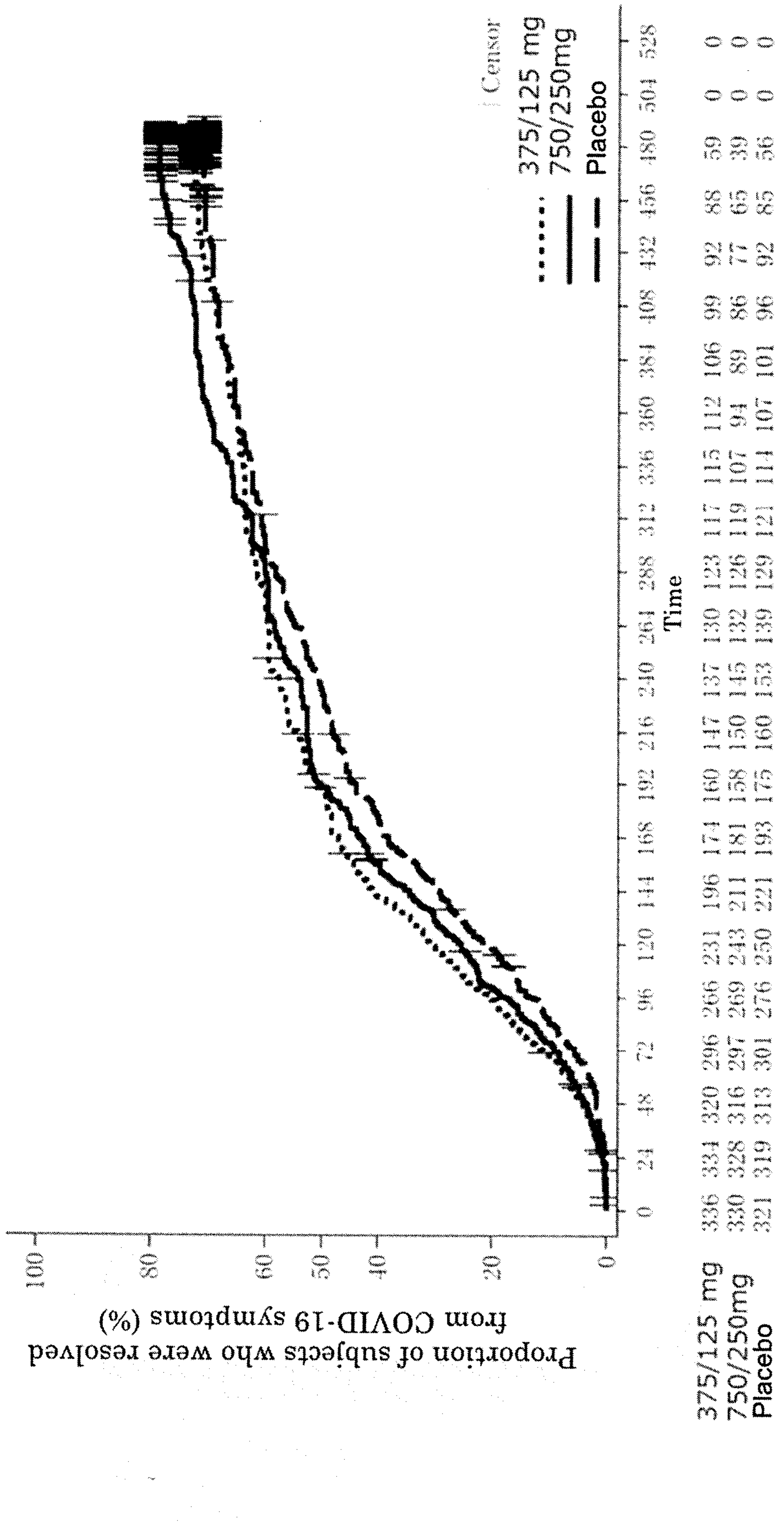
Proportion of subjects who were resolved from COVID-19 symptoms (%)
0 20 40 50 60 80 100
Time
0 24 48 72 96 120 144 168 192 216 240 264 288 312 336 360 384 408 432 456 480 504 528
Censor
375/125 mg
750/250mg
Placebo
375/125 mg 336 334 320 296 266 231 196 174 160 147 137 130 123 117 115 112 106 99 92 88 59 0 0
750/250mg 330 328 316 297 269 243 211 181 158 150 145 132 126 119 107 94 89 86 77 65 39 0 0
Placebo 321 319 313 301 276 250 221 193 175 160 153 139 129 121 114 107 101 96 92 85 56 0 0

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010092966 A
- WO 2012020749 A
- WO 2013089212 A
- WO 2014200078 A
- WO 2012020742 A
- WO 2013118855 A


### Non-patent literature cited in the description


- *J Clin Med*, 2020, vol. 9, 1225
- *J Pathol*, 2004, vol. 203, 631-7
- *WHO Director-General's opening remarks at the media briefing on COVID-19*, 11 March 2020, httpsV/www.who.int/dg/speeches/detail/who-director-general-s-opening-remarks-at-the-media-briefing-on-covid-19---11-march-2020
- *Coronavirus Disease (COVID-19) Dashboard*, 05 October 2020, https://covid19.who.int
- Pfizer's Novel COVID-19 Oral Antiviral Treatment Candidate Reduced Risk Of Hospitalization Or Death By 89% In Interim Analysis Of Phase 2/3 EPIC-HR Study. Pfizer Press, 05 November 2021
- *261st Am Chem Soc (ACS) Natl Meet*, 05 April 2021
- *AIMECS 2021 (AFMC International Medicinal Chemistry Symposium 2021), Online Symposium*, 29 November 2021
- Coronavirus disease 2019 (COVID-19) Guidance of medical examination. Ministry of Health, Labour and Welfare
- **ATHERTON, E.** ; **SHEPPARD, R. C.** In Solid Phase Peptide Synthesis, A Practical Approach. IRL Press at Oxford University Pres, 1989
- *Bioorg. Med. Chem.*, 1997, vol. 5 (9), 1883-1891